# EUROPEAN PATENT APPLICATION

(11) **EP 1 808 491 A1**
(43) Date of publication of application: **18.07.2007**
(21) Application number: 06290094.9
(22) Date of filing: 13.01.2006
(51) Int. Cl.: C12N 15/82, C12N 5/10, A01H 5/00

(54) **Genetically modified plant with modified storage protein content**

(71) Applicant: MERISTEM THERAPEUTICS S.A., 63100 Clermont-Ferrand (FR)
(72) Inventor: Norre, Frederic, 63800 Perignat sur Allier (FR); Burtin, Daniel, 63430 Pont du Château (FR); Marzabal, Pablo, 08027 Barcelona (ES)
(74) Representative: Colombet, Alain André

(57) **Abstract**

The present invention relates to genetically modified plant cells, plant seeds and plants having a modified storage protein content, and the use thereof to produce heterologous molecules, for example having therapeutic activity, in said plants. The cells, seeds and plants thereby obtained are remarkable in that the modified storage protein content enables an increase in the quantity of heterologous molecule produced by these plants.

SEQUENCE LISTING

<110>  Meristem Therapeutics

<120>  Genetically Modified Plant With Modified Storage Protein Content.

<130>  D410 PAT

<160>  63

<170>  PatentIn version 3.3

<210>  1
<211>  34
<212>  DNA
<213>  Artificial

<220>
<223>  Primer for obtaining a full length opaque 2 DNA sequence

<400>  1
cggggtaccc cgatggagca cgtcatctca atgg                                    34

<210>  2
<211>  35
<212>  DNA
<213>  Artificial

<220>
<223>  Primer for obtaining a full length opaque 2 DNA sequence

<400>  2
cccaagcttg ggctaataca tgtccatgtg tatgg                                   35

<210>  3
<211>  42
<212>  DNA
<213>  Artificial

<220>
<223>  Primer for creation of 19/22 alpha zein chimeric sequence from
       maize endosperm cDNAs.

<400>  3
ggaagaaggg aagctatagg agtagatgaa agtagttgta gg                           42

<210>  4
<211>  18
<212>  DNA
<213>  Artificial

<220>
<223>  Primer for creation of 19/22 alpha zein chimeric sequence from
      maize endosperm cDNAs.

<400>  4
ctcctatagc ttcccttc                                                    18


<210>  5
<211>  52
<212>  DNA
<213>  Artificial

<220>
<223>  Primer for creation of 19/22 alpha zein chimeric sequence from
      maize endosperm cDNAs.

<400>  5
cctcgagagc tcactagcaa cgactaacac caatggctac caagatatta gc              52


<210>  6
<211>  38
<212>  DNA
<213>  Artificial

<220>
<223>  Primer for creation of 19/22 alpha zein chimeric sequence from
      maize endosperm cDNAs.

<400>  6
agcttgaatt cctcataaga aatctaaaag agggcacc                              38


<210>  7
<211>  39
<212>  DNA
<213>  Artificial

<220>
<223>  Primer for amplification of the IV2 intron.

<400>  7
ctgcaggaat tcgctagcta cgtaagtttc tgcttctac                             39


<210>  8
<211>  50
<212>  DNA
<213>  Artificial

<220>
<223>  Primer for amplification of the IV2 intron.

<400>  8
atcgataagc ttaggcctgc atgccagctg cacatcaaca atttttggtc                50


<210>  9
<211>  54
<212>  DNA
<213>  Artificial

<220>
<223>  Primer for amplification of gamma zein promoter and nos
       terminator fragment.

<400>  9
aaaggccttt tcggaccgtt ggcgcgccaa ctagcgggtt cttctgcgct ctgg          54


<210>  10
<211>  57
<212>  DNA
<213>  Artificial

<220>
<223>  Primer for amplification of gamma zein promoter and nos
       terminator fragment.

<400>  10
ggtccgaaaa ggccttttca tcgagctcga atttccccga tcgttcaaac atttggc        57


<210>  11
<211>  37
<212>  DNA
<213>  Artificial

<220>
<223>  Primer for amplification of gamma zein promoter and nos
       terminator fragment.

<400>  11
tatctcgagg gccggatcag cttgactgaa atggaag                             37


<210>  12
<211>  45
<212>  DNA
<213>  Artificial

<220>
<223>  Primer for amplification of gamma zein promoter and nos
       terminator fragment.

<400>  12
ctatgttact agatcgggaa ttcggatcga tccggccctc gagca                    45

<210>  13
<211>  20
<212>  DNA
<213>  Artificial

<220>
<223>  Primer for amplification of a gamma zein PCR fragment

<400>  13
gagggtgttg ctcgttgccc                                                    20


<210>  14
<211>  20
<212>  DNA
<213>  Artificial

<220>
<223>  Primer for amplification of a gamma zein PCR fragment

<400>  14
ggcagcagcg taggggcatg                                                    20


<210>  15
<211>  22
<212>  DNA
<213>  Artificial

<220>
<223>  Primer for creation of an opaque 2 fragment minus the bzip
       binding domain.

<400>  15
cctcactctt tcctcggtag gc                                                 22


<210>  16
<211>  42
<212>  DNA
<213>  Artificial

<220>
<223>  Primer for creation of an opaque 2 fragment minus the bzip
       binding domain.

<400>  16
atgcctaccg aggaaagagt gagggactcc ctgaagcggg tg                           42


<210>  17
<211>  42
<212>  DNA
<213>  Artificial

<220>
<223>  Primer for creation of an opaque 2 fragment minus the DNA binding
       domain

<400>  17
atgcctaccg aggaaagagt gaggaggaaa gccgctcacc tg                          42


<210>  18
<211>  63
<212>  DNA
<213>  Artificial

<220>
<223>  Primer for creation of an opaque 2 fragment minus the DNA binding
       domain and including a T7 tag

<400>  18
ggggtaccat ggcctccatg accggcggcc agcagatggg cgagcacgtc atctcaatgg      60

agg                                                                     63


<210>  19
<211>  18
<212>  DNA
<213>  Artificial

<220>
<223>  Primer for RT-PCR analysis of 22 kDa alpha zein coding sequence

<400>  19
acaccgccgg cagcagcg                                                     18


<210>  20
<211>  18
<212>  DNA
<213>  Artificial

<220>
<223>  Primer for RT-PCR analysis of 22 kDa alpha zein coding sequence

<400>  20
agtagttgta ggtagacg                                                     18


<210>  21
<211>  22
<212>  DNA
<213>  Artificial

<220>
<223>  Primer for RT-PCR analysis of 19 kDa alpha zein coding sequence

<400>  21
agcccttatg ccttcttact gc                                              22


<210>  22
<211>  28
<212>  DNA
<213>  Artificial

<220>
<223>  Primer for RT-PCR analysis of 19 kDa alpha zein coding sequence

<400>  22
tatcagcatg agaactttat tattacag                                        28


<210>  23
<211>  19
<212>  DNA
<213>  Artificial

<220>
<223>  Primer for RT-PCR analysis of 19 kDa alpha zein coding sequence

<400>  23
tctacccaca gaatgtggc                                                  19


<210>  24
<211>  18
<212>  DNA
<213>  Artificial

<220>
<223>  Primer for RT-PCR analysis of 19 kDa alpha zein coding sequence

<400>  24
tgccagcgtt aggcgcag                                                   18


<210>  25
<211>  22
<212>  DNA
<213>  Artificial

<220>
<223>  Primer for RT-PCR analysis of 19 kDa alpha zein coding sequence

<400>  25
tggaatccag tagcaacaat ag                                              22


<210>  26
<211>  22

<212>  DNA
<213>  Artificial

<220>
<223>  Primer for RT-PCR analysis of 19 kDa alpha zein coding sequence

<400>  26
ggttgatcag aggtagcacg ag                                                22


<210>  27
<211>  18
<212>  DNA
<213>  Artificial

<220>
<223>  Primer for RT-PCR analysis of 27 kDa gamma zein coding sequence

<400>  27
agtgccagtc gttgcggc                                                     18


<210>  28
<211>  25
<212>  DNA
<213>  Artificial

<220>
<223>  Primer for RT-PCR analysis of 27 kDa gamma zein coding sequence

<400>  28
tacggggaca ccgccggcag cagcg                                             25


<210>  29
<211>  20
<212>  DNA
<213>  Artificial

<220>
<223>  Primer for RT-PCR analysis of 16 kDa gamma zein coding sequence

<400>  29
tggtgatggg tgacactacg                                                   20


<210>  30
<211>  20
<212>  DNA
<213>  Artificial

<220>
<223>  Primer for RT-PCR analysis of 16 kDa gamma zein coding sequence

<400>  30

ggcgctgatg gcggcccagg                                                20

<210>  31
<211>  20
<212>  DNA
<213>  Artificial

<220>
<223>  Primer for RT-PCR analysis of 50 kDa gamma zein coding sequence

<400>  31
aactactata gcagtagctc                                                20

<210>  32
<211>  22
<212>  DNA
<213>  Artificial

<220>
<223>  Primer for RT-PCR analysis of 50 kDa gamma zein coding sequence

<400>  32
accgttatat gagtataacg gc                                             22

<210>  33
<211>  22
<212>  DNA
<213>  Artificial

<220>
<223>  Primer for RT-PCR analysis of 10 kDa delta zein coding sequence

<400>  33
tgcagcacca acaaagggtt gc                                             22

<210>  34
<211>  24
<212>  DNA
<213>  Artificial

<220>
<223>  Primer for RT-PCR analysis of 10 kDa delta zein coding sequence

<400>  34
acgcgtccgg tcgcacatat ctac                                           24

<210>  35
<211>  20
<212>  DNA
<213>  Artificial

<220>
<223>  Primer for RT-PCR analysis of 15 kDa beta zein coding sequence

<400>  35
acaggggttg gtgcggtagc                                                    20


<210>  36
<211>  21
<212>  DNA
<213>  Artificial

<220>
<223>  Primer for RT-PCR analysis of 15 kDa beta zein coding sequence

<400>  36
tacctgaggc agccgcagtg c                                                  21


<210>  37
<211>  22
<212>  DNA
<213>  Artificial

<220>
<223>  Primer for RT-PCR analysis of Opaque 2 coding sequence

<400>  37
tagagaatca gccagacgct cg                                                 22


<210>  38
<211>  19
<212>  DNA
<213>  Artificial

<220>
<223>  Primer for RT-PCR analysis of Opaque 2 coding sequence

<400>  38
tacatgtcca tgtgtatgg                                                     19


<210>  39
<211>  20
<212>  DNA
<213>  Artificial

<220>
<223>  Primer for RT-PCR analysis of Opaque 2 minus bZIP coding sequence

<400>  39
aactcttgtt gctcaggcgc                                                    20

<210>  40
<211>  22
<212>  DNA
<213>  Artificial

<220>
<223>  Primer for RT-PCR analysis of OHPI coding sequence

<400>  40
tgtggagggc ttctggtgca gc                                                22


<210>  41
<211>  22
<212>  DNA
<213>  Artificial

<220>
<223>  Primer for RT-PCR analysis of b32 coding sequence

<400>  41
agcaagctgg tgaagctggt gg                                                22


<210>  42
<211>  22
<212>  DNA
<213>  Artificial

<220>
<223>  Primer for RT-PCR analysis of b32 coding sequence

<400>  42
tcatcgtcgt tgtcagcact gg                                                22


<210>  43
<211>  20
<212>  DNA
<213>  Artificial

<220>
<223>  Primer for PCR detection of silenced alpha zein coding sequence

<400>  43
tgcacttctc catcaccacc                                                   20


<210>  44
<211>  25
<212>  DNA
<213>  Artificial

<220>

<223>  Primer for PCR detection of silenced alpha zein coding sequence

<400>  44
agatgaaagt agttgtaggt agacg                                          25


<210>  45
<211>  23
<212>  DNA
<213>  Artificial

<220>
<223>  Primer for PCR detection of silenced gamma zein coding sequence

<400>  45
agcagcgtag gggcatggag tcg                                            23


<210>  46
<211>  18
<212>  DNA
<213>  Artificial

<220>
<223>  Primer for PCR detection of silenced gamma zein coding sequence

<400>  46
tcgcaagacc ggcaacag                                                  18


<210>  47
<211>  23
<212>  DNA
<213>  Artificial

<220>
<223>  Primer for RT-PCR analysis of OHPI coding sequence

<400>  47
tgctgaggga cgacatatca gac                                            23


<210>  48
<211>  22
<212>  DNA
<213>  Artificial

<220>
<223>  Primer for PCR detection of PBF coding sequence

<400>  48
tgcctctaga tttgtcttgg gc                                             22


<210>  49

<211>  22
<212>  DNA
<213>  Artificial

<220>
<223>  Primer for PCR detection of PBF coding sequence

<400>  49
attgcaccat atgatggcag cg                                              22


<210>  50
<211>  2441
<212>  DNA
<213>  Artificial

<220>
<223>  DNA containg alpha zein inverted repeat sequence

<400>  50
   atggctacca agatattagc cctccttgcg cttcttgccc ttttagtgag cgcaacaaat      60
   gcgttcatta ttccacagtg ctcacttgct cctagtgcca gtattccaca gttcctccca     120
   ccagttactt caatgggctt cgaacatccg gccgtgcaag cctacaggct acaactagcg     180
   cttgcggcga gcgccttaca acaaccaatt gcccaattgc aacaacaatc cttggcacat     240
   ctaaccctac aaaccattgc aacgcaacaa caacaacaac agtttctgcc atcactgagc     300
   cacctagccg tggtgaaccc tgtcacctac ttgcaacagc agctgcttgc atccaaccca     360
   cttgctctgg cgaacgtagc tgcataccag caacaacaac agctgcaaca gtttatgcca     420
   gtgctcagtc aactagccat ggtgaaccct gccgtctacc tacaactact ttcatctact     480
   cctatagctt cccttcttcc ccccatacct ctcaccagcg gtgtcttcaa tgtgtgaaaa     540
   cccaattgtt caaccctaca ggatccaaca ggcaatcgcg acaggcatct taccattatc     600
   acccttgttc ctccaacaac cgtcagccct attacagcag ttacctttgg tccatttggt     660
   ggcacaaaac atcagggcac aacaactaca acaacttgtg ttagcaaacc ttgctgcata     720
   ctctcagcaa catcagtttc ttccattcaa ccaactggct gcattgaact ctgctgctta     780
   tttgcaacaa caattaccat tcagccagct agttgctgcc tacccccagc aatttcttcc     840
   attcaaccaa ctagcagcat tgaactctgc tgcttattta cagcagcaac aactactacc     900
   attcagccag ctagctgatg tgagccctgc tgccttcttg acacaacaac agttgttgcc     960
   gttctacctg cacgctatgc ctaacgctgg caccctctta caactgcaac aattgctgcc    1020
   attcaaccaa cttgctttga caaactcaac agtgttctac caacaaccca tcattggtgg    1080
   tgccctcttt tagatttctt atgaggaatt cgctagctac gtaagtttct gcttctacct 1140
   ttgatatata tataataatt atcattaatt agtagtaata taatatttca aatatttttt 1200
   tcaaaataaa agaatgtagt atatagcaat tgcttttctg tagtttataa gtgtgtatat 1260
   tttaatttat aacttttcta atatatgacc aaaaattgtt gatgtgcagc tggcatgcag 1320
   gcctaagctt gaattcctca taagaaatct aaaagagggc accaccaatg atgggttgtt 1380
   ggtagaacac tgttgagttt gtcaaagcaa gttggttgaa tggcagcaat tgttgcagtt 1440
   gtaagagggt gccagcgtta ggcatagcgt gcaggtagaa cggcaacaac tgttgttgtg 1500
   tcaagaaggc agcagggctc acatcagcta gctggctgaa tggtagtagt tgttgctgct 1560
   gtaaataagc agcagagttc aatgctgcta gttggttgaa tggaagaaat tgctgggggt 1620
   aggcagcaac tagctggctg aatggtaatt gttgttgcaa ataagcagca gagttcaatg 1680
   cagccagttg gttgaatgga agaaactgat gttgctgaga gtatgcagca aggtttgcta 1740
   acacaagttg ttgtagttgt tgtgccctga tgttttgtgc caccaaatgg accaaaggta 1800
   actgctgtaa tagggctgac ggttgttgga ggaacaaggg tgataatggt aagatgcctg 1860
   tcgcgattgc ctgttggatc ctgtagggtt gaacaattgg gttttcacac attgaagaca 1920
   ccgctggtga gaggtatggg gggaagaagg gaagctatag gagtagatga aagtagttgt 1980
   aggtagacgg cagggttcac catggctagt tgactgagca ctggcataaa ctgttgcagc 2040
   tgttgttgtt gctggtatgc agctacgttc gccagagcaa gtgggttgga tgcaagcagc 2100
   tgctgttgca agtaggtgac agggttcacc acggctaggt ggctcagtga tggcagaaac 2160
   tgttgttgtt gttgttgcgt tgcaatggtt tgtagggtta gatgtgccaa ggattgttgt 2220
   tgcaattggg caattggttg ttgtaaggcg ctcgccgcaa gcgctagttg tagcctgtag 2280
   gcttgcacgg ccggatgttc gaagcccatt gaagtaactg gtgggaggaa ctgtggaata 2340
   ctggcactag gagcaagtga gcactgtgga ataatgaacg catttgttgc gctcactaaa 2400
   agggcaagaa gcgcaaggag ggctaatatc ttggtagcca t 2441

<210> 51
<211> 477
<212> DNA
<213> Artificial

<220>
<223> cDNA coding for partial 22 kDa alpha zein protein

<400> 51
atggctacca agatattagc cctccttgcg cttcttgccc ttttagtgag cgcaacaaat 60

gcgttcatta ttccacagtg ctcacttgct cctagtgcca gtattccaca gttcctccca 120

ccagttactt caatgggctt cgaacatccg gccgtgcaag cctacaggct acaactagcg 180

cttgcggcga gcgccttaca acaaccaatt gcccaattgc aacaacaatc cttggcacat 240

ctaaccctac aaaccattgc aacgcaacaa caacaacaac agtttctgcc atcactgagc 300

cacctagccg tggtgaaccc tgtcacctac ttgcaacagc agctgcttgc atccaaccca 360

cttgctctgg cgaacgtagc tgcataccag caacaacaac agctgcaaca gtttatgcca 420

gtgctcagtc aactagccat ggtgaaccct gccgtctacc tacaactact ttcatct 477

<210> 52
<211> 628
<212> DNA
<213> Artificial

<220>
<223> cDNA coding for partial 19 alpha zein

<400> 52
actcctatag cttcccttct tccccccata cctctcacca gcggtgtctt caatgtgtga 60

aaacccaatt gttcaaccct acaggatcca acaggcaatc gcgacaggca tcttaccatt 120

atcacccttg ttcctccaac aaccgtcagc cctattacag cagttacctt tggtccattt 180

ggtggcacaa aacatcaggg cacaacaact acaacaactt gtgttagcaa accttgctgc 240

atactctcag caacatcagt ttcttccatt caaccaactg gctgcattga actctgctgc 300

ttatttgcaa caacaattac cattcagcca gctagttgct gcctaccccc agcaatttct 360

tccattcaac caactagcag cattgaactc tgctgcttat ttacagcagc aacaactact 420

accattcagc cagctagctg atgtgagccc tgctgccttc ttgacacaac aacagttgtt 480

gccgttctac ctgcacgcta tgcctaacgc tggcaccctc ttacaactgc aacaattgct 540

gccattcaac caacttgctt tgacaaactc aacagtgttc taccaacaac ccatcattgg 600

tggtgccctc ttttagattt cttatgag 628

<210> 53
<211> 1578
<212> DNA
<213> Artificial

<220>
<223> DNA containing a gamma zein inverted repeat sequence

<400> 53
atgagggtgt tgctcgttgc cctcgctctc ctggctctcg ctgcgagcgc cacctccacg 60

catacaagcg gcggctgcgg ctgccagcca ccgccgccgg ttcatctacc gccgccggtg 120

catctgccac ctccggttca cctgccacct ccggtgcatc tcccaccgcc ggtccacctg 180

ccgccgccgg tccacctgcc accgccggtc catgtgccgc cgccggttca tctgccgccg 240

ccaccatgcc actaccctac tcaaccgccc cggcctcagc ctcatcccca gccacaccca 300

tgcccgtgcc aacagccgca tccaagcccg tgccagctgc agggaacctg cggcgttggc 360

agcaccccga tcctgggcca gtgcgtcgag tttctgaggc atcagtgcag cccgacggcg 420

acgccctact gctcgcctca gtgccagtcg ttgcggcagc agtgttgcca gcagctcagg 480

caggtggagc cgcagcaccg gtaccaggcg atcttcggct tggtcctcca gtccatcctg 540

cagcagcagc cgcaaagcgg ccaggtcgcg gggctgttgg cggcgcagat agcgcagcaa 600

ctgacggcga tgtgcggcct gcagcagccg actccatgcc cctacgctgc tgccatcgaa 660

ttaattcgct agctacgtaa gtttctgctt ctacctttga tatatatata ataattatca 720

ttaattagta gtaatataat atttcaaata tttttttcaa aataaaagaa tgtagtatat 780

agcaattgct tttctgtagt ttataagtgt gtatatttta atttataact tttctaatat 840

atgaccaaaa attgttgatg tgcagctggc atgcaggcct aagcttatcg ataatcaagc 900

ttatcgatac cgtcgaaatt cgatggcagc agcgtagggg catggagtcg gctgctgcag 960

gccgcacatc gccgtcagtt gctgcgctat ctgcgccgcc aacagccccg cgacctggcc 1020

gctttgcggc tgctgctgca ggatggactg gaggaccaag ccgaagatcg cctggtaccg 1080

gtgctgcggc tccacctgcc tgagctgctg gcaacactgc tgccgcaacg actggcactg 1140

aggcgagcag tagggcgtcg ccgtcgggct gcactgatgc ctcagaaact cgacgcactg 1200

gcccaggatc ggggtgctgc caacgccgca ggttccctgc agctggcacg ggcttggatg 1260

cggctgttgg cacgggcatg ggtgtggctg gggatgaggc tgaggccggg gcggttgagt 1320

agggtagtgg catggtggcg gcggcagatg aaccggcggc ggcacatgga ccggcggtgg 1380

caggtggacc ggcggcggca ggtggaccgg cggtgggaga tgcaccggag gtggcaggtg 1440

aaccggaggt ggcagatgca ccggcggcgg tagatgaacc ggcggcggtg gctggcagcc 1500

gcagccgccg cttgtatgcg tggaggtggc gctcgcagcg agagccagga gagcgagggc 1560

aacgagcaac accctcat 1578

<210> 54
<211> 654
<212> DNA
<213> Artificial

<220>
<223> cDNA coding for 27 kDa gamma zein

<400> 54
atgagggtgt tgctcgttgc cctcgctctc ctggctctcg ctgcgagcgc cacctccacg 60

catacaagcg gcggctgcgg ctgccagcca ccgccgccgg ttcatctacc gccgccggtg 120

catctgccac ctccggttca cctgccacct ccggtgcatc tcccaccgcc ggtccacctg 180

ccgccgccgg tccacctgcc accgccggtc catgtgccgc cgccggttca tctgccgccg 240

ccaccatgcc actaccctac tcaaccgccc cggcctcagc ctcatcccca gccacaccca 300

tgcccgtgcc aacagccgca tccaagcccg tgccagctgc agggaacctg cggcgttggc 360

agcaccccga tcctgggcca gtgcgtcgag tttctgaggc atcagtgcag cccgacggcg 420

acgccctact gctcgcctca gtgccagtcg ttgcggcagc agtgttgcca gcagctcagg 480

caggtggagc cgcagcaccg gtaccaggcg atcttcggct tggtcctcca gtccatcctg 540

cagcagcagc cgcaaagcgg ccaggtcgcg gggctgttgg cggcgcagat agcgcagcaa 600

ctgacggcga tgtgcggcct gcagcagccg actccatgcc cctacgctgc tgcc 654

<210> 55
<211> 1326
<212> DNA
<213> Zea mays

<220>
<221> misc_feature
<222> (1)..(1326)
<223> Full length coding sequence for Opaque 2

<400> 55
atggagcacg tcatctcaat ggaggagatc ctcgggccct tctgggagct gctaccaccg 60

ccagcgccag agccagagcc agagcgagag cagcctccgg taaccggcat cgtcgtcggc 120

agtgtcatag acgttgctgc tgctggtcac ggtcatggtg gcggcgacat gatggatcag 180

cagcacgcca cagagtggac ctttgagagg ttactggaag aggaggctct gacgacaagc 240

acaccgccgc cggtggtggt ggtgccgaac tcttgttgct caggcgccct aaatgttgac 300

cggccgccgg tgatggaaga ggcggtaatg atggcgcctg cggcggtgag tagtgccgta 360

gtaggggacc ccatggagta caatgccata ctgaggagga agctggagga ggacctcgag 420

gccttcaaaa tgtggagggc ggcctccagt gttgtgacct cagatcaacg ttctcaaggc 480

tcaaacaatc acactggagg tagcagcatc aggaataatc cagtgcagaa caagctgatg 540

aacggcgaag atccaatcaa caataaccac gctcaaaccg caggccttgg cgtgaggctt 600

gctactagct cttcctcgag agatccttca ccatcagacg aagacatgga cggagaagta 660

gagattctgg ggttcaagat gcctaccgag gaaagagtga ggaaaagaaa ggaatccaat 720

agagaatcag ccagacgctc gagatacagg aaagccgctc acctgaaaga actggaggac 780

caggtagcac agctaaaagc cgagaattct tgcctgctga ggcgcattgc cgctctgaac 840

cagaagtaca acgacgctaa cgtcgacaac agggtgctga gagcggacat ggagacccta 900

agagctaagg tgaagatggg agaggactcc ctgaagcggg tgatagagat gagctcatca 960

gtgccgtcgt ccatgcccat ctcggcgccg acccccagct ccgacgctcc agtgccgccg 1020

ccgcctatcc gagacagcat cgtcggctac ttctccgcca cagccgcaga cgacgatgct 1080

tcggtcggca acggtttctt gcgactgcaa gctcatcaag agcctgcatc catggtcgtc 1140

ggtggaactc tgagcgccac agagatgaac cgagtagcag cagccacgca ttgcgcgggg 1200

gccatggagc tcatccagac ggcgatggga tccatgccgc cgacctccgc ctccggatct 1260

acaccgccgc cgcaggatta tgagctgctg ggtccaaatg ggccatacac catggacatg 1320

tattag 1326

<210> 56
<211> 2510
<212> DNA
<213> Artificial

<220>
<223> DNA containing Opaque 2 inverted repeat sequence

<400> 56
cccgatggag cacgtcatct caatggagga gatcctcggg cccttctggg agctgctacc 60

accgccagcg ccagagccag agccagagcg agagcagcct ccggtaaccg gcatcgtcgt 120

cggcagtgtc atagacgttg ctgctgctgg tcacggtcat ggtggcggcg acatgatgga 180

tcagcagcac gccacagagt ggacctttga gaggttactg gaagaggagg ctctgacgac 240
aagcacaccg ccgccggtgg tggtggtgcc gaactcttgt tgctcaggcg ccctaaatgt 300
tgaccggccg ccggtgatgg aagaggcggt aatgatggcg cctgcggcgg tgagtagtgc 360
cgtagtaggg gaccccatgg agtacaatgc catactgagg aggaagctgg aggaggacct 420
cgaggccttc aaaatgtgga gggcggcctc cagtgttgtg acctcagatc aacgttctca 480
aggctcaaac aatcacactg gaggtagcag catcaggaat aatccagtgc agaacaagct 540
gatgaacggc gaagatccaa tcaacaataa ccacgctcaa accgcaggcc ttggcgtgag 600
gcttgctact agctcttcct cgagagatcc ttcaccatca gacgaagaca tggacggaga 660
agtagagatt ctggggttca agatgcctac cgaggaaaga gtgagggact ccctgaagcg 720
ggtgatagag atgagctcat cagtgccgtc gtccatgccc atctcggcgc cgacccccag 780
ctccgacgct ccagtgccgc cgccgcctat ccgagacagc atcgtcggct acttctccgc 840
cacagccgca gacgacgatg cttcggtcgg caacggtttc ttgcgactgc aagctcatca 900
agagcctgca tccatggtcg tcggtggaac tctgagcgcc acagagatga accgagtagc 960
agcagccacg cattgcgcgg gggccatgga gctcatccag acggcgatgg gatccatgcc 1020
gccgacctcc gcctccggat ctacaccgcc gccgcaggat tatgagctgc tgggtccaaa 1080
tggggccata cacatggaca tgtattagcc caagcttggg atcgaattaa ttcgctagct 1140
acgtaagttt ctgcttctac ctttgatata tatataataa ttatcattaa ttagtagtaa 1200
tataatattt caaatatttt tttcaaaata aaagaatgta gtatatagca attgcttttc 1260
tgtagtttat aagtgtgtat attttaattt ataacttttc taatatatga ccaaaaattg 1320
ttgatgtgca gctggcatgc aggcctaagc ttatcgataa tcaagcttat cgataccgtc 1380
gaaattcgat cccaagcttg ggctaataca tgtccatgtg tatggcccca tttggaccca 1440
gcagctcata atcctgcggc ggcggtgtag atccggaggc ggaggtcggc ggcatggatc 1500
ccatcgccgt ctggatgagc tccatggccc ccgcgcaatg cgtggctgct gctactcggt 1560
tcatctctgt ggcgctcaga gttccaccga cgaccatgga tgcaggctct tgatgagctt 1620
gcagtcgcaa gaaaccgttg ccgaccgaag catcgtcgtc tgcggctgtg gcggagaagt 1680
agccgacgat gctgtctcgg ataggcggcg gcggcactgg agcgtcggag ctgggggtcg 1740
gcgccgagat gggcatggac gacggcactg atgagctcat ctctatcacc cgcttcaggg 1800

agtccctcac tctttcctcg gtaggcatct tgaaccccag aatctctact tctccgtcca 1860

tgtcttcgtc tgatggtgaa ggatctctcg aggaagagct agtagcaagc ctcacgccaa 1920

ggcctgcggt ttgagcgtgg ttattgttga ttggatcttc gccgttcatc agcttgttct 1980

gcactggatt attcctgatg ctgctacctc cagtgtgatt gtttgagcct tgagaacgtt 2040

gatctgaggt cacaacactg gaggccgccc tccacatttt gaaggcctcg aggtcctcct 2100

ccagcttcct cctcagtatg gcattgtact ccatggggtc ccctactacg gcactactca 2160

ccgccgcagg cgccatcatt accgcctctt ccatcaccgg cggccggtca acatttaggg 2220

cgcctgagca acaagagttc ggcaccacca ccaccggcgg cggtgtgctt gtcgtcagag 2280

cctcctcttc cagtaacctc tcaaaggtcc actctgtggc gtgctgctga tccatcatgt 2340

cgccgccacc atgaccgtga ccagcagcag caacgtctat gacactgccg acgacgatgc 2400

cggttaccgg aggctgctct cgctctggct ctggctctgg cgctggcggt ggtagcagct 2460

cccagaaggg cccgaggatc tcctccattg agatgacgtg ctccatcggg 2510

<210> 57
<211> 1112
<212> DNA
<213> Artificial

<220>
<223> cDNA coding for Opaque 2 minus bZIP region

<400> 57
cccgatggag cacgtcatct caatggagga gatcctcggg cccttctggg agctgctacc 60

accgccagcg ccagagccag agccagagcg agagcagcct ccggtaaccg gcatcgtcgt 120

cggcagtgtc atagacgttg ctgctgctgg tcacggtcat ggtggcggcg acatgatgga 180

tcagcagcac gccacagagt ggacctttga gaggttactg gaagaggagg ctctgacgac 240

aagcacaccg ccgccggtgg tggtggtgcc gaactcttgt tgctcaggcg ccctaaatgt 300

tgaccggccg ccggtgatgg aagaggcggt aatgatggcg cctgcggcgg tgagtagtgc 360

cgtagtaggg gaccccatgg agtacaatgc catactgagg aggaagctgg aggaggacct 420

cgaggccttc aaaatgtgga gggcggcctc cagtgttgtg acctcagatc aacgttctca 480

aggctcaaac aatcacactg gaggtagcag catcaggaat aatccagtgc agaacaagct 540

gatgaacggc gaagatccaa tcaacaataa ccacgctcaa accgcaggcc ttggcgtgag 600

gcttgctact agctcttcct cgagagatcc ttcaccatca gacgaagaca tggacggaga 660

agtagagatt ctggggttca agatgcctac cgaggaaaga gtgagggact ccctgaagcg 720

ggtgatagag atgagctcat cagtgccgtc gtccatgccc atctcggcgc cgacccccag 780

ctccgacgct ccagtgccgc cgccgcctat ccgagacagc atcgtcggct acttctccgc 840

cacagccgca gacgacgatg cttcggtcgg caacggtttc ttgcgactgc aagctcatca 900

agagcctgca tccatggtcg tcggtggaac tctgagcgcc acagagatga accgagtagc 960

agcagccacg cattgcgcgg gggccatgga gctcatccag acggcgatgg gatccatgcc 1020

gccgacctcc gcctccggat ctacaccgcc gccgcaggat tatgagctgc tgggtccaaa 1080

tggggccata cacatggaca tgtattagcc ca 1112

<210> 58
<211> 1308
<212> DNA
<213> Artificial

<220>
<223> cDNA coding for Opaque 2 minus DNA binding domain fused to T7 tag

<400> 58
atggcctcca tgaccggcgg ccagcagatg ggcgagcacg tcatctcaat ggaggagatc 60

ctcgggccct tctgggagct gctaccaccg ccagcgccag agccagagcc agagcgagag 120

cagcctccgg taaccggcat cgtcgtcggc agtgtcatag acgttgctgc tgctggtcac 180

ggtcatggtg gcggcgacat gatggatcag cagcacgcca cagagtggac ctttgagagg 240

ttactggaag aggaggctct gacgacaagc acaccgccgc cggtggtggt ggtgccgaac 300

tcttgttgct caggcgccct aaatgttgac cggccgccgg tgatggaaga ggcggtaatg 360

atggcgcctg cggcggtgag tagtgccgta gtaggggacc ccatggagta caatgccata 420

ctgaggagga agctggagga ggacctcgag gccttcaaaa tgtggagggc ggcctccagt 480

gttgtgacct cagatcaacg ttctcaaggc tcaaacaatc acactggagg tagcagcatc 540

aggaataatc cagtgcagaa caagctgatg aacggcgaag atccaatcaa caataaccac 600

gctcaaaccg caggccttgg cgtgaggctt gctactagct cttcctcgag agatccttca 660

ccatcagacg aagacatgga cggagaagta gagattctgg ggttcaagat gcctaccgag 720

gaaagagtga ggaggaaagc cgctcacctg aaagaactgg aggaccaggt agcacagcta 780

aaagccgaga attcttgcct gctgaggcgc attgccgctc tgaaccagaa gtacaacgac 840

gctaacgtcg acaacagggt gctgagagcg gacatggaga ccctaagagc taaggtgaag 900

atgggagagg actccctgaa gcgggtgata gagatgagct catcagtgcc gtcgtccatg 960

cccatctcgg cgccgacccc cagctccgac gctccagtgc cgccgccgcc tatccgagac 1020

agcatcgtcg gctacttctc cgccacagcc gcagacgacg atgcttcggt cggcaacggt 1080

ttcttgcgac tgcaagctca tcaagagcct gcatccatgg tcgtcggtgg aactctgagc 1140

gccacagaga tgaaccgagt agcagcagcc acgcattgcg cgggggccat ggagctcatc 1200

cagacggcga tgggatccat gccgccgacc tccgcctccg gatctacacc gccgccgcag 1260

gattatgagc tgctgggtcc aaatggggcc atacacatgg acatgtat 1308

<210> 59
<211> 33
<212> DNA
<213> Artificial

<220>
<223> T7 tag used in SEQ.ID 58

<400> 59
atggcctcca tgaccggcgg ccagcagatg ggc 33

<210> 60
<211> 1278
<212> DNA
<213> Artificial

<220>
<223> cDNA coding for Opaque 2 minus DNA binding domain (BD) used in
SEQ.ID58

<400> 60
gagcacgtca tctcaatgga ggagatcctc gggcccttct gggagctgct accaccgcca 60

gcgccagagc cagagccaga gcgagagcag cctccggtaa ccggcatcgt cgtcggcagt 120

gtcatagacg ttgctgctgc tggtcacggt catggtggcg gcgacatgat ggatcagcag 180

cacgccacag agtggacctt tgagaggtta ctggaagagg aggctctgac gacaagcaca 240

ccgccgccgg tggtggtggt gccgaactct tgttgctcag gcgccctaaa tgttgaccgg 300

ccgccggtga tggaagaggc ggtaatgatg gcgcctgcgg cggtgagtag tgccgtagta 360

ggggacccca tggagtacaa tgccatactg aggaggaagc tggaggagga cctcgaggcc 420

ttcaaaatgt ggagggcggc ctccagtgtt gtgacctcag atcaacgttc tcaaggctca 480

aacaatcaca ctggaggtag cagcatcagg aataatccag tgcagaacaa gctgatgaac 540

ggcgaagatc caatcaacaa taaccacgct caaaccgcag gccttggcgt gaggcttgct 600

actagctctt cctcgagaga tccttcacca tcagacgaag acatggacgg agaagtagag 660

attctggggt tcaagatgcc taccgaggaa agagtgagga ggaaagccgc tcacctgaaa 720

gaactggagg accaggtagc acagctaaaa gccgagaatt cttgcctgct gaggcgcatt 780

gccgctctga accagaagta caacgacgct aacgtcgaca acagggtgct gagagcggac 840

atggagaccc taagagctaa ggtgaagatg ggagaggact ccctgaagcg ggtgatagag 900

atgagctcat cagtgccgtc gtccatgccc atctcggcgc cgacccccag ctccgacgct 960

ccagtgccgc cgccgcctat ccgagacagc atcgtcggct acttctccgc cacagccgca 1020

gacgacgatg cttcggtcgg caacggtttc ttgcgactgc aagctcatca agagcctgca 1080

tccatggtcg tcggtggaac tctgagcgcc acagagatga accgagtagc agcagccacg 1140

cattgcgcgg gggccatgga gctcatccag acggcgatgg gatccatgcc gccgacctcc 1200

gcctccggat ctacaccgcc gccgcaggat tatgagctgc tgggtccaaa tggggccata 1260

cacatggaca tgtattag 1278

<210> 61
<211> 441
<212> PRT
<213> Zea mays

<220>
<221> PEPTIDE
<222> (1)..(441)
<223> Opaque 2 protein

<400> 61

Met Glu His Val Ile Ser Met Glu Glu Ile Leu Gly Pro Phe Trp Glu
1               5                   10                  15

Leu Leu Pro Pro Pro Ala Pro Glu Pro Glu Pro Glu Arg Glu Gln Pro
            20                  25                  30

Pro Val Thr Gly Ile Val Val Gly Ser Val Ile Asp Val Ala Ala Ala
        35                  40                  45

Gly His Gly His Gly Gly Gly Asp Met Met Asp Gln Gln His Ala Thr

50                  55                  60

Glu Trp Thr Phe Glu Arg Leu Leu Glu Glu Glu Ala Leu Thr Thr Ser
65                  70                  75                  80

Thr Pro Pro Pro Val Val Val Val Pro Asn Ser Cys Cys Ser Gly Ala
                85                  90                  95

Leu Asn Val Asp Arg Pro Pro Val Met Glu Glu Ala Val Met Met Ala
            100                 105                 110

Pro Ala Ala Val Ser Ser Ala Val Val Gly Asp Pro Met Glu Tyr Asn
        115                 120                 125

Ala Ile Leu Arg Arg Lys Leu Glu Glu Asp Leu Glu Ala Phe Lys Met
    130                 135                 140

Trp Arg Ala Ala Ser Ser Val Val Thr Ser Asp Gln Arg Ser Gln Gly
145                 150                 155                 160

Ser Asn Asn His Thr Gly Gly Ser Ser Ile Arg Asn Asn Pro Val Gln
                165                 170                 175

Asn Lys Leu Met Asn Gly Glu Asp Pro Ile Asn Asn Asn His Ala Gln
            180                 185                 190

Thr Ala Gly Leu Gly Val Arg Leu Ala Thr Ser Ser Ser Ser Arg Asp
        195                 200                 205

Pro Ser Pro Ser Asp Glu Asp Met Asp Gly Glu Val Glu Ile Leu Gly
    210                 215                 220

Phe Lys Met Pro Thr Glu Glu Arg Val Arg Lys Arg Lys Glu Ser Asn
225                 230                 235                 240

Arg Glu Ser Ala Arg Arg Ser Arg Tyr Arg Lys Ala Ala His Leu Lys
                245                 250                 255

Glu Leu Glu Asp Gln Val Ala Gln Leu Lys Ala Glu Asn Ser Cys Leu
            260                 265                 270

Leu Arg Arg Ile Ala Ala Leu Asn Gln Lys Tyr Asn Asp Ala Asn Val
        275                 280                 285

Asp Asn Arg Val Leu Arg Ala Asp Met Glu Thr Leu Arg Ala Lys Val
    290                 295                 300

Lys Met Gly Glu Asp Ser Leu Lys Arg Val Ile Glu Met Ser Ser Ser
305                 310                 315                 320

Val Pro Ser Ser Met Pro Ile Ser Ala Pro Thr Pro Ser Ser Asp Ala
                325                 330                 335

Pro Val Pro Pro Pro Pro Ile Arg Asp Ser Ile Val Gly Tyr Phe Ser
            340                 345                 350

Ala Thr Ala Ala Asp Asp Asp Ala Ser Val Gly Asn Gly Phe Leu Arg
        355                 360                 365

Leu Gln Ala His Gln Glu Pro Ala Ser Met Val Val Gly Gly Thr Leu
    370                 375                 380

Ser Ala Thr Glu Met Asn Arg Val Ala Ala Ala Thr His Cys Ala Gly
385                 390                 395                 400

Ala Met Glu Leu Ile Gln Thr Ala Met Gly Ser Met Pro Pro Thr Ser
                405                 410                 415

Ala Ser Gly Ser Thr Pro Pro Pro Gln Asp Tyr Glu Leu Leu Gly Pro
            420                 425                 430

Asn Gly Ala Ile His Met Asp Met Tyr
        435                 440

<210>  62
<211>  2136
<212>  DNA
<213>  Homo sapiens

<220>
<221>  misc_feature
<222>  (1)..(2136)
<223>  cDNA coding for mature recombinant human lactoferrin

<400> 62
atgaaacttg tcttcctcgt cctgctgttc ctcggggccc tcggactgtg tctggctggc 60
cgtaggagaa ggagtgttca gtggtgcgcc gtatcccaac ccgaggccac aaaatgcttc 120
caatggcaaa ggaatatgag aaaagtgtgt ggccctcctg tcagctgcat aaagagagac 180
tcccccatcc agtgtatcca ggccattgcg gaaaacaggg ccgatgctgt gacccttgat 240
ggtggtttca tatacgaggc aggcctggcc ccctacaaac tgcgacctgt agcggcggaa 300
gtctacggga ccgaaagaca gccacgaact cactattatg ccgtggctgt ggtgaagaag 360
ggcggcagct ttcagctgaa cgaactgcaa ggtctgaagt cctgccacac aggccgtcgc 420
aggaccgctg gatggaatgt ccctataggg acacttcgtc cattcttgaa ttggacgggt 480
ccacctgagc ccattgaggc agctgtggcc aggttcttct cagccagctg tgttcccggt 540
gcagataaag gacagttccc caacctgtgt cgcctgtgtg cggggacagg ggaaaacaaa 600
tgtgccttct cctcccagga accgtacttc agctactctg gtgccttcaa gtgtctgaga 660
gacggggctg gagacgtggc ttttatcaga gagagcacag tgtttgagga cctgtcagac 720
gaggctgaaa gggacgagta tgagttactc tgcccagaca acactcggaa gccagtggac 780
aagttcaaag actgccatct ggcccgggtc ccttctcatg ccgttgtggc acgaagtgtg 840
aatggcaagg aggatgccat ctggaatctt ctccgccagg cacaggaaaa gtttggaaag 900
gacaagtcac cgaaattcca gctctttggc tcccctagtg ggcagaaaga tctgctgttc 960
aaggactctg ccattgggtt ttcgagggtg cccccgagga tagattctgg gctgtacctt 1020
ggctccggct acttcactgc catccagaac ttgaggaaaa gtgaggagga agtggctgcc 1080
cggcgtgcgc gggtcgtgtg gtgtgcggtg ggcgagcagg agctgcgcaa gtgtaaccag 1140
tggagtggct tgagcgaagg cagcgtgacc tgctcctcgg cctccaccac agaggactgc 1200
atcgccctgg tgctgaaagg agaagctgat gccatgagtt tggatggagg atatgtgtac 1260
actgcaggca aatgtggttt ggtgcctgtc ctggcagaga actacaaatc ccaacaaagc 1320
agtgaccctg atcctaactg tgtggataga cctgtggaag gatatcttgc tgtggcggtg 1380
gttaggagat cagacactag ccttacctgg aactctgtga aaggcaagaa gtcctgccac 1440
accgccgtgg acaggactgc aggctggaat atccccatgg gcctgctctt caaccagacg 1500
ggctcctgca aatttgatga atatttcagt caaagctgtg cccctgggtc tgacccgaga 1560
tctaatctct gtgctctgtg tattggcgac gagcagggtg agaataagtg cgtgcccaac 1620

agcaacgaga gatactacgg ctacactggg gctttccggt gcctggctga gaatgctgga 1680

gacgttgcat ttgtgaaaga tgtcactgtc ttgcagaaca ctgatggaaa taacaatgag 1740

gcatgggcta aggatttgaa gctggcagac tttgcgctgc tgtgcctcga tggcaaacgg 1800

aagcctgtga ctgaggctag aagctgccat cttgccatgg ccccgaatca tgccgtggtg 1860

tctcggatgg ataaggtgga acgcctgaaa caggtgttgc tccaccaaca ggctaaattt 1920

gggagaaatg gatctgactg cccggacaag ttttgcttat tccagtctga aaccaaaaac 1980

cttctgttca atgacaacac tgagtgtctg gccagactcc atggcaaaac aacatatgaa 2040

aaatatttgg gaccacagta tgtcgcaggc attactaatc tgaaaaagtg ctcaacctcc 2100

cccctcctgg aagcctgtga actcctcagg aagtaa 2136

<210> 63
<211> 1105
<212> DNA
<213> Artificial

<220>
<223> DNA containing chimeric alpha 19 and alpha 22 zein sequence

<400> 63
atggctacca agatattagc cctccttgcg cttcttgccc ttttagtgag cgcaacaaat 60

gcgttcatta ttccacagtg ctcacttgct cctagtgcca gtattccaca gttcctccca 120

ccagttactt caatgggctt cgaacatccg gccgtgcaag cctacaggct acaactagcg 180

cttgcggcga gcgccttaca acaaccaatt gcccaattgc aacaacaatc cttggcacat 240

ctaaccctac aaaccattgc aacgcaacaa caacaacaac agtttctgcc atcactgagc 300

cacctagccg tggtgaaccc tgtcacctac ttgcaacagc agctgcttgc atccaaccca 360

cttgctctgg cgaacgtagc tgcataccag caacaacaac agctgcaaca gtttatgcca 420

gtgctcagtc aactagccat ggtgaaccct gccgtctacc tacaactact ttcatctact 480

cctatagctt cccttcttcc ccccatacct ctcaccagcg gtgtcttcaa tgtgtgaaaa 540

cccaattgtt caaccctaca ggatccaaca ggcaatcgcg acaggcatct taccattatc 600

acccttgttc ctccaacaac cgtcagccct attacagcag ttacctttgg tccatttggt 660

ggcacaaaac atcagggcac aacaactaca acaacttgtg ttagcaaacc ttgctgcata 720

ctctcagcaa catcagtttc ttccattcaa ccaactggct gcattgaact ctgctgctta 780

tttgcaacaa caattaccat tcagccagct agttgctgcc tacccccagc aatttcttcc 840

attcaaccaa ctagcagcat tgaactctgc tgcttattta cagcagcaac aactactacc 900

attcagccag ctagctgatg tgagccctgc tgccttcttg acacaacaac agttgttgcc 960

gttctacctg cacgctatgc ctaacgctgg caccctctta caactgcaac aattgctgcc 1020

attcaaccaa cttgctttga caaactcaac agtgttctac caacaaccca tcattggtgg 1080

tgccctcttt tagatttctt atgag 1105

## Description

The present invention relates to genetically modified plants, their cells, and products produced in such plants and plant cells. Genetic modification of plants and plant cells as such is now a well known field. It has been shown possible to alter the genetic characteristics of plant cells, and the plants regenerated therefrom, and to use these traits to various ends, for example for producing heterologous molecules, for example, therapeutically active recombinant polypeptides, in plants. Examples of such therapeutically active polypeptides are antibodies, and antibody fragments, enzymes, in particular digestive enzymes, such as phytase, dog gastric lipase, and pancreatic lipase, but also other polypeptides such as proteolytic enzymes, for example, alpha anti-trypsin, proteins with a catalytic activity, for example glucocerebrosidase, structural proteins, for example, collagen, transport proteins, for example, haemoglobin, multifunctional proteins with antimicrobial activity, for example, lactoferrin, blood-derived proteins, for example, albumin, and antigenic proteins, for example, rabies glycoprotein G.

A particular problem that has been encountered with expressing such non-plant, or heterologous molecules, for example, therapeutically active recombinant polypeptides, is the relatively low amounts of protein actually produced in the cell of the plant, compared to other recombinant protein expression systems. Previous attempts to overcome this problem have been made by forcing the plant to activate production of the recombinant polypeptide only in the seed, or in one or more specific tissues, for example, in the leaf or stem or roots. Where expression in seed is concerned, the nucleic acid sequence coding for the polypeptide has been associated with a transcriptional promoter that is only active after the seed forming stage of the plant's life has been initiated. In this way, the recombinant polypeptide is only expressed in the seed of the plant, along with the other native seed storage proteins that may be used for the seed's future germination and plantlet development when the seed is sowed. However, even this general approach has not been found to be totally satisfactory for some recombinant proteins, and does still not provide the levels of production of recombinant polypeptides that are of sufficient economic interest for the industrialisation techniques required to produce large scale recombinant therapeutic polypeptides.

The present invention proposes to significantly improve this situation, and the inventors have discovered that it is possible to genetically modify a plant cell in such a way that the cell will have a modified storage protein production profile, and in particular, a modified seed storage production profile. This modified storage protein production profile is useful for a variety of purposes. One such use is that the modified storage protein production profile, when associated with the expression of a heterologous molecule, will cause far more of the heterologous molecule to accumulate in the target tissue than was hitherto possible, thus opening the way for large scale and economically viable industrial production of said molecules.

Accordingly, one of the objects of the present invention is a genetically modified plant cell comprising at least one stably integrated nucleic acid sequence that specifically modulates a production profile of at least one plant storage protein.

In accordance with the objects of the present invention, reference is made to a "heterologous" molecule. As used in the present description and claims, this expression means that the modified plant produces molecules that are not naturally produced in corresponding non-genetically modified plants of the same species, or their wild-type mutants. In other words, the use of the term "heterologous" in this context means that the molecule in question is not normally produced in the target plants or target plant cells, except *via* man-made genetic intervention in the genetic make-up of the plant.

Exemplary heterologous molecules can be small molecules, for example, alkanes, alkenes, alkylenes, carboxylic acids, cyclic aliphatic and aromatic molecules, sugars, starches and modified starches, all optionally branched, and / or substituted, or long chain saturated or unsaturated fatty acids, ketones, ketals, and the like, which are not naturally produced in plants and plant cells, but may be produced in other host cells, for example in prokaryote cells such as bacteria, or yeast, fungal or mammalian cells. Other, and from the invention's standpoint, most preferred heterologous molecules are:
- therapeutically active molecules: this includes all heterologous molecules from other host cells that have a beneficial physiological effect on a human or animal subject in a recognised disease state or pathological dysfunction of said subject, including for prophylactic purposes; such molecules include peptides, polypeptides, proteins, hormones, enzymes and the like, and most preferably a polypeptide having an activity selected from the group consisting of digestive, pancreatic, biliary, antiviral, anti-inflammatory, pulmonary, antimicrobial, haematological, neurological, cardiovascular, ophthalmic, antigenic, cerebral, anti-tumour, immunostimulating, and immunomodulating activities; in particularly preferred embodiments, the therapeutically active polypeptide is chosen from the group consisting of insulins, interferons, gastric lipases, pancreatic lipases or biliary lipases, elastases, anti-proteases such as alpha-1 anti-trypsin, structure-forming proteins such as collagen, transferrins such as lactoferrin, blood-derived proteins, such as human haemoglobin or albumin, and blood cofactors, antioxidants such as superoxide dismutase, antibodies, antibody fragments and antigens. Where the polypeptide is selected from the group of antibodies and antibody fragments, these can comprise immunoglobulin molecules, immunoglobulin heavy chain, substantially intact immunoglobulin molecules, and any portion of an immunoglobulin that contains the paratope, including Fab fragments, Fab' fragments, F(ab').sub.2 fragments and Fv-fragments, immunoglobulin light chain, and F.sub.V fragments;
- cosmetically active molecules, under many legislations, are those molecules that have an activity which is selectively limited to the epiderm, that is, the molecules do not pass through to the derm, or if they do, have no action on the derm and the basal cells. Such molecules are well known to the skilled person per se, examples being alpha-hydroxy acids, ceramides, moisturising agents, bactericidal agents, and the like;
- nutraceutically active molecules, that is, molecules that are related to the molecules commonly found in human and animal dietary regimes, and which can be found in food or part of a food and which provides health benefits. A nutraceutical may be a naturally nutrient-rich or medicinally active food, such as garlic or soybeans, or it may be a specific component of a food, such as the omega-3 fish oil that can be derived from salmon and other cold-water fish;
- as examples of active heterologous molecules not normally expressed in the target host plant, one can cite all of those molecules having a therapeutic or cosmetic activity in humans or animals, such as modified or derivatized PAL (phenylalanine ammonia lyase), allergens, for examples, birch and Graminae, poplar, superoxide dismutase (SOD), and the like.

The term "nucleic acid sequence", as used in the present application, means a single or double-stranded oligomer or polymer of nucleotide or nucleoside bases read from the 5' end towards the 3' end, which comprises self-replicating plasmids, genes and DNA or RNA polymers which are or are not infectious, functional or non-functional DNA or RNA, and fused DNA / RNA oligomers or polymers. In the nucleotide notation used in the present application, except where specifically mentioned, the left-hand end of a single-stranded nucleotide sequence is the 5' end.

As mentioned above, the invention involves the modulation of the production profile of at least one plant storage protein. Such plant storage proteins are well known per se and can be found in various parts of the plant, including the vascular system, the leaves, roots, stems, and of course, seeds.

Plants accumulate storage substances such as starch, lipids and proteins in certain phases of development. Storage proteins accumulate in both vegetative and reproductive tissues and serve as a reservoir to be used in later stages of plant development. The accumulation of storage protein is thus beneficial for plant development.

Plant storage proteins can be classified into two classes; seed storage proteins, known as SSPs, and vegetative storage proteins, known as VSPs.

SSPs are a set of proteins that accumulate in high levels in seeds during the late stages of seed development, whereas VSPs are proteins that accumulate in vegetative tissues such as leaves, cotyledons, stems and, depending on plant species, tubers.

VSPs accumulate in the vegetative tissues when excess resources are available and serve as a temporal reservoir of amino acids for use in subsequent phases of growth and development. A review of storage proteins can be found in "The Arabidopsis Book", 2002, under the heading "Storage Proteins" by Toru Fujiwaraa, Eiji Nambarab, Kazutoshi Yamagishib, Derek B. Gotoc, and Satoshi Naito.

Insofar as seed storage proteins are concerned, these are relatively well known and described in the art. A review of seed storage proteins is given in the article published in the journal The Plant Cell, Vol. 7, 945-956, July 1995, and entitled "Seed Storage Proteins: Structures and Biosynthesis" by Peter R. Shewry, Johnathan A. Napier, and Arthur S. Tatham. Excerpts from this review are given below for reference purposes.

Seed storage proteins were among the earliest of all proteins to be characterized. For example, wheat gluten was first isolated in 1745 (Beccari, 1745), and Brazil nut globulin was crystallized in 1859 (Maschke, 1859). However, the detailed study of seed storage proteins only started at the beginning of the twentieth century when Osborne (1924) classified them into groups on the basis of their extraction and solubility in water (albumins), dilute saline (globulins), alcohol/water mixtures (prolamins), and dilute acid or alkali (glutelins). The major storage protein groups include prolamins, 2S albumins, 7S globulins and 11S/12S globulins. Whereas the 2S albumin and globulin storage proteins are widely distributed in flowering plants, the prolamins are the major storage proteins of the Graminae, thereby including the major cereal plants. There are exceptions to this general rule, notably for rice, where the 11S globulin-like (glutelins) are the major storage proteins and prolamins are present in low levels. The other widely distributed groups of proteins include lectins, oil body protein (also known as oleosins), LEA proteins, proteases, protease inhibitors, antifungal proteins and thionins.

With regard to the plant seed storage protein that can be modified according to the present invention, a non-exhaustive list of known classes and examples of seed storage proteins is given below:
- 2S Albumins - soluble in water or dilute buffer at neutral pH, commonly found in dicotyledonous seeds and mainly studied in Crucifereae
   - Oilseed rape napins;
   - Arabidopsis napins or arabins;
   - Lupin conglutin δ;
   - Sunflower SFA8;
   - Castor bean albumins;
   - Sunflower albumins.
- Prolamins - of variable solubility in 50% to 90% aqueous alcohols, and the major storage proteins of cereal plants. Some occur in alcohol-insoluble polymers, but all are nevertheless alcohol-soluble in the reduced state.
   - Wheat S-rich prolamins (α-, β- and γ-gliadins and LMW-glutenins), S-poor prolamins (ω-gliadins) and HMW prolamins (HMW-glutenins);
   - Barley S-rich prolamins (β- and γ-hordeins), S-poor prolamins (C-hordein) and HMW prolamins (D-hordeins);
   - Rye S-rich prolamins (γ-secalins), S-poor prolamins (ω-secalins) and HMW prolamins (HMW-secalins);
   - Oat prolamins (avenins);
   - Maize prolamins (α-, β-, γ- and δ-zeins);
   - Sorghum prolamins (α-, β- and γ-kafirins);
   - rice prolamins;
   - Coix prolamins (α-coixin-a3A, α-coixin-a3B and β-coixins);
- 7S and 11S/12S Globulins - soluble in dilute alkali or acid solutions, the major storage proteins of dicots;
   - Bean phaseolins (trimeric vicilin-like 7S globulin) and lectin related proteins (arcelin, phytohemagglutinin, and α-amylase);
   - Soybean glycinin (11S globulin) and β-conglycinin (7S globulin);
   - Mungbean vicilin type (8S globulin), basic 7S globulin and legumin type (11S globulin);
   - Peanut arachin (legumin) and con-arachin (vicilin);
   - Arabidopsis and oilseed rape cruciferin (12S globulin);
   - Lupin conglutins β (7S globulins) and conglutins α (11S globulins);
   - Rice glutelins (belong to the 11S globulins) and α globulin (26kDa);
   - Oat 11-12S globulins;
   - Wheat triticin (proteins related to legumins) and leucosine;
- Oleosins (oil body protein) - hydrophobic proteins present in the membrane of oil bodies in the storage tissues of seeds (Huang *et al.,* 1987; Qu *et al.,* 1986; Vance and Huang, 1987). They are of low Mw (16-26 kDa), and are abundant in oilseeds. Within each seed species, there are usually two or more oleosins of different Mw. The two best-studied oleosins are oleosin 16 kDa and oleosin 18 kDa from maize embryo. Each oil body has a matrix of triacylglycerol (TAG) surrounded by a layer of phospholipids (PL) embedded with abundant structural proteins called oleosins. Oleosins have Mw values between 15kDa and 26kDa, depending on the isoforms and plant species in which they occur. They completely cover the surface of the oil bodies and prevent the PL layers of adjacent oil bodies from contacting and the oil bodies from coalescing. Maintaining the oil bodies as small individual entities provides a large surface area per unit TAG for lipase to act on during seed germination. Oleosins are abundant, amphipathic proteins present on the surface of oil bodies in seeds, tapetum, and other tissues.
- Lectins - capable of binding sugars and agglutinating red blood cell.
- Late Embryogenesis Abundant (LEA) proteins - highly hydrophilic proteins that accumulate late in seed development.

Since the amino acid sequences are available, some of the SSPs listed above have been regrouped in the cereal prolamin superfamily after sequence comparison. Among them, there are the prolamins of Triticae (wheat, barley and rye), the prolamins of maize (except for the α-zeins) and of other panicoid cereals (sorghum, pear millet and Job's tear), the prolamins of oats and rice and the 2S albumins of dicots. All proteins of this enlarged group, except C hordein and δ-zein, are characterized by the presence of three conserved regions, denoted A, B, and C in the Shewry review article at page 946. These three regions also show homology with each other and contain cysteine residues that may be conserved within or between the different groups of proteins. For example, the 2S albumins mentioned above all contain eight cysteine residues that are conserved in terms of context and position, including Cys-Cys and Cys-Xaa-Cys motifs, which are present in many of the other proteins.

As mentioned above, the prolamins of maize (known as the zeins), fall into four groups, three of which belong to the prolamin superfamily: β-, γ-, and δ-zein, in which both the β- and γ-zeins contains regions A, B, and C. The δ-zeins, do not contain repeats or any other distinguishing features, but homology with the prolamin superfamily can be inferred from some sequence identity with the 2S albumin of Brazil nut. The β-, γ-, and δ-zein (minor groups of zeins) are rich in cysteine and/or methionine, which are residues deficient in the α-zeins (major group of zeins). Consequently, the α-zeins are present in the grain as monomers or oligomer while the β-, γ-, and δ-zein form polymers. The α-zeins, which do not belong to the prolamin superfamily, are classified into two slightly different groups (Mw 19 kDa and Mw 22 kDa). They have similar structures consisting of unique N- and C-terminal domains flanking repeated sequences of about 20 amino acids residues, with nine such blocks present in the 19α-zein and ten in the 22α-zein. There is no evidence of homology with repeated sequences present in other prolamins, and the unique N- and C-terminal sequences do not appear to be related to any other protein. Thus, the α-zeins do not seem to be related to any other prolamins except the α-type prolamins of other panicoid cereals.

It is to be understood that the present invention also includes other storage proteins that are not mentioned in the above review, but which have since been determined to show similar structural and functional properties and relationships.

Broadly, storage proteins can be defined as any proteins produced in amounts sufficient to constitute a useful reserve. The storage proteins are degraded and mobilized at a later time, the catabolites supporting the anabolic metabolism associated with new growth. Most often this definition is applied to specific proteins found in abundance in seeds. However, it is becoming increasingly evident that particular proteins, called Vegetative Storage Protein (VSP), also can be defined as any proteins produced in amount to constitute a useful reserve and as any particular proteins which play a major role in vegetative nitrogen storage and cycling phenomena in both herbaceous annuals and woody perennials, cf. Plant Physiol. (1991) 97, 771-777, entitled "The 32-Kilodalton Vegetative Storage Protein of Salix microstachya Turz - Characterization and Immunolocalization" by Suzanne Wetzel and John S. Greenwood. So, specific proteins are synthesized and sequestered within particular vegetative cells in response to certain environmental, physiological, or developmental cues. These proteins are subsequently catabolized and mobilized from the vegetative tissues, the products supporting the anabolic metabolism associated with new, rapid growth. These proteins, therefore, have been classified as vegetative storage proteins.

For example, vegetative storage proteins (VSPs) were first described in leaves of depodded soybean plants (Wittenbach, 1982) and accumulated in soybean leaves to approximately 50% of the soluble proteins in sink-deprived plants (Wittenbach, 1983). Soybean has two types of VSPs, VSPa and VSPb (Staswick, 1988). Both VSPs are glycoproteins and share a high degree of sequence identity (about 80%) with a molecular mass of 27 kDa and 29 kDa, respectively, and carry phosphatase activity. These proteins are expressed in young seedlings, elongating hypocotyls, young leaves, stems, flowers and pods (Staswick, 1988). VSPs have also been described in a variety of other tissues, such as the tubers of potato and sweet potato (Wiltshire and Cobb, 1996, for review) and the bark of poplar (Lawrence et al., 1997; Zhu and Coleman 2001). These storage proteins accumulate in such tissues when excess photosynthates are produced.

Vegetative storage proteins have been isolated from various plant organs including soybean pods and leaves, potato and yam tubers, and roots of perennial weeds and from the wood, bark, and leaves of temperate trees. In general, they are not homologous to seed storage proteins of the same species. Vegetative storage proteins have been localized in protein-storage vacuoles within parenchymal cells of vegetative tissues, the organelles being similar in both ontogeny and structure to protein bodies found in mature dicotyledonous seed storage parenchymal cells.

In herbaceous annuals, vegetative storage protein accumulation is not restricted temporally. Rather, it is controlled by the nitrogen requirement of other portions of the plant. However, in perennial woody species the proteins appear to cycle annually. They are degraded and mobilized in the spring in support of early growth and accumulate in the fall either through increased synthesis or decreased degradation or mobilization.

Synthesis during the fall is presumably being supported by the catabolism of leaf proteins and mobilization of amino acids to the retained shoot tissues. In Salix, a protein of Mw 32 kDa acts as a vegetative storage protein accounting for approximately 30% of the total protein content of the bark tissue during the overwintering period and <5% of the total protein during summer. Accumulation of this storage protein coincides with the appearance of protein-storage vacuoles in the cambial cells, phloem parenchyma, and cortical parenchymal cells of the inner bark during the late summer and autumn.

As mentioned above, one of the objects of the present invention is a genetically modified plant cell comprising at least one stably integrated nucleic acid sequence that specifically modulates a production profile of at least one plant storage protein. The modulation may result in an increase or a decrease of the production profile of the plant storage protein. Examples of storage proteins across many different plant species have been given and described, and it is recognised that the vast majority share either structural similarity or functionality. It is to be noted that the present invention deals with genetically modified plant cells, plants and seeds produced therefrom. By "genetically modified", it is to be understood that the plant cell undergoes is subjected to a human or machine made process whereby a nucleic acid sequence in accordance with the invention is stably introduced into the genome of said plant. Techniques for carrying out such a process, often known as transformation, are well known to the skilled person. Such techniques may comprise, but are not limited to, biolistic transformation or particle bombardment, microinjection of DNA or RNA, vacuum infiltration, wounding, bacteria-mediated transformation techniques, such as the *Agrobacterium* mediated transformation technique described by Ishida *et al.* (1994). These techniques can also include transient transformation techniques, in which stable integration into the plant genome can be brought about by selection, cultivation, segregation and cross-breeding to inbreed the transgene into a stable plant line. Here again, such cross, top, and inbreeding techniques are well known and described in the literature.

In accordance with a particularly preferred feature of the invention, the integrated nucleic acid sequence specifically modulates a production profile of at least one plant storage protein. It is to be understood here that the modulation of the production profile of at least one plant storage protein may involve either increasing or decreasing production of said storage protein to any desirable level, depending on the intended result to be achieved. In a particularly preferred embodiment, the modulation involves reducing production of said at least one storage protein to the extent that it is effectively silenced, in other words, no expression takes place. Techniques for silencing genes are also well known in the art in general, and can involve, for example:
- using inverted nucleic acid sequences coding for the storage protein that are misread in the open reading frame;
- or any other suitably well known technique, such as RNA interference, also known as RNAi;
- disrupting translation, and optionally transcription, of the sequence coding for the storage protein.

In a particularly preferred embodiment of the present invention, the at least one stably integrated nucleic acid sequence comprises a first nucleic acid sequence that interferes directly with expression of the at least one plant storage protein. In an equally preferred alternative embodiment, the at least one stably integrated nucleic acid sequence comprises a first nucleic acid sequence that interferes indirectly with expression of the at least one plant storage protein.

In an alternative and still preferred embodiment, the modulation of the production profile of at least one plant storage protein occurs through the use of direct or indirect trans-dominant transcriptional factors. Other alternative and equally preferred embodiments involve modulation of the production profile of at least one plant storage protein through direct action of the first nucleic acid sequence on plant promoter activity, or direct or indirect action on translational processing.

Most preferably, the at least one stably integrated nucleic acid sequence comprises a first nucleic acid sequence that interferes directly or indirectly with expression of the at least one plant storage protein, and is associated with a second nucleic acid sequence that codes for a heterologous molecule. In such a case, the heterologous molecule coded for by the second nucleic acid sequence is preferably selected from the group consisting of a therapeutically active polypeptide, a cosmetically active molecule, and a nutraceutical. The association may be obtained in well known manners per se, for example, by fusing the nucleic acid sequences together, co-integrating the nucleic acid sequences into the plant genome on the same or different vectors, or in the same or different expression cassettes, crossing of plants, for example, by cross-pollination, grafting, or other well known methods, of a first plant expressing a first nucleic acid modulating the storage protein production profile with a second plant expressing the nucleic acid coding for a desirable heterologous molecule.

In a more preferred embodiment, the heterologous molecule is a therapeutically active polypeptide having an activity selected from the group consisting of digestive, pancreatic, biliary, antiviral, anti-inflammatory, pulmonary, antimicrobial, haematological, neurological, cardiovascular, ophthalmic, antigenic, cerebral, immunostimulating, and immunomodulating activities.

Most preferably, the polypeptide is selected from the group consisting of insulins, interferons, gastric, pancreatic or biliary lipases, elastases, anti-proteases such as alpha-1 anti-trypsin, structure-forming proteins such as collagen, transferrins such as lactoferrin, blood-derived proteins, such as human haemoglobin or albumin, and blood cofactors, antioxidants such as superoxide dismutase, antibodies, antibody fragments and antigens. In a particularly preferred embodiment, the therapeutically active polypeptide is an antibody.

In accordance with another preferred feature of the present invention, the first nucleic acid sequence interfering directly or indirectly with the at least one storage protein production profile is selected from the group consisting of the nucleic acid sequences identified by the SEQ.ID numbers SEQ.ID01, SEQ.ID02, SEQ. ID03, SEQ.ID04, SEQ.ID05, SEQ.ID06, SEQ.ID13, SEQ.ID14, SEQ.ID15, SEQ.ID16, SEQ.ID17, SEQ.ID18, SEQ.ID19, SEQ.ID20, SEQ.ID21, SEQ.ID22, SEQ.ID23, SEQ.ID24, SEQ.ID25, SEQ.ID26, SEQ.ID27, SEQ.ID28, SEQ.ID29, SEQ.ID30, SEQ.ID33, SEQ.ID34, SEQ.ID35, SEQ.ID36, SEQ. ID37, SEQ.ID38, SEQ.ID39, SEQ.ID44, SEQ.ID45, SEQ.ID50, SEQ.ID51, SEQ.ID52, SEQ.ID53, SEQ.ID54, SEQ.ID56, SEQ. ID57, SEQ.ID58, SEQ.ID60, and SEQ.ID63, their complementary sequences, or sequences that hybridize thereto under stringent conditions. Particularly preferred is where the at least one nucleic acid sequence modulates a seed storage protein production profile and is selected from the group consisting of the nucleic acid sequences identified by the SEQ.ID numbers SEQ. ID01, SEQ. ID02, SEQ.ID03, SEQ.ID04, SEQ.ID05, SEQ.ID06, SEQ.ID13, SEQ.ID14, SEQ. ID15, SEQ.ID16, SEQ.ID17, SEQ.ID18, SEQ.ID19, SEQ.ID20, SEQ.ID21, SEQ.ID22, SEQ.ID23, SEQ.ID24, SEQ.ID25, SEQ.ID26, SEQ.ID27, SEQ.ID28, SEQ.ID29, SEQ.ID30, SEQ.ID33, SEQ.ID34, SEQ.ID35, SEQ.ID36, SEQ. ID37, SEQ.ID38, SEQ.ID39, SEQ.ID44, SEQ.ID45, SEQ.ID50, SEQ.ID51, SEQ.ID52, SEQ.ID53, SEQ.ID54, SEQ.ID56, SEQ. ID57, SEQ.ID58, SEQ.ID60, and SEQ.ID63, their complementary sequences, or sequences that hybridize thereto under stringent conditions .

In yet another preferred aspect of the invention, the at least one plant seed storage protein is a zein. Preferably, the plant seed storage protein is selected from the group consisting of a gamma zein, an alpha zein, a beta zein, a delta zein, or combinations thereof.

The production profile of said at least one storage protein preferably shows reduced production of said at least one storage protein compared to a corresponding non-genetically modified plant cell. Alternatively, and still preferably, the production profile of said at least one storage protein shows substantial depletion of said at least one storage protein compared to a corresponding non-genetically modified plant cell.

Even more preferably, the production profile of said at least one storage protein shows a reduction greater than or equal to 90% in production of said at least one storage protein compared to a corresponding non-genetically modified plant cell. Other preferred values of reduction in the production profile of said at least one storage protein are greater than or equal to 75%, 50% or 25%, compared to a corresponding non-genetically modified plant cell.

In particularly preferred embodiments of this aspect of the invention, the production profile of the said at least one seed storage protein shows a reduction:
- of up to about 100% in production of gamma zein compared to a corresponding non-genetically modified plant cell, or;
- of up to about 95% in production of alpha zein compared to a corresponding non-genetically modified plant cell, or;
- of between about 95% and about 50%, but preferably, up to about 50% in production of beta zein compared to a corresponding non-genetically modified plant cell, or;
- of up to about 80% in production of delta zein compared to a corresponding non-genetically modified plant cell, or;
- a combination of at least any two of the above.

In a most preferred embodiment, the production profile of said at least one seed storage protein is as follows compared to a corresponding non-genetically modified plant cell:
- gamma zein : between about 0% to about 15% of total zein content;
- delta zein : about 0,08% to about 0.5% of total zein content;
- alpha zein : between about 5% to about 60% of total zein content;
- beta zein : about 0.25% to about 1.25% of total zein content.

In yet another aspect of the present invention, the modulation of the production profile of said at least one storage protein causes the cell to produce and store an increased amount of heterologous molecule compared to a corresponding genetically modified plant cell producing the same molecule without the modulated production profile of said at least one storage protein.

According to still yet another object of the invention, the inventors have provided a genetically modified plant comprising genetically modified plant cells as described above. Such plants can be obtained using known techniques, for example, cultivating the cells in a suitable media to induce cell multiplication to form calli, and then separating the calli into smaller groups of cells, and changing the growth media to induce bud formation, then root, and thereby obtain a plantlet, which can be grown on, for example, supplemented Murashige and Skoog media (MS media) to a size and height sufficient for it to be replanted in soil.

According to still yet another object of the invention, the inventors have provided a genetically modified plant comprising genetically modified plant cells as described above. Such plants can be obtained using known techniques, for example, cultivating the cells in a suitable media to induce cell multiplication to form calli, and then separating the calli into smaller groups of cells, and changing the growth media to induce somatic embryogenesis and then germinate the said embryo to obtain a plantlet which can be grown on the appropriate media to a size and height sufficient for it to be replanted in soil.

Still yet another object is a genetically modified plant seed obtained, or obtainable, from a genetically modified plant or modified plant cells as previously described. Such seeds can be obtained by cultivating the genetically modified plants as described above, and then when the plant flowers, pollinating said plant with its own pollen if such is produced, or with the pollen of another plant of the same or similar crossable species, to induce seed formation in the plant, and then harvest the seeds once formed. These steps can be reproduced as often as necessary to obtain a homozygous seed bank consisting of the genetic modification. As a consequence, other objects of the present invention will become apparent, and notably a method for the production of a genetically modified plant cell comprising:
- stably introducing into the genome of said plant cell at least one nucleic acid sequence that specifically modulates a production profile of at least one plant storage protein;
- cultivating said plant cell.

In addition, the invention also provides a method for the production of a genetically modified plant seed comprising:
- cultivating a genetically modified plant cell as described to yield a plant;
- pollinating the genetically modified plant obtained in the preceding step;
- harvesting the seeds obtained from the pollinated plant;
- optionally selecting the genetically modified seeds from said plant.

In a most preferred embodiment of the above method, the selection step can be carried out on the basis of the degree of opacity of the genetically modified seed. Other preferred and further embodiments are to be found in the properties of the genetically modified seeds of the invention maintaining germinative capability, and preferably displaying a germination success rate comprised between about 90% and about 100%. In addition, the genetically modified plant cell, plant, or plant seeds according to the present invention display the same resistance to abiotic and / or biotic stresses as a wild-type non-genetically modified correspondent.

In all of the above objects, the plant cell or plant seed is preferably selected from a dicotyledonous or monocotyledonous plant species. More preferably, the plant cell or plant seed is selected from the plant families consisting of Solanaceae, Cruciferae, Cucurbitaceae, Brassicae, and Graminae. In yet another preferred embodiment, the plant cell or plant seed is selected from the group consisting of arabidopsis, tobacco, potato, alfalfa, lettuce, tomato, bean, castor bean, lupin, pea, carrot, beetroot, turnip, parsnip, banana, pumpkin, melon, lucerne, soya, sorghum, barley, cotton, rice, wheat, maize, rye and oats. Even more preferably, the plant seed of the invention is homozygous for the at least one stably integrated nucleic acid sequence that specifically modulates a production profile of at least one plant storage protein.

The following figures and detailed examples will serve to further illustrate the invention, it being understood that these figures and examples are non-limiting.

### Brief Description of the Figures

Figure 1 represents a plasmid designated pMRT1719, created by insertion of an O2 PCR fragment into the *Eco*RV site of the pBluescriptII SK+ vector, illustrated by Figure 3.
Figure 2 represents a plasmid designated pMRT1745, created by cloning a chimeric 19/22αZein PCR fragment into the *Eco*RV site of the pBluescriptII SK+ vector, illustrated by Figure 3.
Figure 3 represents the pBluescriptII SK+ vector.
Figure 4 represents a plasmid designated pMRT1631, and is a modified pBluescriptII SK+ plasmid of Figure 3 in which the IV2 intron was cloned into the *Eco*RV restriction site.
Figure 5 represents a plasmid designated pMRT1144 described by Norre *et al*., 2002 and from which the IV2 intron was amplified by PCR amplification.
Figure 6 represents a binary vector designated as pMRT1611 and constructed by inserting the full length γ-zein promoter (pγZ, Reina *et al.,* 1990) and the nopaline synthase terminator (T*nos*) of *Agrobacterium tumefaciens* ([p*γ*Z*-Tnos*] fragment) into the *XhoI* digested pMRT1195, illustrated by Figure 7.
Figure 7 represents the binary vector designated pMRT1195 described by Comeau and Gruber, 2001.
Figure 8 represents the plasmid pMRT1276 described by Norre (2002) used for obtaining by SOE technique the [pγZ-*Tnos*] fragment of plasmid pMRT1611 illustrated by Figure 6.
Figure 9 represents the plasmid designated as pMRT1630 resulting from cloning of the γ-zein PCR fragment into the *Eco*RV site of the pBluescriptII SK+ vector of Figure 3.
Figure 10 represents a plasmid designated pMRT1632 which was obtained by successively cloning the γZein fragment from the plasmid pMRT1630 of Figure 9 upstream and downstream of the IV2 intron of the plasmid of Figure 4, using the *Eco*RI and *Sal*I restriction sites respectively, to create an inverted repeat.
Figure 11 represents a plasmid designated pMRT1655 obtained by excising the sequence containing the γZein inverted repeat and the IV2 intron from the plasmid pMRT1632 of Figure 10 with *Sma*I and *Xho*I, and then cloning this sequence into the *Stu*I site of the plasmid pMRT1611 of Figure 6.
Figure 12 represents a plasmid designated pMRT1751 which contains the *Sac*I/*Eco*RI and *Hind*III/*Xho*I chimeric 19/22αZein fragments isolated from pMRT1745 of Figure 2, cloned upstream and downstream of the IV2 intron in the corresponding sites of pMRT1631 of Figure 4, in order to create an inverted repeat.
Figure 13 represents a plasmid designated pMRT1774 resulting from the digestion of the plasmid pMRT1751 of Figure 12 by *Sac*I to isolate the chimer 19/22αZein inverted repeat surrounding the IV2 intron fragment, and blunted insertion thereof into the *Stu*I site of pMRT1611 of Figure 6 to give pMRT1774.
Figure 14 represents a plasmid designated pMRT1772, created by cloning of an O2ΔbZIP PCR fragment into the *EcoRV* site of the pBluescriptII SK+ vector of Figure 3.
Figure 15 represents a plasmid designated pMRT1793, obtained by excising the *Kpn*I/*Eco*RI O2ΔbZIP fragment from pMRT1772 (Figure 14), and cloning it successively upstream and downstream of the IV2 intron from pMRT1631 (Figure 4) into the EcoRl and Sall sites respectively, to create an inverted repeat.
Figure 16 represents a plasmid designated pMRT1802 containing the O2ΔbZIP inverted repeat fragment of Figure 15. The O2ΔbZIP inverted repeat fragment was isolated from the plasmid pMRT1793 of Figure 15 by *Sal*I/*Eco*RI, and cloned into the *Stu*I site of the plasmid pMRT1611 of Figure 6.
Figure 17 represents a plasmid designated pMRT1729, created by cloning of an O2ΔBD PCR fragment into the *EcoRV* site of the pBluescriptII SK+ vector of Figure 3.
Figure 18 represents a plasmid designated pMRT1794, containing a *Hind*III O2ΔBD fragment, excised from pMRT1729 of Figure 17, and then cloned into the *Stu*I site of pMRT1611 of Figure 6.
Figure 19 is a schematic representation of some of the inverted repeats created in the present invention.
Figure 20, panel A represents schematically, and in descending order, from upper to lower, the modular organisation of:
   - the full-length O2 transcription factor (upper schema);
   - the same without the bZIP domain (O2ΔbZIP) (middle schema);
   - the same without the DNA binding domain (lower schema).

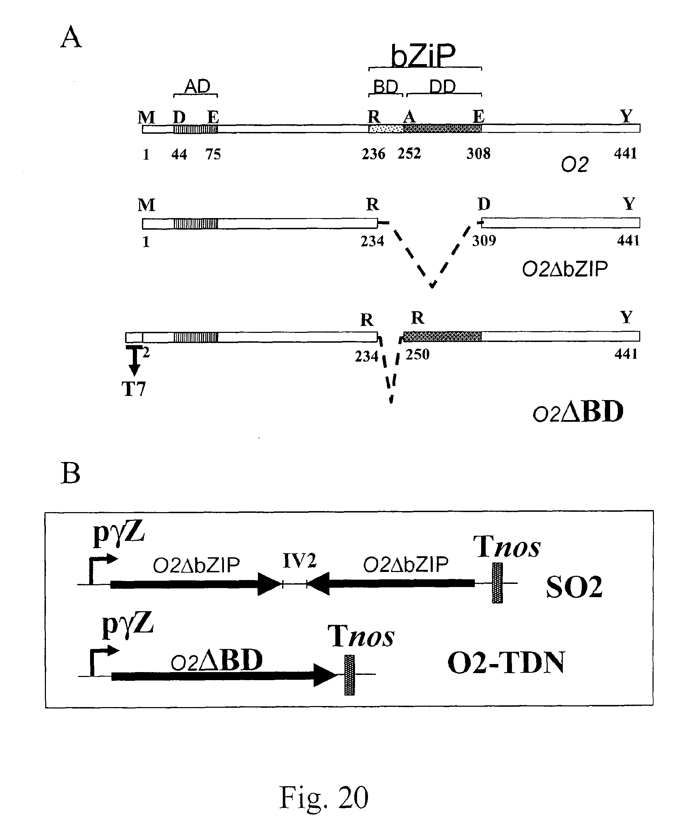

Figure 20 , panel B represents yet another schema of two expression cassettes used in the present invention, i.e. an inverted repeat sequence of O2ΔbZIP, designated as the SO2 construct (upper schema), and O2 without the DNA binding domain created for a trans-dominant suppression approach, i.e. the O2-TDN construct.
   In all of the following figures, where present, the molecular weights of each protein are indicated with their corresponding name alongside the stains or blots.

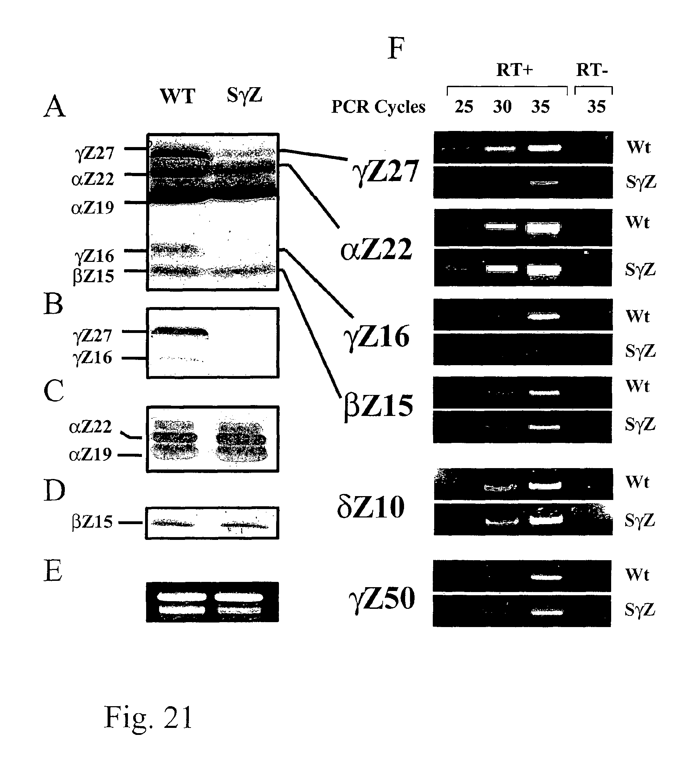

Figure 21 represents analytical data from 18 DAP seeds of SγZ mutant maize line and showing:
   - in panel A, Coomassie Blue staining;
   - in panel B, an immunoblot using anti-γZein antibodies;
   - in panel C, an immunoblot using anti-αZein antibodies;
   - in panel D, an immunoblot using anti-βZein antibodies;
   - in panel E, total RNA analysis;
   - in panel F, RT-PCR analysis, where RT+ indicates presence of reverse transcriptase, and RT- indicates that no reverse transcriptase was used in the reaction mixture;
   where the SγZ mutant seed is compared to a Wild Type (WT) seed from the same cob of maize having both transgenic and non-transgenic seeds.

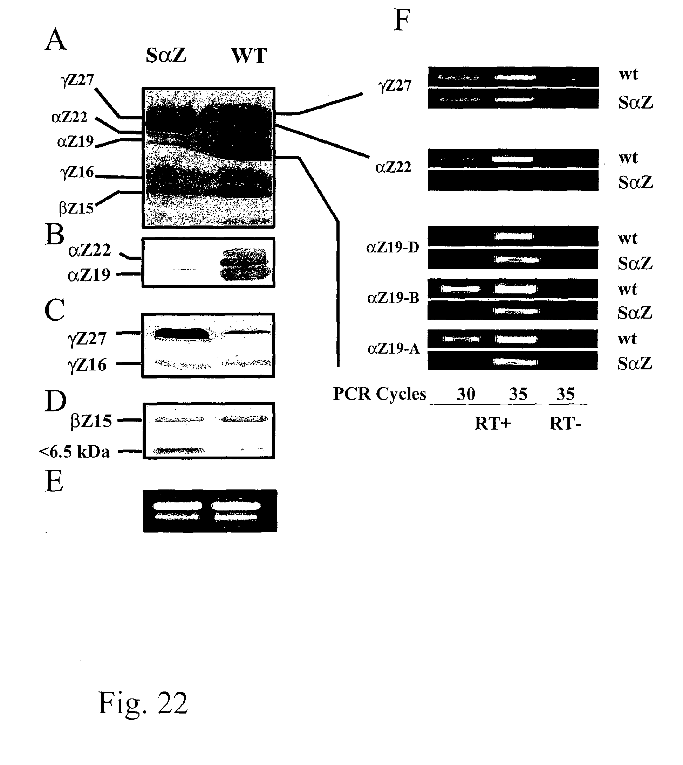

Figure 22 represents analytical data from 18 DAP seeds of SαZ mutant maize line and showing:
   - in panel A, Coomassie Blue staining;
   - in panel B, an immunoblot using anti-αZein antibodies;
   - in panel C, an immunoblot using anti-γZein antibodies;
   - in panel D, an immunoblot using anti-βZein antibodies;
   - in panel E, total RNA analysis;
   - in panel F, RT-PCR analysis, where RT+ indicates presence of reverse transcriptase, and RT- indicates that no reverse transcriptase was used in the reaction mixture;
   where the SαZ mutant seed is compared to a Wild Type (WT) seed from the same cob of maize having both transgenic and non-transgenic seeds.

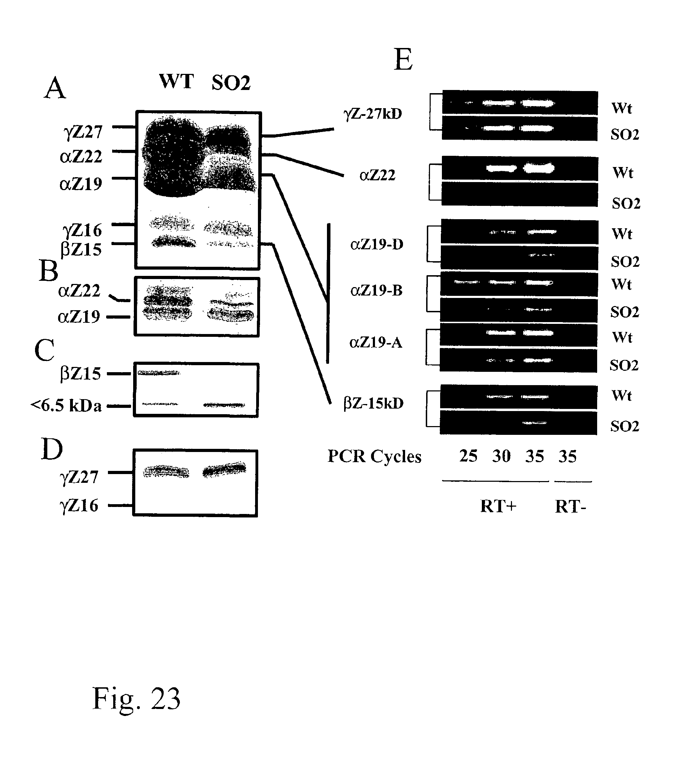

Figure 23 represents analytical data from 18 DAP seeds of SO2 mutant maize line and showing:
   - in panel A, Coomassie Blue staining;
   - in panel B, an immunoblot using anti-αZein antibodies;
   - in panel C, an immunoblot using anti-βZein antibodies;
   - in panel D, an immunoblot using anti-γZein antibodies;
   - in panel E, RT-PCR analysis, where RT+ indicates presence of reverse transcriptase, and RT- indicates that no reverse transcriptase was used in the reaction mixture;
   where the SO2 mutant seed is compared to a Wild Type (WT) seed from the same cob of maize having both transgenic and non-transgenic seeds.

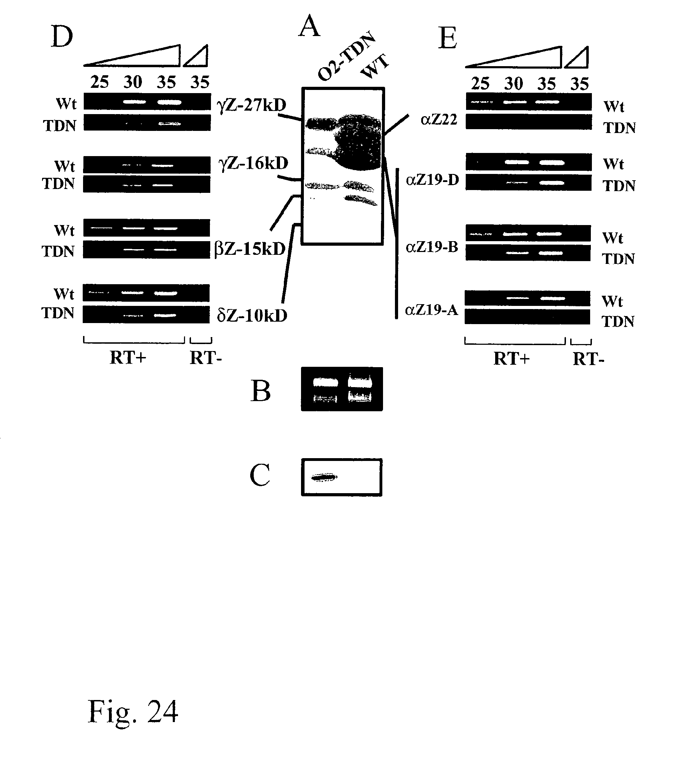

Figure 24 represents analytical data from 18 DAP seeds of O2-TDN mutant maize line and showing:
   - in panel A, Coomassie Blue staining;
   - in panel B, total RNA analysis;
   - in panel C, an immunoblot using anti-T7 antibodies;
   - in panel D, RT-PCR analysis of 16γZ, 27γZ, 15βZ, and 10δZ mRNAs, where RT+ indicates presence of reverse transcriptase, and RT- indicates that no reverse transcriptase was used in the reaction mixture;
   - in panel E, RT-PCR analysis of 19αZ (A,B,D families), and 22αZ mRNAs, where RT+ indicates presence of reverse transcriptase, and RT- indicates that no reverse transcriptase was used in the reaction mixture;
   where the O2-TDN mutant seed is compared to a Wild Type (WT) seed from the same cob of maize having both transgenic and non-transgenic seeds.

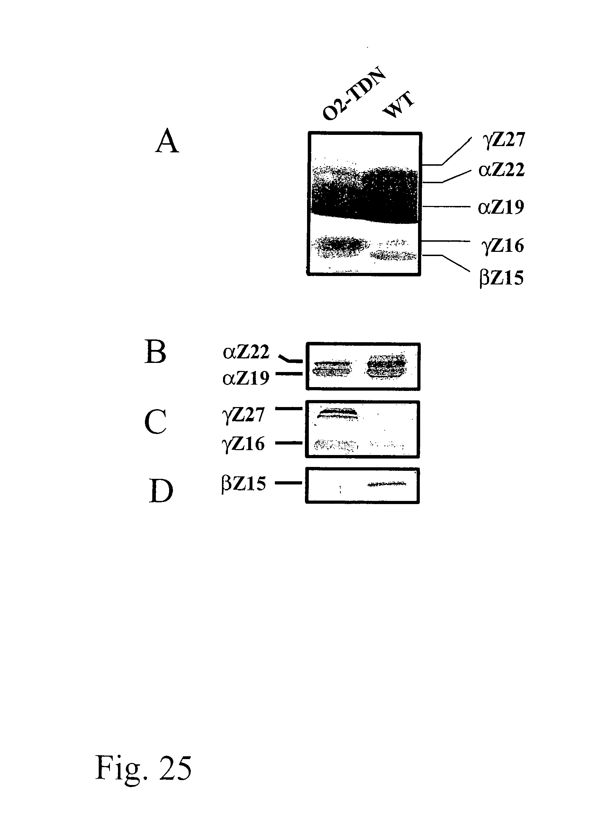

Figure 25 represents analytical data from mature seeds of O2-TDN mutant maize line and showing:
   - in panel A, Coomassie Blue staining;
   - in panel B, an immunoblot using anti-αZein antibodies;
   - in panel C, an immunoblot using anti-γZein antibodies;
   - in panel D, an immunoblot using anti-βZein antibodies;
   where the O2-TDN mutant seed is compared to a Wild Type (WT) seed from the same cob of maize having both transgenic and non-transgenic seeds.

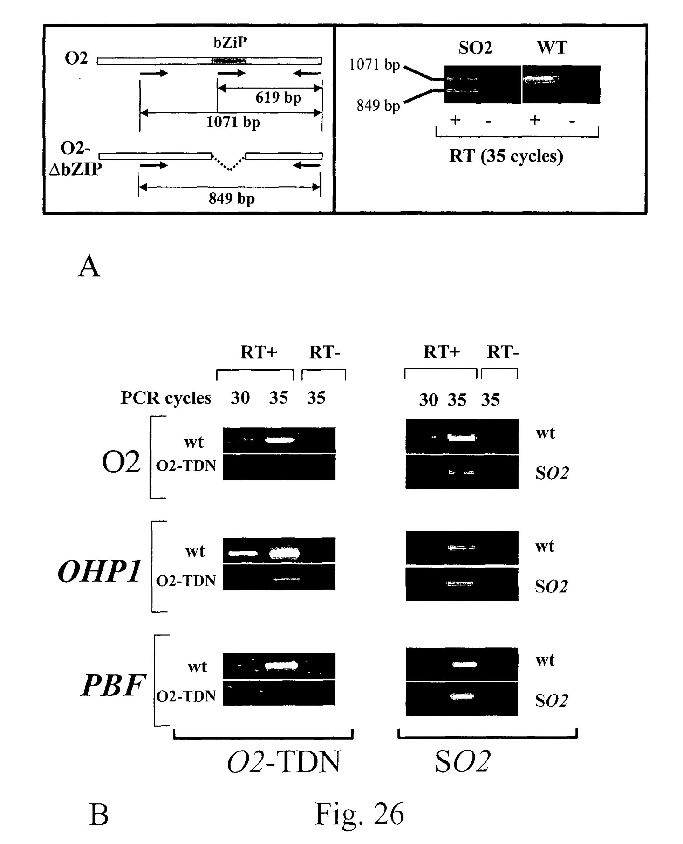

Figure 26 represents:
   - in panel A, schematically on the left the position of primers on the full-length O2 sequence (upper schema) and the O2ΔbZIP sequence (lower schema). On the right of the same figure are shown corresponding RT-PCR analyses from O2 mRNAs in mutant SO2 and wild-type seeds, where (+) indicates presence of reverse transcriptase, and (-) indicates that no reverse transcriptase was used in the reaction mixture;
   - in panel B, RT-PCR analyses from O2, OHP1 and PBF mRNAs in mutant O2-TDN (left-hand side) and mutant SO2 (right-hand side) plants according to the invention compared to the corresponding respective wild-type seeds, where RT+ indicates presence of reverse transcriptase, and RT- indicates that no reverse transcriptase was used in the reaction mixture;

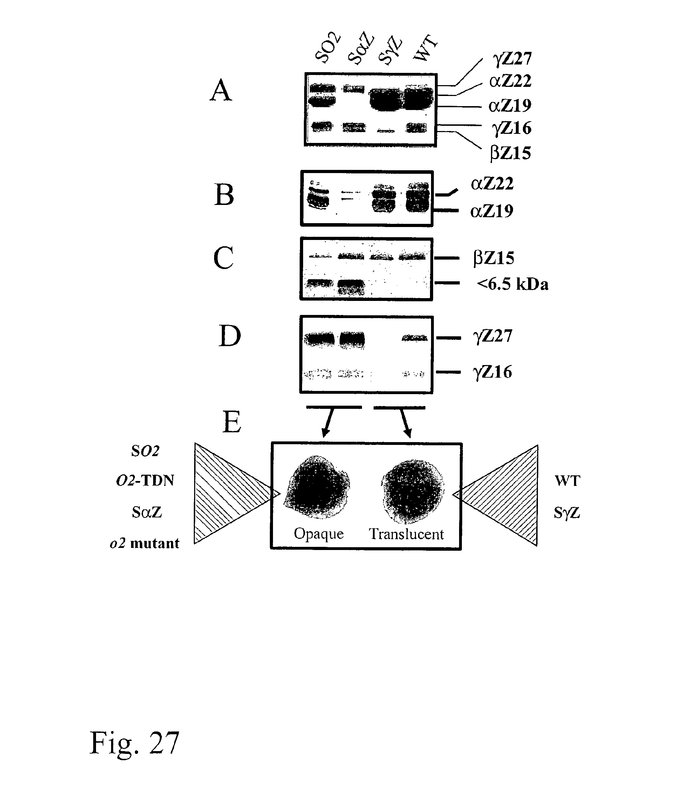

Figure 27 represents analytical data from mature seeds of SO2, SαZ, and SγZ mutant maize line and showing:
   - in panel A, Coomassie Blue staining;
   - in panel B, an immunoblot using anti-αZein antibodies;
   - in panel C, an immunoblot using anti-βZein antibodies;
   - in panel D, an immunoblot using anti-γZein antibodies;
   - in panel E, the photograph on the left illustrates an opaque phenotype observed in the SO2, SαZ and O2-TDN seeds, whereas the photograph on the right, illustrating a translucent phenotype observed in the SγZ and Wild Type (WT) seeds.

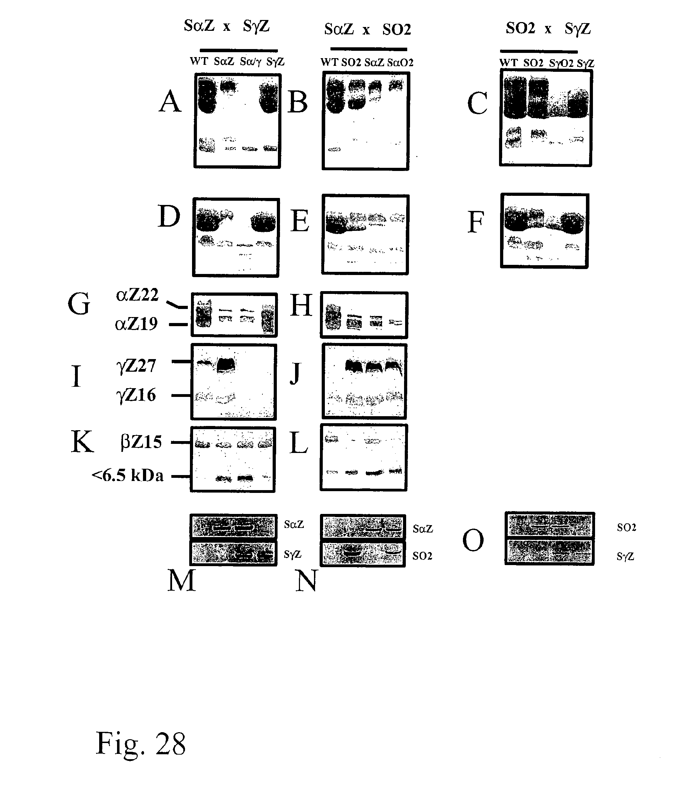

Figure 28 represents:
   - in panels A, B, and C, a series of Coomassie blue stains (18 DAP seeds);
   - in panels D, E, and F, a series of Coomassie blue stains (mature seeds);
   - in panels G and H, immunoblots using anti-αZein antibodies;
   - in panels I and J, immunoblots using anti-γZein antibodies;
   - in panels K and L, immunoblots using anti-βZein antibodies;
   - in panels M, N and O, PCR screening of the transgenes SαZ, SγZ, and SO2;
   where the leftmost column represents the sexual crossing of the SαZ and SγZ mutant plants, the middle column represents the sexual crossing of the SαZ and SO2 mutant plants, and the rightmost column represents sexual crossing of the SO2 and SγZ mutant plants;
   and where each panel displays a comparison of the single mutant seed to the wild type WT and respective double mutant seed.

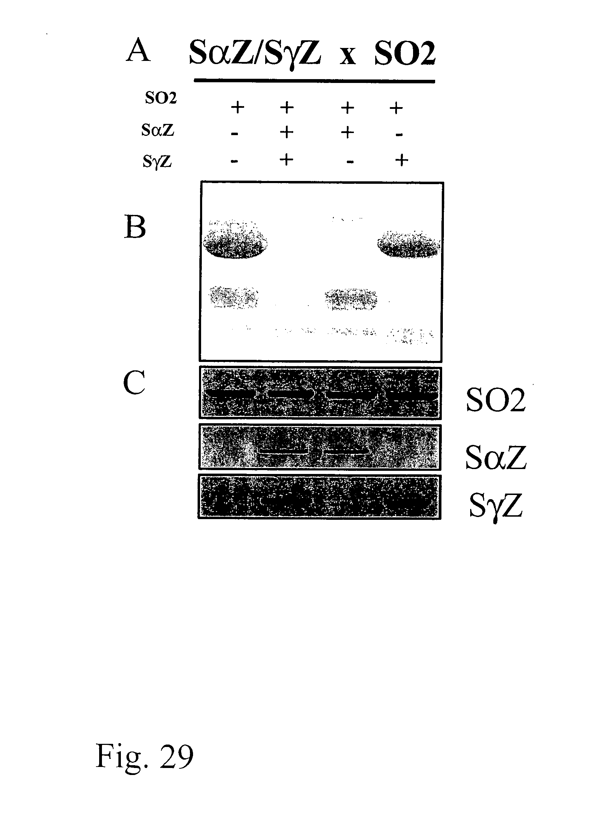

Figure 29 represents mature seed analysis of the progeny seeds of the maize lines obtained by crossing the double mutant SαZ/SγZ plants with the single SO2 mutant plants:
   - panel A is a synopsis of the results of the images presented below it and illustrating the genetic backgrounds;
   - panel B is a Coomassie blue stain from mature seeds of the zein pattem;
   - panel C is a PCR screening of the SO2, SαZ and the SγZ transgenes respectively from top to bottom.

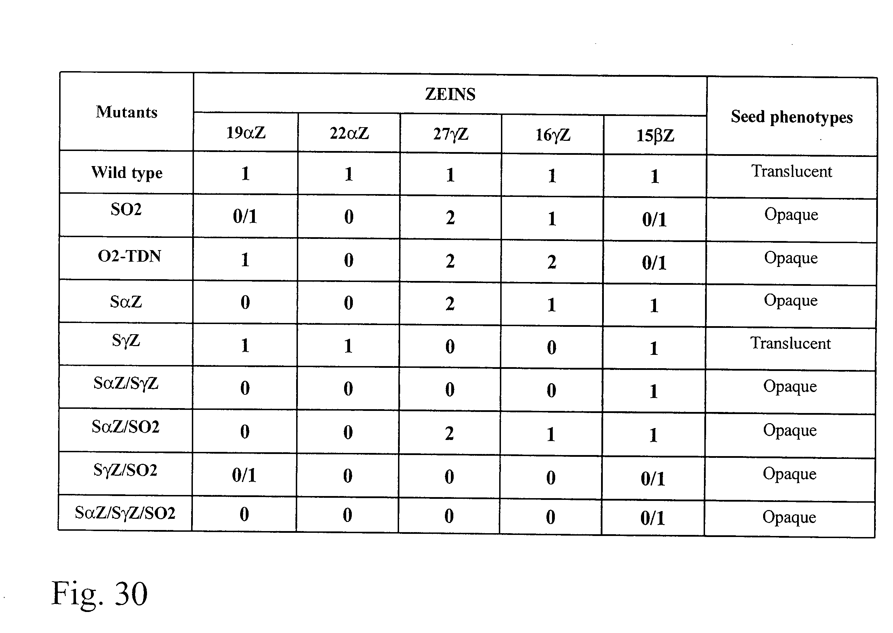

Figure 30 represents a comparison of the impact of each transgene and of the association of different transgenes in zein accumulation in maize endosperm, where the values"0", "1 ", "2", and "0/1" indicate a relative quantity of a given zein produced in a mutant seed when compared to a corresponding respective wild type seed, "1" being an identical quantity in the mutant, "2" being a greater quantity in the mutant, "0" tending to no zein production in the mutant, and "0/1" indicating a significant reduction in zein production of the mutant compared to the wild type.

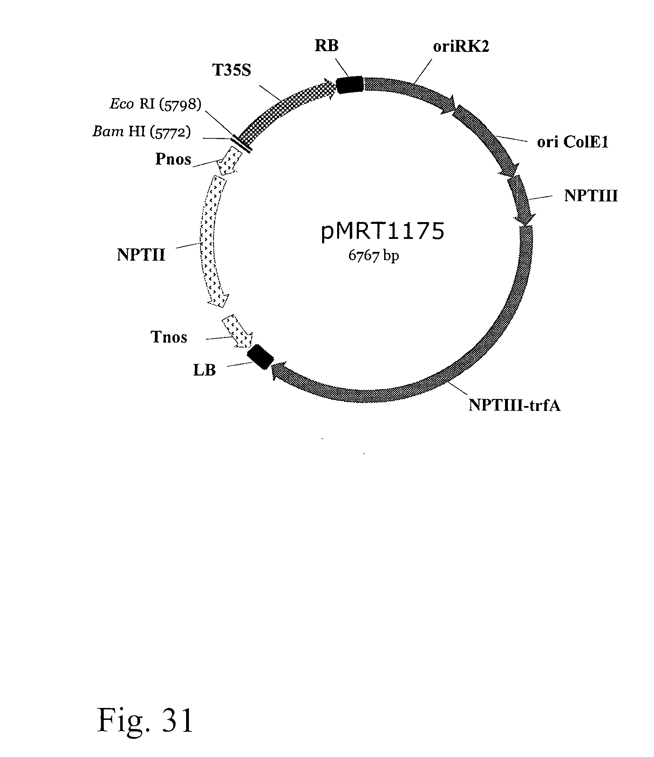

Figure 31 represents the binary vector designated pMRT1175 described by Comeau and Gruber, 2001.

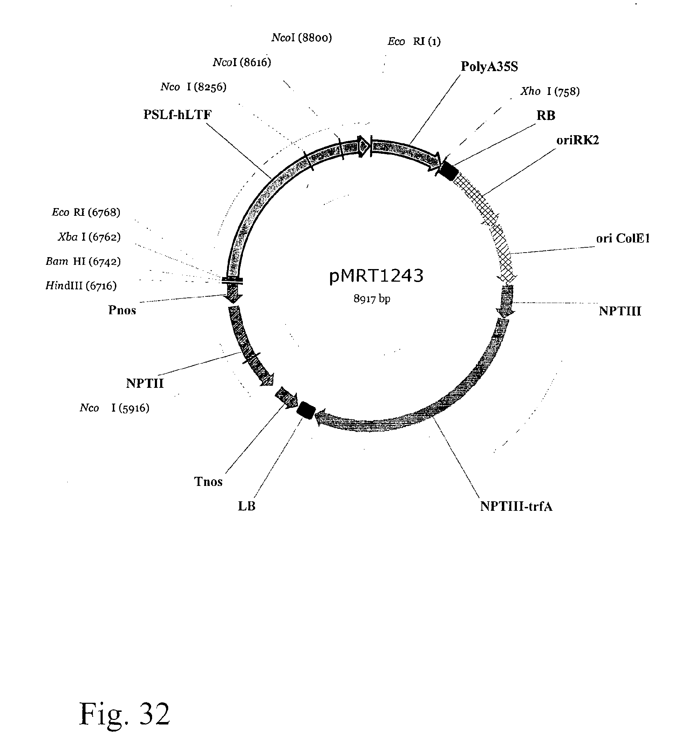

Figure 32 represents the vector pMRT1243 obtained by isolating an *Eco*RI fragment from pSB-Lf12 (PSLf-hLTF) and cloned into an *Eco*RI site in the pMRT1175 vector of Figure 31.

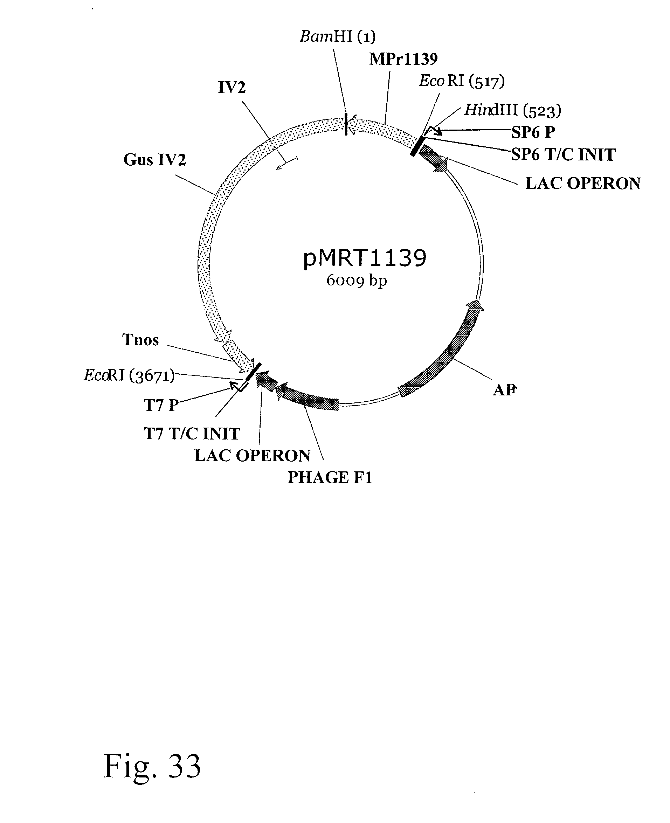

Figure 33 represents the plasmid designated pMRT1139 described by Norre et al, 2001.

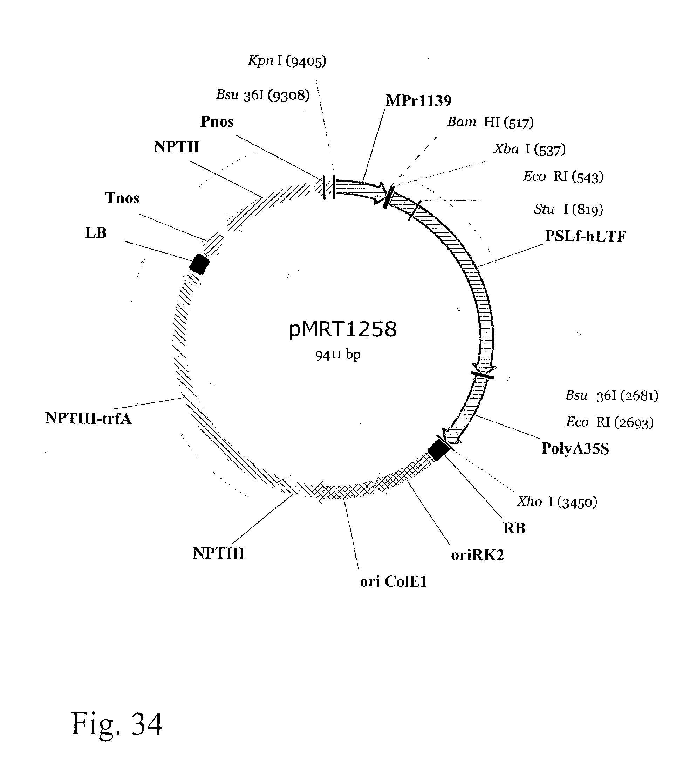

Figure 34 represents the plasmid pMRT1258 which was obtained by cloning of the blunt *Eco*RI*-Bam*HI fragment from the plasmid pMRT1139 of Figure 33 into the end-filled *Hin*dIII-*Bam*HI sites of plasmid pMRT1243 of Figure 32.

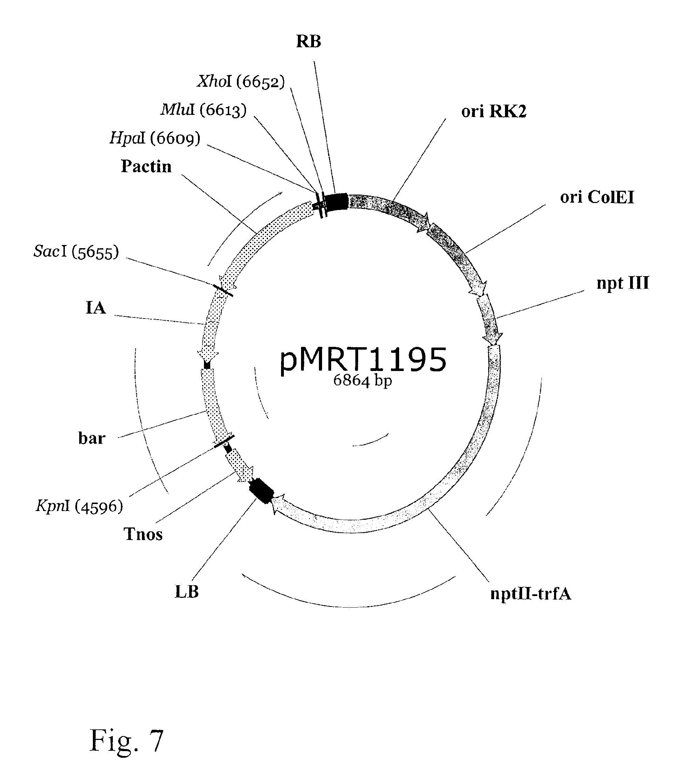

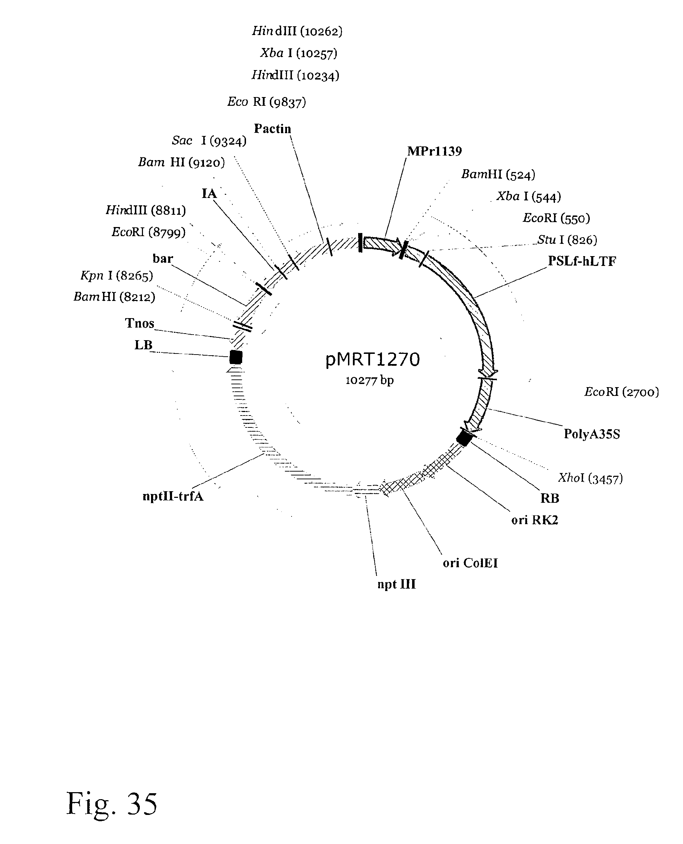

Figure 35 represents the plasmid pMRT1270 which was obtained by cloning the blunt *Kpn*I*-Xho*I fragment (MPr1139 - PSLf - hLTF - polyA35S) from pMRT1258 (Figure 34) into the *Hpa*I*-Xho*I sites of pMRT1195 of Figure 7.

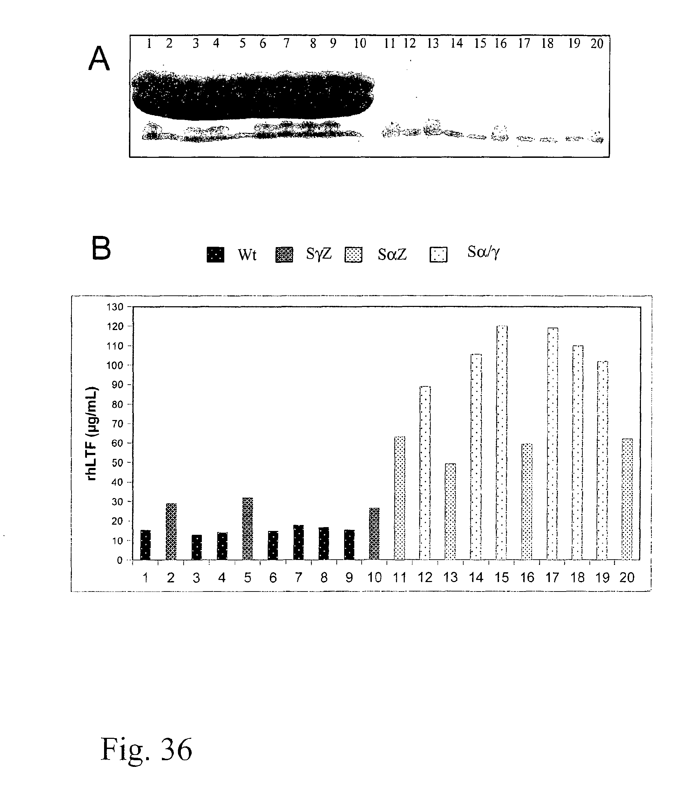

Figure 36 represents analytical data from mature seeds of SαZ/SγZ/rhLTF maize line and showing:
   - in panel A, Coomassie Blue staining obtained from individual mature seeds;
   - in panel B, a graph of the quantity of recombinant human lactoferrin produced in each corresponding seed of panel A.

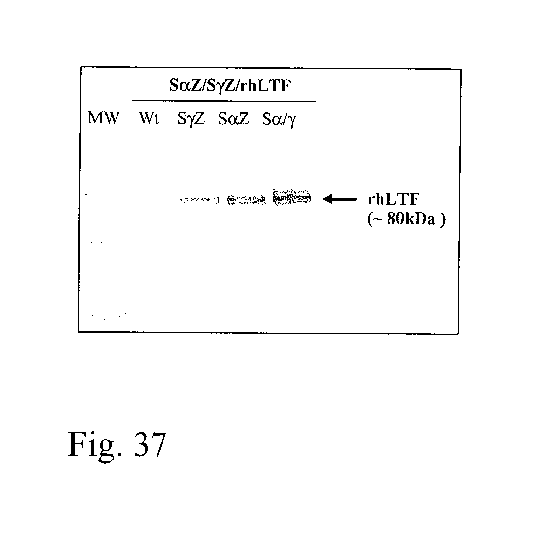

Figure 37 represents an anti-lactoferrin immunoblot obtained from four seeds selected from the various segregated genetic backgrounds of the maize seeds analysed in Figure 36.

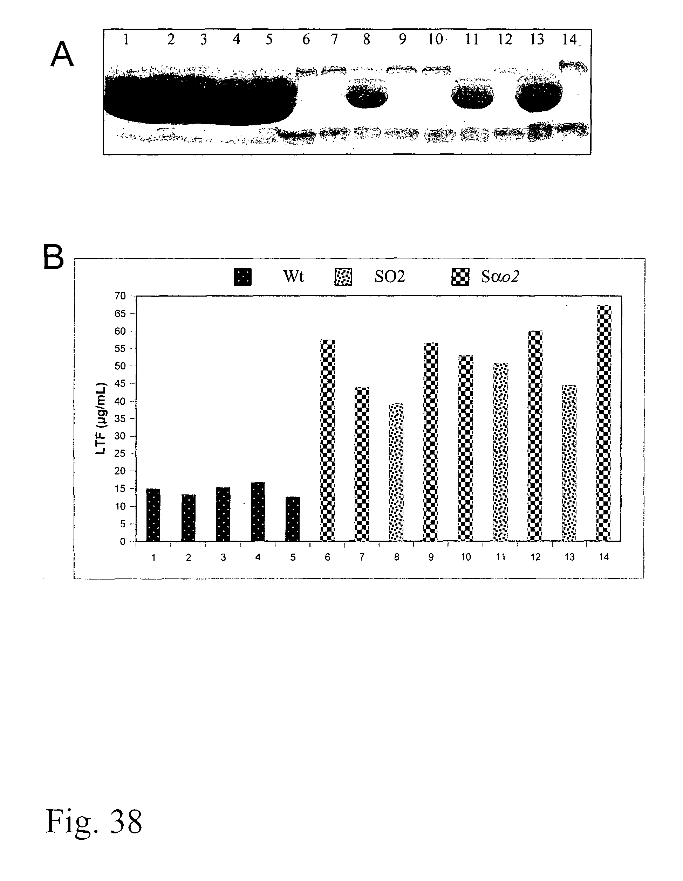

Figure 38 represents analytical data from mature seeds of SαZ/SO2/rhLTF maize line and showing:
   - in panel A, Coomassie Blue staining obtained from individual mature seeds;
   - in panel B, a graph of the quantity of recombinant human lactoferrin produced in each corresponding seed of panel A.

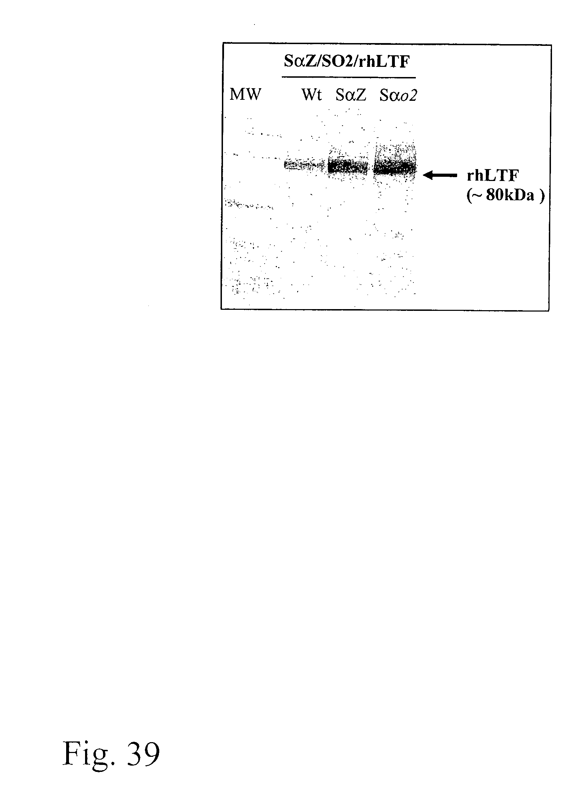

Figure 39 represents an anti-lactoferrin immunoblot obtained from three seeds selected from the various segregated genetic backgrounds of the maize seeds analysed in Figure 38.

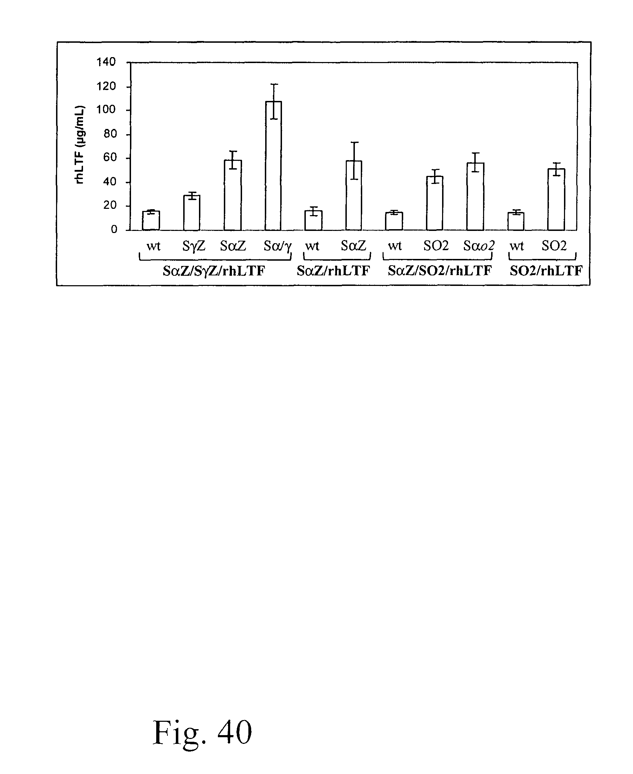

Figure 40 represents a graph of the comparative average quantities of recombinant lactoferrin produced for each segregated genetic background from the following maize lines:
   - SαZ/SγZ/rhLTF (cf. Figure 36);
   - SαZ/rhLTF;
   - SαZ/SO2/rhLTF (cf. Figure 38);
   - SO2/rhLTF.

### Examples

### Example 1

Production of mutant maize plants for zein accumulation.
Construction of synthetic genes and vectors.
Synthesis of synthetic cDNA.

Maize (Zea mays) plants from a commercial elite line were grown in the greenhouse and hand pollinated. Total RNA extracted from immature endosperm collected 13 days after pollination (DAP) using TRI REAGENT (Sigma) were used as template for first-strand cDNA synthesis with oligo dT primer and reagents of SuperScript II Kit (Invitrogen).

Isolation of an Opaque-2 sequence from maize endosperm cDNAs.

An Opaque-2 (O2) full length coding sequence (SEQ.ID N°55) was obtained by PCR amplification using the previously described cDNA as a template and the two specific primers:
- 5'-CGGGGTACCCCGATGGAGCACGTCATCTCAATGG-3' (SEQ.ID N°1)
- 5'-CCCAAGCTTGGGCTAATACATGTCCATGTGTATGG-3'(SEQ.ID N°2)

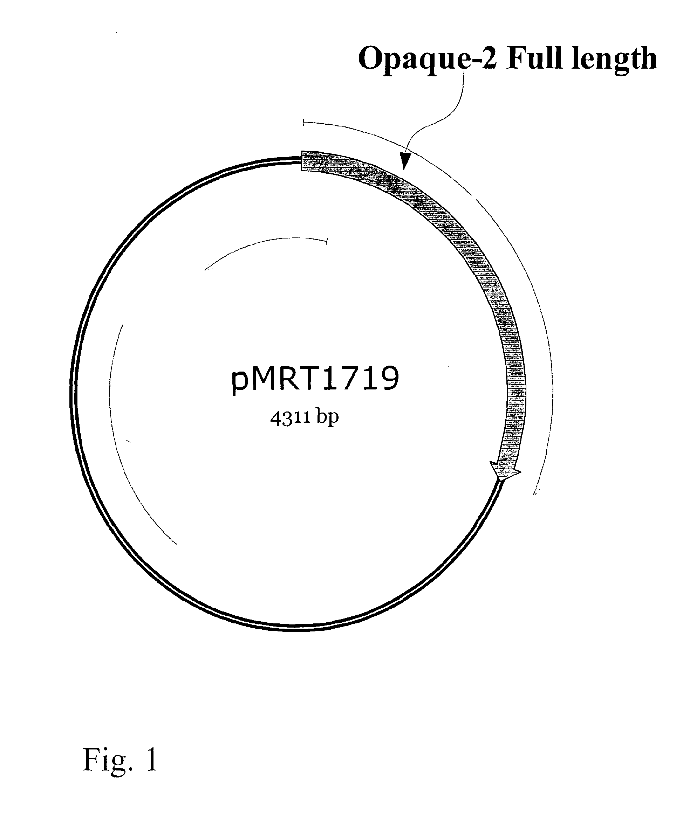

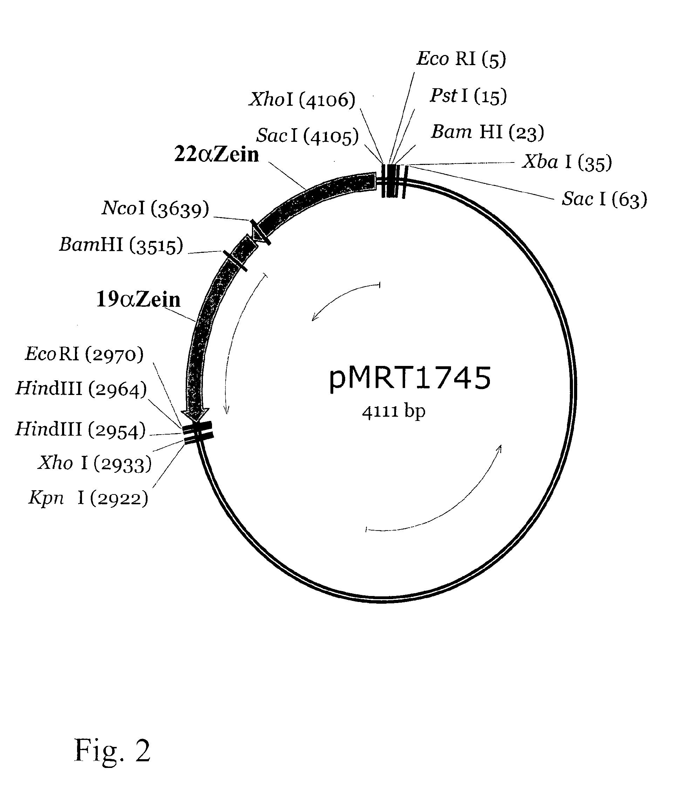

The pMRT1719 plasmid (Figure 1) was created by insertion of the resulting O2 PCR fragment into the *Eco*RV site of the pBluescriptII SK+ vector (Stratagene, Figure 2). The amplified fragment was then sequenced to confirm the nucleic sequence.

Creation of 19/22αZein chimeric sequence from maize endosperm cDNAs.

A chimeric 19/22αZein cDNA sequence (SEQ. ID N°63) was generated by "splicing overlap extension" (SOE, Wurch *et al.,* 1998) using the previously described cDNA as a template and the following primers:
- 5'-GGAAGAAGGGAAGCTATAGGAGTAGATGAAAGTAGTTGTAGG-3', (SEQ.ID N°3)
- 5'-CTCCTATAGCTTCCCTTC-3', (SEQ.ID N°4)
- 5'-CCTCGAGAGCTCACTAGCAACGACTAACACCAATGGCTACCAAGATATTAGC-3', (SEQ. ID N°5)
- 5'-AGCTTGAATTCCTCATAAGAAATCTAAAAGAGGGCACC-3' (SEQ.ID N°6)

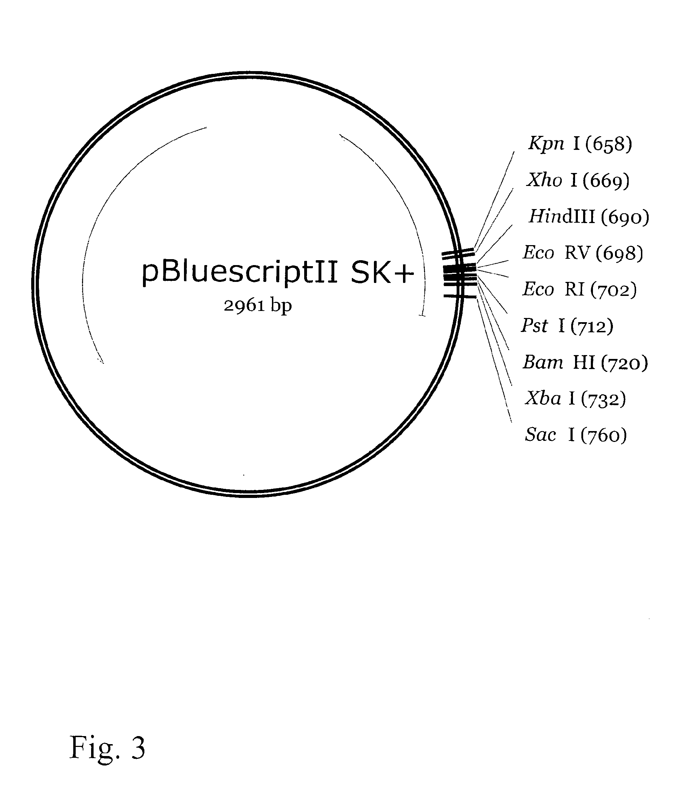

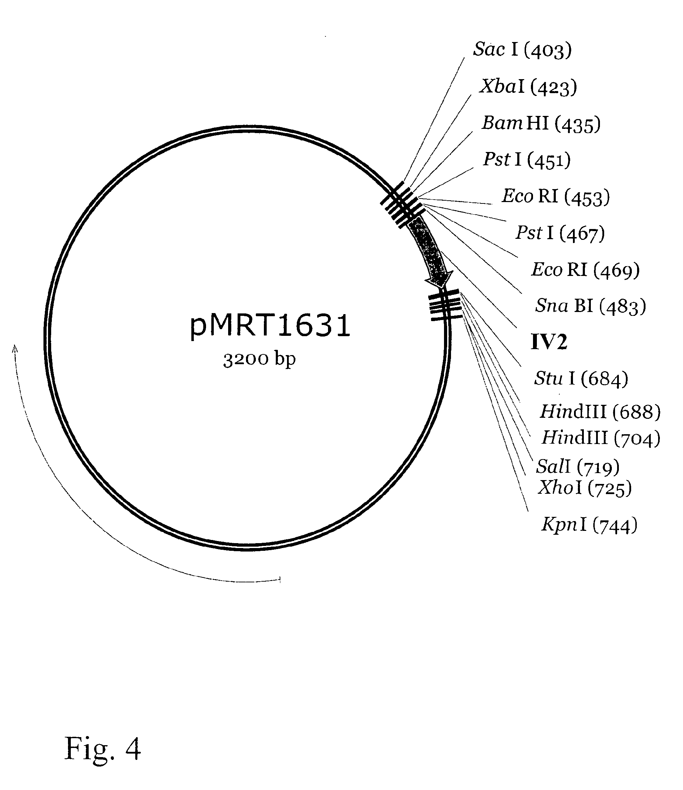

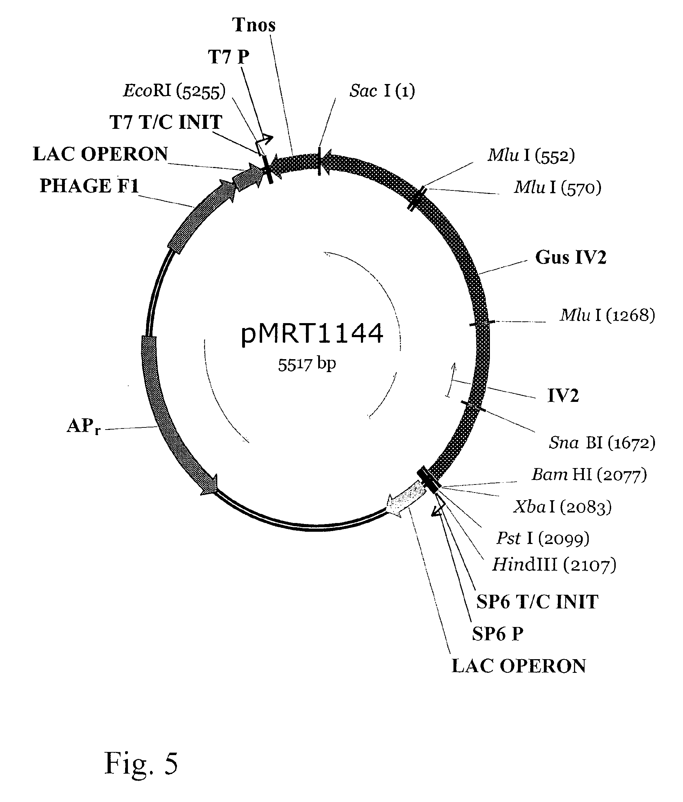

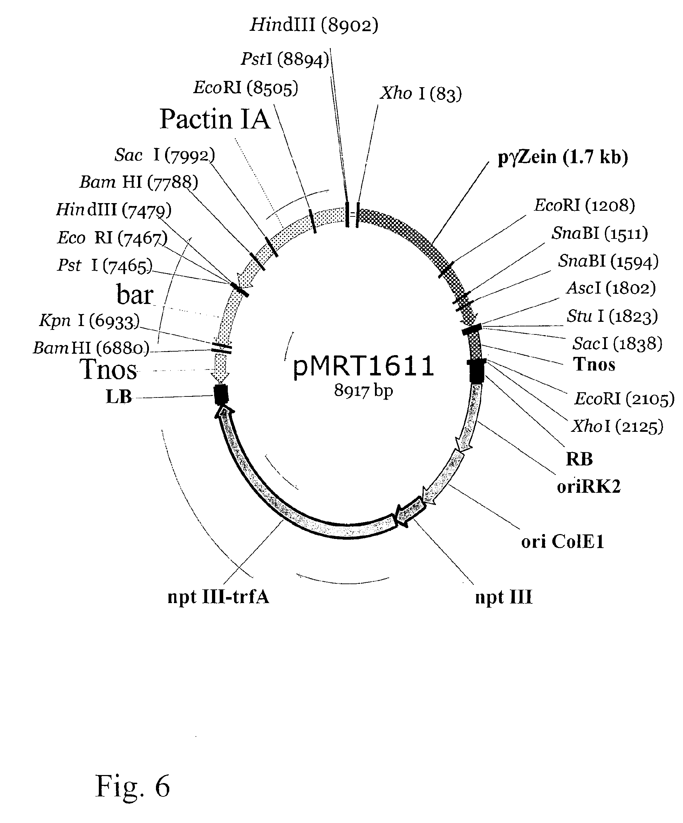

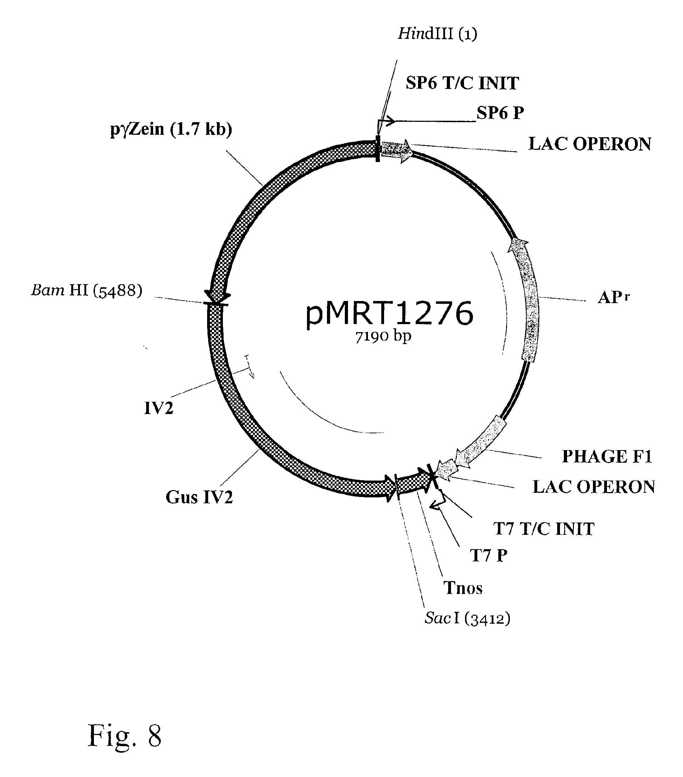

The pMRT1745 plasmid (Figure 2) was created by cloning the resulting chimeric 19/22αZein PCR fragment into the *Eco*RV site of the pBluescriptII SK+ vector.
Plasmid constructs.
Generic plasmids.
(i) pMRT1631 (Figure 4) is a pBluescriptII SK+ plasmid (Figure 3) in which the IV2 intron (Vancanneyt *et al.,* 1990) was cloned into the *Eco*RV restriction site. The IV2 intron was amplified by PCR amplification using pMRT1144 (Norre *et al.,* 2002; Norre, 2002; Figure 5) as a template and the two specific primers:
   - 5'-CTGCAGGAATTCGCTAGCTACGTAAGTTTCTGCTTCTAC-3' (SEQ.ID N°7)
   - 5'-ATCGATAAGCTTAGGCCTGCATGCCAGCTGCACATCAACAATTTTTGGTC-3' (SEQ. ID N°8)
(ii) pMRT1611 (Figure 6) is a binary vector constructed by inserting the full length γ-zein promoter (pγZ, Reina *et al.,* 1990) and the nopaline synthase terminator (T*nos*) of *Agrobacterium tumefaciens* into the *Xho*I digested pMRT1195 (Comeau and Gruber, 2001; Figure 7). The [pγZ-*Tnos*] fragment was generated by SOE using pMRT1276 (Norre *et al.,* 2002; Norre, 2002; Figure 8) as a template and the four synthetic primers:
   - 5' -AAAGGCCTTTTCGGACCGTTGGCGCGCCAACTAGCGGGTTCTTCTGCGCTCTGG-3' (SEQ.ID N°9)
   - 5'-GGTCCGAAAAGGCCTTTTCATCGAGCTCGAATTTCCCCGATCGTTCAAACATTTGGC-3' (SEQ. ID N°10)
   - 5'-TATCTCGAGGGCCGGATCAGCTTGACTGAAATGGAAG-3' (SEQ.ID N°11)
   - 5'-CTATGTTACTAGATCGGGAATTCGGATCGATCCGGCCCTCGAGCA-3' (SEQ.ID N°12)

Molecular construct designed for the γZein RNAi experiments.

A 652 bp γ-zein PCR fragment (SEQ.ID 54) was amplified using cDNA described above as a template and the two synthetic primers:
- 5'-GAGGGTGTTGCTCGTTGCCC-3' (SEQ. ID N°13)
- 5'-GGCAGCAGCGTAGGGGCATG-3' (SEQ. ID N°14)

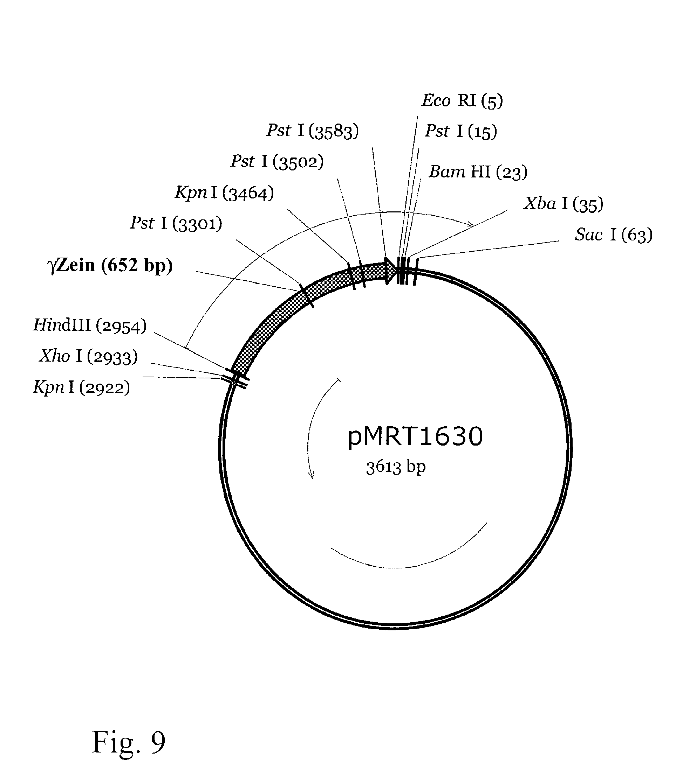

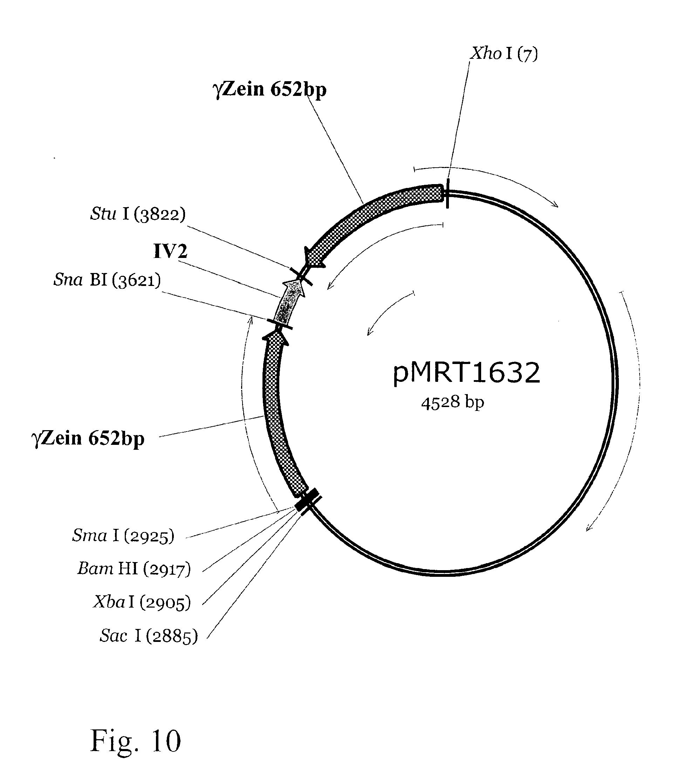

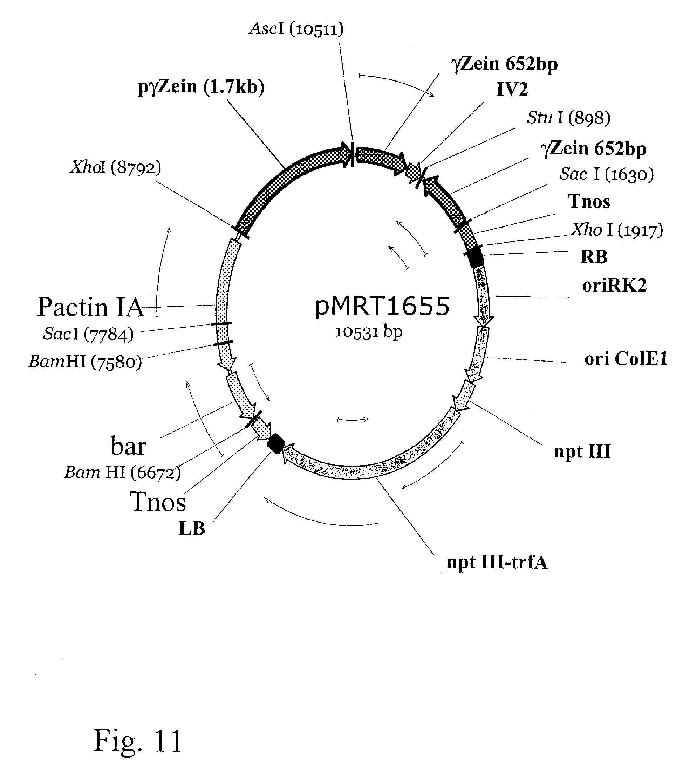

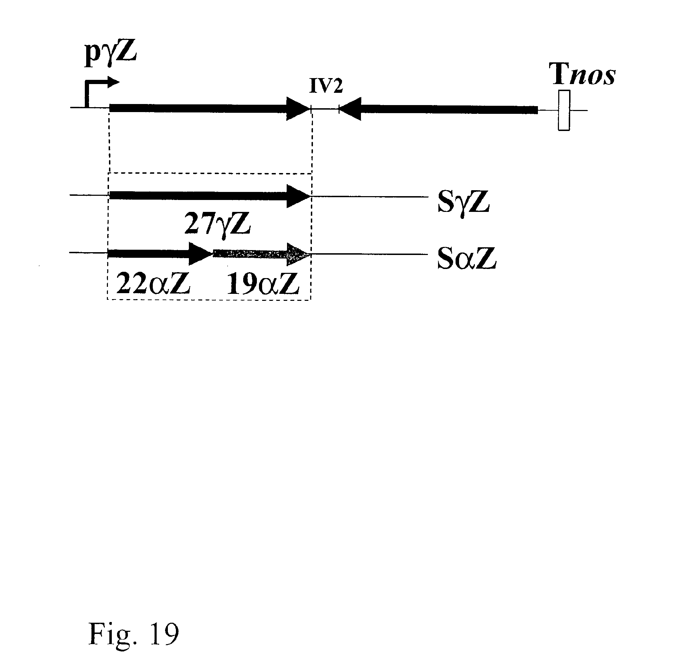

The γ-zein PCR fragment was cloned into the *Eco*RV site of the pBluescriptII SK+ vector. The resulting pMRT1630 plasmid (Figure 9) was digested by *Eco*RI/*Hind*III*.* The γ-Zein fragment (filled in with Klenow enzyme) was cloned successively upstream and downstream of the IV2 intron of pMRT1631 (Figure 4) using *Eco*RI and *Sal*I sites respectively (filled in with Klenow enzyme) in order to create an inverted repeat (SEQ.ID 53) containing pMRT1632 (Figure 10) as shown in Figure 19. The sequence containing the γ-zein inverted repeat and the IV2 intron, was then excised by digesting pMRT1632 with *Smal*I and *Xho*I*,* filled in with Klenow enzyme, and cloned into the *Stu*l site of pMRT1611 (Figure 6). The resulting binary plasmid was named pMRT1655 (Figure 11).

Molecular construct designed for the α-Zein family RNAi experiments.

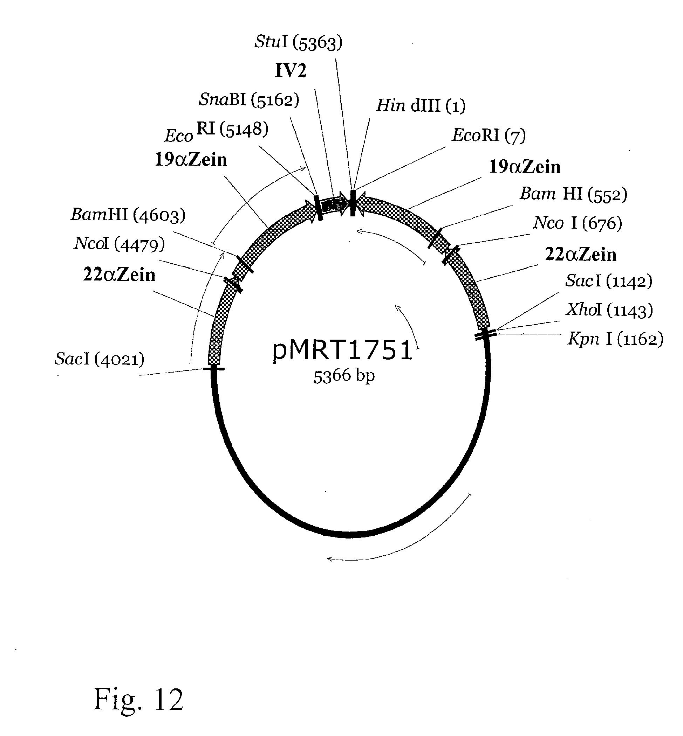

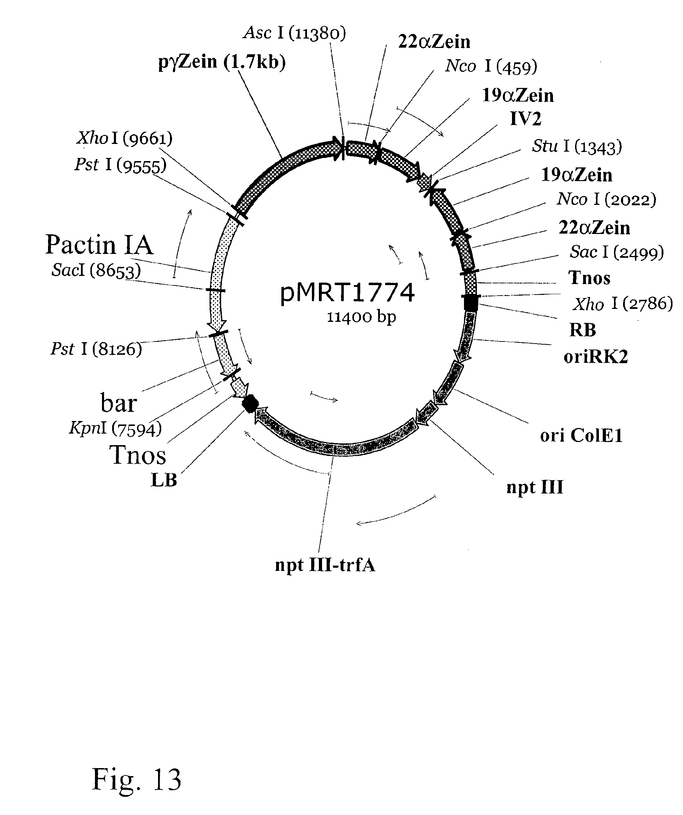

The *Sac*I/*Eco*RI and *Hind*III/*Xho*I chimeric 19/22αZein fragments (SEQ.ID N°63) isolated from the previously described pMRT1745 (Figure 2) were successively cloned upstream and downstream of the IV2 intron in the corresponding sites of pMRT1631 (Figure 4) in order to create an inverted repeat as shown in Figure 19. The resulting pMRT1751 (Figure 12) was digested by *Sac*I to isolate the chimer 19/22αzein inverted repeat (SEQ.ID 50) surrounding the IV2 intron fragment. The blunted insert (T4 DNA Polymerase) was cloned in the Stul site of pMRT1611 (Figure 6) to give pMRT1774 (Figure 13).

Molecular construct designed for the O2 RNAi experiments.

An inverted repeat (SEQ.ID 56) of the O2 coding region (SEQ.ID 55) without the conserved the BZIP domain (O2ΔbZIP, panel A of figure 20) was created for the opaque.2 RNAi approach. The O2ΔbZIP fragment (SEQ.ID 57) was generated by SOE using the previously described pMRT1719 (Figure 1) as a template, and the four primers:
- 5'-CCTCACTCTTTCCTCGGTAGGC-3' (SEQ.ID N°15)
- 5'-ATGCCTACCGAGGAAAGAGTGAGGGACTCCCTGAAGCGGGTG-3' (SEQ.ID N°16)
- 5'-CGGGGTACCCCGATGGAGCACGTCATCTCAATGG-3' (SEQ.ID N°1)
- 5'-CCCAAGCTTGGGCTAATACATGTCCATGTGTATGG-3' (SEQ.ID N°2)

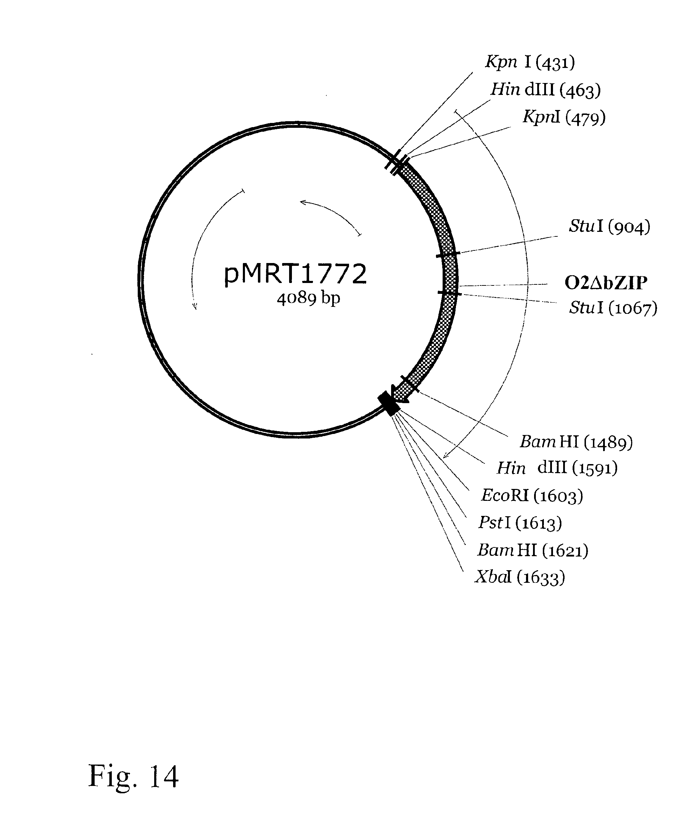

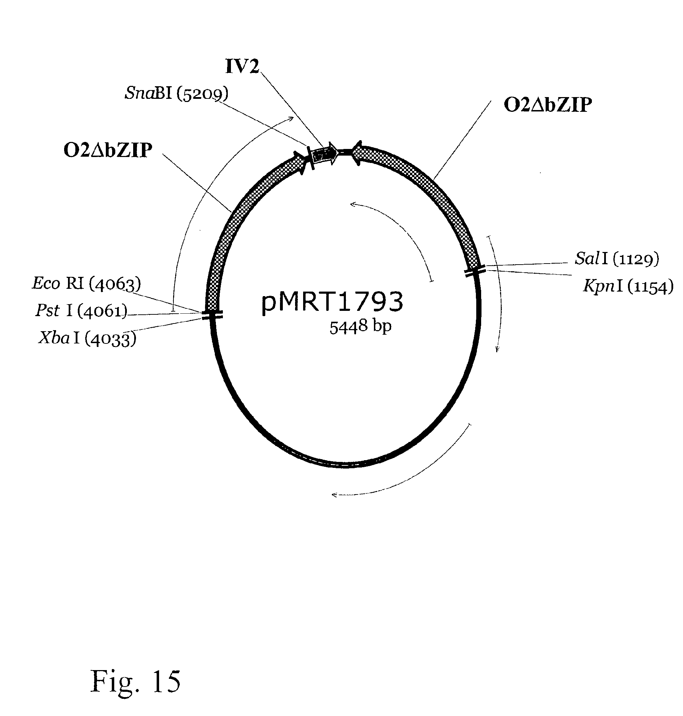

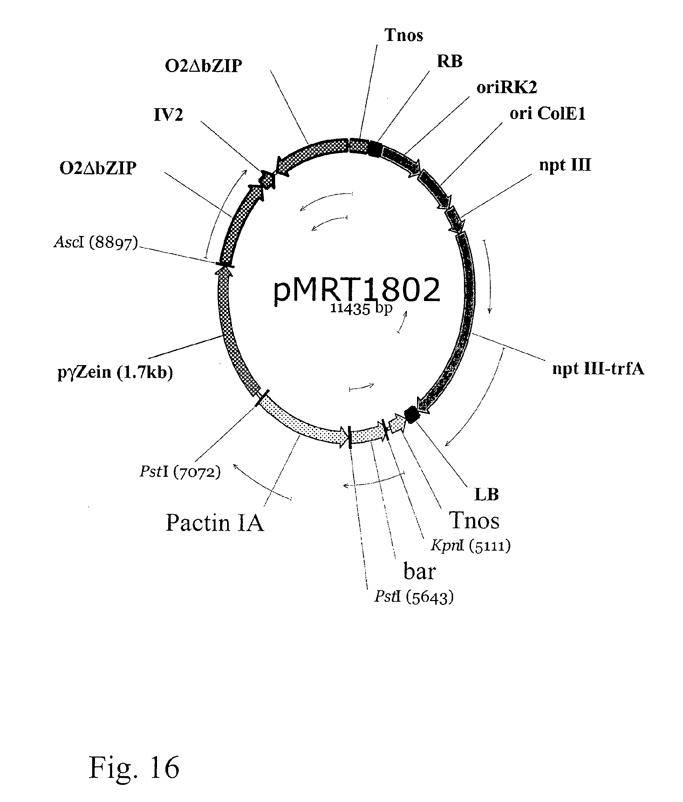

The pMRT1772 plasmid (Figure 14) was created by cloning of the resulting O2ΔbZIP PCR fragment into the *Eco*RV site of the pBluescriptII SK+ vector. Then, the *Kpn*I/*Eco*RI O2ΔbZIP fragment, made blunt using T4 DNA Polymerase, from pMRT1772, was cloned successively upstream and downstream of the IV2 intron from pMRT1631 (Figure 4) into *Eco*RI and *Sal*I sites respectively, that had been filled in with Klenow enzyme, in order to create the inverted repeat as shown in panel B of figure 20 and give the plasmid pMRT1793 (Figure 15). The O2ΔbZIP inverted repeat fragment (SEQ.ID 56) was isolated from the resulting pMRT1793 (Figure 15) by *Sal*I/*Eco*RI, that had been filled in with Klenow enzyme, and cloned into the *Stu*l site of pMRT1611 (Figure 6) to construct pMRT1802 (Figure 16).

Molecular construct designed for the O2-TDN experiment.

An O2 coding region (SEQ.ID 55) without the DNA binding domain, (O2ΔBD, panel A of figure 20) was created for a trans-dominant suppression approach (O2-TDN, panel B of figure 20). The O2ΔBD fragment (SEQ.ID 60) fused to the T7 tag sequence (SEQ.ID 59) in its 5'-end was generated by SOE using the previously described pMRT1719 (Figure 1) as a template, and the four synthetic primers:
- 5'-ATGCCTACCGAGGAAAGAGTGAGGAGGAAAGCCGCTCACCTG-3' (SEQ.ID N°17)
- 5'-CCTCACTCTTTCCTCGGTAGGC-3' (SEQ.ID N°15)
- 5'-CCCAAGCTTGGGCTAATACATGTCCATGTGTATGG-3' (SEQ.ID N°2)
-

5'-GGGGTACCATGGCCTCCATGACCGGCGGCCAGCAGATGGGCGAGCACGTCATCTCAATG
GAGG-3' (T7-tag is underlined). (SEQ.ID N°18)

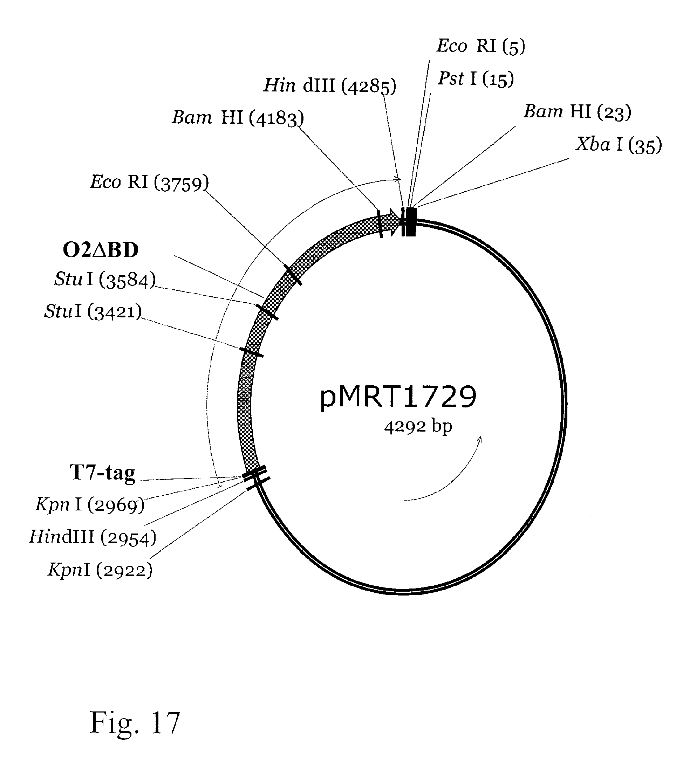

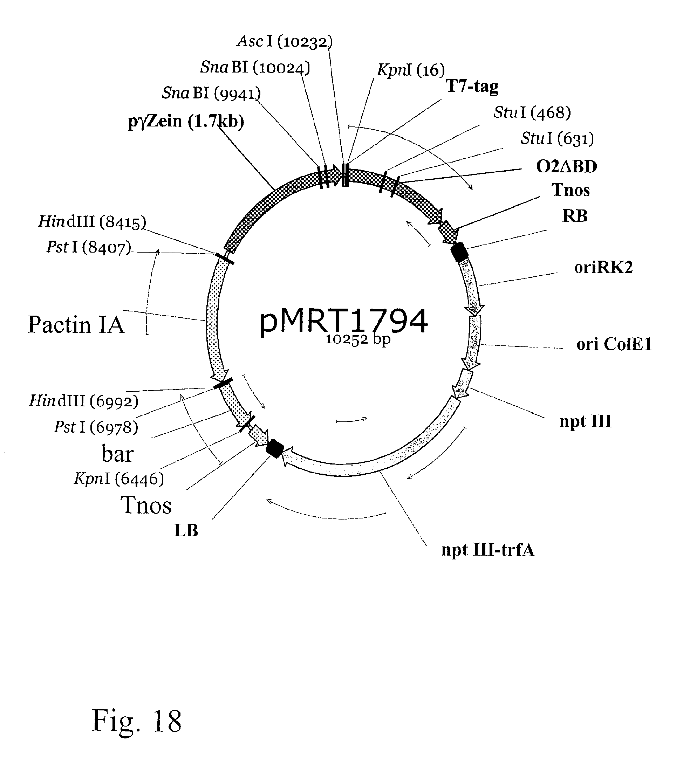

The pMRT1729 plasmid (Figure 17) was created by cloning of the resulting T7-O2ΔBD PCR fragment (SEQ.ID 58) into the *Eco*RV site of the pBluescriptII SK+ vector. Then, the *Hind*III T7-O2ΔBD fragment, excised from pMRT1729 and filled in with Klenow enzyme, was cloned into the *Stu*l site of pMRT1611 (Figure 6) to create the binary plasmid pMRT1794 (Figure 18).

Generation of mutant maize plants affected in zein accumulation.

Stable maize plant transformation.

Immature embryos from the inbred line A188 were transformed with *Agrobacterium tumefaciens* LBA4404 that carried the pSB1 plasmid and one of the binary plasmids pMRT1655 (SγZ, Figure 11), pMRT1774 (SαZ, Figure 13), pMRT1802 (SO2, Figure 16), or pMRT1794 (O2-TDN, Figure 18), described above, according to the procedure described by Ishida *et al.* (1996). Independent primary transformants were crossed with an elite line to produce T1 seeds. T2, T3 and T4 seeds were obtained by self pollination or by backcrosses with the elite inbred.

Cross pollination of mutant transgenic lines.

Cross pollination of different mutant maize lines, i.e. SαZ, SγZ and SO2, were carried out in order to obtain double mutant maize lines: SαZ/SγZ, SO2/SαZ and SγZ/SO2 respectively. Triple mutant maize lines, i.e. SαZ/SγZ/SO2 were obtained by cross pollination of a SO2 mutant maize line and a SαZ/SγZ double mutant maize line.

Biochemical analysis of mutant maize plants.

Zein extraction and SDS-PAGE analysis.

Zein protein fraction from immature or mature seeds was respectively extracted with 5 or 10 volumes of a mixture of 70% ethanol and 2% β-mercaptoethanol under agitation (400rpm) for one hour at room temperature. The crude extract was centrifuged at 16060g for 30min to remove the ethanol insoluble material. After separation on 12% or 15% SDS-PAGE gel, zeins were stained with Coomassie Brilliant Blue R250.

Zein immunodetection.

Production of anti α-, γ-, and β-zeins polyclonal rabbit antibodies.

The α-, β- and γ-zein polypeptides were respectively isolated from SγZ, SαZ/SγZ and SαZ/SO2 mutant maize lines described above in order to obtain each zein with an optimal purity. Each protein (α-, β- and γ-zeins) was extracted from transgenic endosperm using 70% ethanol and 2% β-mercaptoethanol (1w/10v) for one hour under agitation (400rpm) at room temperature.

Standard rabbit immunisation protocols with the purified zeins were used to produce specific antibodies.

Western blot analysis.

For immunodetection, electrophoretically separated zeins were transferred to nitrocellulose membranes as described by Sambrook *et al.* (1989). Zeins from different mutant maize lines were identified using the rabbit anti-zeins α, β, or γ (described above) and visualized by anti-Rabbit immunoglobulins (IgG) conjugated to alkaline phosphatase (Sigma). Alkaline phosphatase was detected using BCIP/NBT Alkaline Phosphatase Substrate (B-5655, Sigma).

Molecular analysis of mutant maize plants.

RT-PCR analysis.

Total RNA was extracted from dissected frozen transgenic and syngenic maize endosperm harvested from greenhouse-grown plants at 18 DAP using TRI REAGENT (Sigma). One microgram RNA was treated with 2U of DNAse and used as template for first-strand cDNA synthesis with oligo dT primer and reagents of SuperScript II Kit (Invitrogen). In order to estimate the expression level of specific RNAs, 35 cycles of PCR amplification [denaturation (94°C for 1 min), annealing (55°C for 1 min) and synthesis (72°C for 1 min 30)] were carried out and aliquot fractions were taken at 25, 30 and 32 cycles. PCR amplifications were performed using 1µL of cDNA synthesis reaction, Platinum Taq DNA polymerase High fidelity (Invitrogen) and 25µM of each pair of primers described below:
αZ22:
   - 5'-ACACCGCCGGCAGCAGCG-3' (SEQ.ID N° 19)
   - 5'-AGTAGTTGTAGGTAGACG-3' (SEQ.ID N°20)
αZ19-D:
   - 5'-AGCCCTTATGCCTTCTTACTGC-3' (SEQ. ID N°21)
   - 5'-TATCAGCATGAGAACTTTATTATTACAG-3' (SEQ. ID N ° 22)
αZ19-B:
   - 5'-TCTACCCACAGAATGTGGC-3' (SEQ.ID N°23)
   - 5'-TGCCAGCGTTAGGCGCAG-3' (SEQ.ID N°24)
αZ19-A:
   - 5'-TGGAATCCAGTAGCAACAATAG-3' (SEQ.ID N°25)
   - 5'-GGTTGATCAGAGGTAGCACGAG-3' (SEQ.ID N°26)
γZ27:
   - 5'-AGTGCCAGTCGTTGCGGC-3' (SEQ.ID N°27)
   - 5'-TACGGGGACACCGCCGGCAGCAGCG-3' (SEQ.ID N°28)
γZ16:
   - 5'-TGGTGATGGGTGACACTACG-3' (SEQ.ID N°29)
   - 5'-GGCGCTGATGGCGGCCCAGG-3' (SEQ.ID N°30)
γZ50:
   - 5'-AACTACTATAGCAGTAGCTC-3' (SEQ.ID N° 31)
   - 5'-ACCGTTATATGAGTATAACGGC-3' (SEQ.ID N°32)
δZ10:
   - 5'-TGCAGCACCAACAAAGGGTTGC-3' (SEQ. ID N°33)
   - 5'-ACGCGTCCGGTCGCACATATCTAC-3' (SEQ.ID N°34)
βZ15:
   - 5'- ACAGGGGTTGGTGCGGTAGC-3' (SEQ.ID N°35)
   - 5'-TACCTGAGGCAGCCGCAGTGC-3' (SEQ.ID N°36)
O2:
   - 5'-TAGAGAATCAGCCAGACGCTCG-3' (SEQ.ID N°37)
   - 5'-TACATGTCCATGTGTATGG-3' (SEQ.ID N°38)
O2ΔbZIP:
   - 5'-AACTCTTGTTGCTCAGGCGC-3' (SEQ.ID N°39)
   - 5'-TACATGTCCATGTGTATGG-3' (SEQ.ID N°38)
OHPI:
   - 5'-TGTGGAGGGCTTCTGGTGCAGC-3' (SEQ.ID N°40)
   - 5'-TGCTGAGGGACGACATATCAGAC-3' (SEQ.ID N°47)
PBF:
   - 5'-TGCCTCTAGATTTGTCTTGGGC-3' (SEQ. ID N°48)
   - 5'-ATTGCACCATATGATGGCAGCG-3' (SEQ. ID N° 49)
b-32:
   - 5'-AGCAAGCTGGTGAAGCTGGTGG-3' (SEQ. ID N°41)
   - 5'-TCATCGTCGTTGTCAGCACTGG-3' (SEQ. ID N°42)

Screening of double mutant maize seedling.

Transgene detection (SαZ, SO2 and SγZ) was carried out by PCR amplification from a leaf disc (1cm²) of each maize seedling using RedExtract-N-Amp^{™} Plant PCR Kit (Sigma) according to the procedure described by the supplier. In order to detect each transgene, 35 cycles of PCR amplification [denaturation (94°C for 45 sec), annealing (56°C for 1 min) and synthesis (72°C for 1 min)] were carried out. The primers described below were used:
SαZ:
   - 5'-TGCACTTCTCCATCACCACC-3' (SEQ.ID N°43)
   - 5'-AGATGAAAGTAGTTGTAGGTAGACG-3' (SEQ.ID N°44)
SγZ:
   - 5'-AGCAGCGTAGGGGCATGGAGTCG-3' (SEQ.ID N°45)
   - 5'-TCGCAAGACCGGCAACAG-3' (SEQ.ID N°46)
SO2:
   - 5'-TGCACTTCTCCATCACCACC-3' (SEQ.ID N°43)
   - 5'-CCTCACTCTTTCCTCGGTAGGC-3' (SEQ.ID N°15)

Screening of double and triple mutant maize seeds.

Transgene detection (SαZ, SγZ or SO2) was carried out by PCR amplification from endosperm tissue of each seed using RedExtract-N-Amp^{™} Seed PCR Kit (Sigma) according to the procedure described by the supplier. In order to detect each transgene, 35 cycles of PCR amplification [denaturation (94°C for 45 sec), annealing (56°C for 1 min) and synthesis (72°C for 1 min)] were carried out. The same primers as described above were used:
SαZ: SEQ.ID N°43 and SEQ.ID N°44
SγZ: SEQ.ID N°45 and SEQ.ID N°46
SO2: SEQ.ID N°43 and SEQ.ID N°15

### RESULTS AND DISCUSSION.

Generation of transgenic maize plants with reduced or deficient zein accumulation.

One of the objects of the present invention resides in the fact it is possible to reduce the alcohol-soluble protein fraction from maize endosperm, by depleting the αZein and γZein polypeptides families, representing approximately 90-95% of total storage proteins in maize. A RNA interference (RNAi) technique, and a dominant inhibitor of bZIP trans-acting factor approach, was used to obtain this depletion.

Modulation of zein accumulation in maize endosperm.

The RNAi technique which confers post-transcriptional gene silencing (PTGS) is an efficient way for depleting individual mRNA in eukaryotes (Tavernarakis *et al.,* 2000; Matzke *et al.,* 2001). This approach was chosen in the present invention to specifically suppress a single or a family of zeins, without affecting the accumulation of non targeted zein polypeptides or other proteins. RNAi silencing applied to genes encoding maize storage proteins has previously been described by Segal *et al.,* 2003. The authors of this paper obtained transgenic plants with a reduced accumulation of the 22α-zein storage protein.

The 27γZ gene was selected as one of the targets of our RNAi approach for three main reasons: (i) the 27γZ polypeptide represents 15-25% of total zein content, (ii) no 27γZ mutant are available and (iii) there is a putative role attributed to the 27γZ gene in protein body stabilization which could be confirmed or denied. A construct was made containing an inverted repeat (SEQ.ID 53) with a partial 27γZ cDNA fragment (SEQ.ID 54; 652 bp, spanning from +2 to +654 relative to the translational start; Reina *et al.,* 1990) separated by the IV2 intron (Vancanneyt *et al.,* 1990) as illustrated schematically in Figure 19 (See SγZ).

As αZein polypeptides represent 60-80% of the zein pool, the inventors also decided to target both the 19αZein and 22αZein multigene families (SαZ) to create a maize endosperm phenotype characterized by a specific depletion of all αZein proteins. To simultaneously reduce the accumulation of all αZeins, a 19/22αZein chimeric sequence (SEQ.ID 63) was synthesised to construct an inverted repeat (SEQ. ID 50) as described above and shown in Figure 19 (See SαZ) created. As the αZein gene family is relatively complex (Woo *et al.*, 2001), the targeted 19αZein and 22αzein sequences were selected from the most abundant mRNAs. The amplified 22αZein gene sequence used (SEQ.ID 51) shared 99.8% homology with a 478 bp fragment (spanning from ATG to nucleotide +478) from the AF371274 cDNA (Woo *et al.,* 2001) whereas the 19αZein PCR fragment used (SEQ.ID 52) in the constructs of the present invention has 93.5% homology with a 629 bp sequence (spanning from position +88 to +718 relative to the ATG) from the AF371269 cDNA (Woo *et al*., 2001).

The RNAi approach described above was designed to specifically target genes encoding storage proteins of the zein family at the post transcriptional level (PTGS). Zein accumulation can also be down regulated by affecting expression of zein coding sequences at the transcriptional level. Several *trans*-acting factors have been shown to control expression of zein genes during maize endosperm development. The transcription factor opaque-2 is known to regulate numerous genes specifically expressed in endosperm tissue. In *opaque-2* mutants, 22αzein accumulation is significantly reduced compared to the wild type phenotype. As the mutation is recessive, the RNAi approach adopted by the inventors and targeted to the O2 sequence would lead to a dominant opaque-2 like mutant.

The O2 cDNA sequence (SEQ.ID 55) used for the RNAi approach shared 99.9% homology with an O2 cDNA sequence referenced under accession number ZMA491297. In order to specifically affect O2's, an inverted repeat (SEQ.ID 56) of the O2 cDNA minus the highly conserved BZIP sequence (spanning from position +705 to +924) was designed (Panel A of figure 20, compare *O2* and *O2*ΔbZIP). To do this, the o2 cDNA sequence depleted of BZIP domain sequence (*O2*ΔbZIP; SEQ.ID 57) was placed into RNAi cassette as shown in panel B of Figure 20 (cf. SO2 construct).

Finally, a dominant negative mutant of the O2 *trans*-acting factor (O2-TDN) was created. The strategy for obtaining this was based on the "scissors grip" model of BZIP protein-DNA interactions in which the basic domain of both partners is essential for stable DNA binding (Miao and Lam, 1995; Ellenberger *et al*., 1992; Vinson *et al.,* 1989). Heterodimerisation between a mutant lacking the basic domain and a wild type partner is predicted to produce an inactive dimer. The *trans*-dominant inhibitor approach to specifically suppress the BZIP activity of *trans*-acting factors has previously been successfully applied to members of the tobacco TGA protein family (Pontier *et al.,* 2001; Miao and Lam, 1995) and potato PG13 protein (Rieping *et al.,* 1994). The O2 cDNA sequence (SEQ.ID 55) used in the O2-TDN strategy has been described above. The corresponding amino acid sequence (SEQ.ID 61) is homologous to the protein sequence described under the accession number CAD36195 except for the replacement of a threonine by an alanine at position 191 which has been shown to be among several variants of the O2 protein (accession number P12959 or A34800). As illustrated in panel A of figure 20 (compare O2 and O2ΔBD), the inventors suppressed the DNA sequence encoding a 15 amino acid region (spanning from amino acid 235 to 249 relative to the "translational start") localized into the basic domain (BD) of O2. A T7 epitope was inserted at the N-terminus end of the modified O2 protein to specifically detect the O2ΔBD mutant protein without cross reactivity with the wild type O2 (Panel A of figure 20, See O2ΔBD). The expression cassette of the negative mutant of O2 *trans*-acting factor is illustrated in panel B of figure 20 (See O2-TDN construct).

All cDNA sequences described above (Figure 19 and panel B of figure 20) were fused to the full length γZein promoter (1,7 kb; Reina *et al.,* 1990) and to the nopaline synthase terminator (T*nos)* of *Agrobacterium tumefaciens* in order to obtain strong endosperm specific expression. Each expression cassette was independently introduced into Zea mays (A188 line, Green and Phillips, 1975) *via Agrobacterium tumefaciens* mediated transformation. Primary transformants were pollinated with pollen from a commercial elite line or A188 untransformed plants. T1 transgenic and non transgenic segregating immature endosperms were collected after embryo rescue and analyzed by SDS-PAGE for zein content. At least 10 T₁ immature seeds (18 days after pollination, DAP) per plant were independently studied. The zein patterns of transgenic and segregating non transgenic endosperms were analyzed in parallel. T₂, T₃ and T₄ seeds were obtained by self pollination or by cross pollination with an elite inbred line.

Post-transcriptional silencing of zein genes.

Depletion of the γZein polypeptides (SγZ).

From a total of 29 independent transgenic lines analyzed, 21 were characterized by a drastic decrease in the 27γZein and 16γZein contents in the transgenic endosperm. The suppression of both the 27γZein and 16γZein polypeptides did not affect remaining zein accumulation (cf. panel A, B, C and D of figure 21). Residual band visualized in coomassie blue (cf. panel A of figure 21) at the position of the depleted 27γZein is probably a divergent Zein or a non zein protein that comigrates with the targeted proteins; indeed, this residual protein was not detected by immunoblotting using an anti-γZein antibody (cf. panel B of figure 21). However, it seemed to be visualized using an anti-αZeins antibody, which indicates that it is probably a protein from the family of αZeins (cf. panel C of figure 21).

RT-PCR experiments carried out with RNA from wild type and transgenic maize endosperms (18 DAP, cf. panel E of figure 21) showed a correlation between the 27γZein and 16γZein depletion and a strong reduction to traces of the corresponding mRNAs. The mRNA level of other zein genes was not affected by the expression of 27γZein RNAi cassette (cf. panel F of figure 21). These results confirm that the RNAi technique is an efficient way of depleting specific mRNA or mRNA families in maize endosperm. The concomitant suppression of the 16γZein and 27γZein can be explained by the high degree of similarity between the two storage proteins over partial sequences. Although, the overall homology was as low as 67.3%, a 497 bp 27γZein sequence shared more than 80% homology with the 16γZein coding sequence. In contrast, the larger DNA fragment from other zeins (γ50, α22, β15 and δ10) with a homology superior to 80% to the 27γZein one has a maximal size of 20bp.

The zein patterns analysis and their characterization by western blot from mature seeds showed that 27γZein and 16γZein depletion is stable during all endosperm development stages and that the αZein and the βZein families were unaffected in mature seeds (cf. panel A, B, C and D of figure 27, see lane SγZ).

The inventors also examined whether the 27γZein post-transcriptional gene silencing is stably transmitted. In this perspective, it was decided to study the progeny of a T₀ line. At least nine T₁ transgenic plants were propagated and self-pollinated. Zein analysis has shown that 16γZein and 27γZein depletion was stable in T₂ transgenic seeds produced from the nine T₁ plants. Finally, it was demonstrated that 16γZein and 27γZein transgene suppression can be stable for at least four generations, since 16γZein and 27γZein depletion was observed in T₃ and T₄ transgenic seeds from this line (data not shown). The first homozygote plants were identified in third generation (T₃) plants. Finally, no developmental phenotype associated with an alteration of γZein expression was observed in any of the generations of genetically modified plants produced (Figure 27, Panel E).

Depletion of the αZein polypeptide families (SαZ).

Seven primary transformants expressing an inverted repeat of the 19/22αZein chimeric sequence were regenerated. T₁ seeds from all transgenic lines were all found to segregate with normal and "highly" reduced αZein phenotype in 18 DAP maize endosperm as illustrated in panel A of figure 22. Both 19αZein and 22αZein families were simultaneously depleted with high efficiency without apparent compensatory changes in the 16γZein and 15βZein content. However, the suppression of both the 22αZein and 19αZein polypeptides had two main consequences:
(i) a marked over accumulation of the 27γZein polypeptide (Panel A and C of figure 22, compare lanes SαZ and WT), as already observed in *o2* mutant maize lines (Hunter *et al.,* 2002). Thus, the data obtained in the present invention indicate that the greater compensatory effect on 27γZein polypeptide accumulation in *o2* mutant maize lines is an indirect consequence of the absence of the O2 trans-acting factor *via* strong reduction of αZein polypeptide accumulation, and certainly through a post-transcriptional and/or translational and/or post-translational mechanisms.
(ii) a marked over accumulation of a protein of low molecular weight (<6.5kDa) visualized using an anti-βZein antibody (Panel D of figure 22, compare lanes SαZ and WT); this protein which was not described before today is probably a protein from the βZein family.

Residual proteins detected at the position of the depleted αZeins are probably divergent αZeins, partially or not affected by the post-transcriptional gene silencing; this hypothesis was confirmed by immunoblotting, since proteins which migrate at the 19αZein and 22αZein expected sizes were detected using anti-αZein antibodies (Panel B of figure 22, lane SαZ). Owing to the complexity of the αZein family, it is possible that some minority αZeins with a lower homology of nucleic sequence are not targeted by the RNAi silencing. For example, the 19αZein from subfamily A presents only 66% of homology with that from subfamily B which was used to construct the RNAi cassette.

RT-PCR analyses carried out RNA from wild type and mutant maize endosperm (18 DAP, panel E of figure 22) show a variable impact at mRNA level of 19/22αZein RNAi silencing when considering the αZein subfamilies (Figure 22, panel F). Specific RT-PCR analyses from a member of each αZein subfamily indicate a strong reduction of mRNA corresponding to 22αZein (AF371274) and 19αZein-B (AF371269) used to construct the RNAi cassette. In contrast, the impact on mRNA level from the 19αZein-A (AF371271) and 19αZein-D (AF371267) subfamilies seems to be less important, but not detrimental, since the near total of 19αZein and 22αZein was depleted. The mRNA level of other zein genes is not affected by the expression of the 19/22αZein RNAi cassette as illustrated by RT-PCR carried out on 27γZein mRNA (Figure 22, panel F and data not shown).

Zein pattern analysis in mature seeds indicated that 19αZein and 22αZein depletion is stable during all endosperm development stages (Figure 27, panel A, B, C and D, see SαZ lane) and is concomitant with the production of an opaque phenotype in the transgenic seeds (Figure 27, panel E) as previously observed in maize *o2* mutants (Motto *et al.,* 1989) or in maize with specific 22αZein depletion (Segal *et al.,* 2003). In mature seeds, it was also seen that both of the marked over-accumulation of the 27γZein and on the protein of low molecular weight (<6.5kDa), identified using an anti-βZein antibody, were stable during all endosperm development stages (Figure 27, panel D and C respectively).

The inventors also demonstrated that 19/22αZein transgene suppression is stable for at least three generations. From at least two initial independent T₀ lines, 19αZein and 22αZein depletion was respectively conserved in T₂ and T₃ transgenic seeds. As was observed in the first generation, a near total depletion of 19αZein and 22αZein in maize endosperm from mature seeds was associated with an opaque phenotype. The first homozygote plants were obtained from third generation (T₂) plants.

The results obtained by the inventors indicate that the RNAi technique can be adapted to induce a specific and simultaneous post-transcriptional gene silencing of at least two distinct gene families. Indeed, this approach allowed the inventors to obtain the first maize line in which the endosperm totally lacks 19αZein and 22αZein storage proteins, in spite of the low homology (<50%) between the two complex gene subfamilies, as if they were the product of one gene.

Transcriptional deregulation of zein genes.

Knock out of the Opaque-2 transcription factor (SO2).

An opaque-2 (O2) maize mutant was created by RNAi silencing in order to reproduce the effect of natural mutation. Eight transgenic lines were regenerated after integration of the SO2 construct (Figure 20, panel B) into their genome. T₁ seeds from all transgenic lines were all found to segregate with normal and modified zein phenotype. Several modifications of the zein pattern were observed in transgenic maize endosperm at 18 DAP (Figure 23, panel A). The 22αZein polypeptides were highly depleted and, the 15βZein and the 19αZein accumulation were significantly decreased. Moreover, as previously described in o2 mutant maize lines (Hunter *et al.,* 2002), the 27γZein polypeptides were also strongly over-accumulated in SO2 mutant seeds (Figure 23, panel A and D, compare lanes SO2 and WT). Thus, the zeins affected by SO2 transgene expression are the same as those affected in *o2* mutant lines. However, as already observed in the SαZ mutant seeds (see above), the SO2 transgene induced strong over-accumulation of a protein of low molecular weight (<6.5kDa), identified using an anti-βZein antibody, which had not been previously observed in the *o2* mutant maize line (Figure 23, panel C, compare lanes SO2 and WT). A small band visualized at the position of the 22αZein is probably a residual 22αZein from a gene partially or not regulated by O2 trans-acting factor; its detection using an anti-αZein antibody support this hypothesis (Figure 23, panel B, see SO2 lane). Indeed, Song *et al.* (2001) have shown some of the 22αZeins are still synthesized in homozygous BSSS53 (*o2*), indicating the ineffectiveness of o2 variants in achieving a complete repression of all 22αZein genes. Thus, with regard to 22αZein depletion it is probable that the 19/22αZein RNAi described and designed by the inventors is more effective than the O2 RNAi owing to redundancy of regulatory factors.

Zein pattern modifications have been correlated with the silencing of the O2 trans-acting factor by a RT-PCR approach using several primer pairs as illustrated in panel A of figure 26. A strong decrease in the O2 mRNA level was detected in transgenic maize endosperm at 18DAP with specific primers for O2 endogene mRNA (619bp, panel B of figure 26). Interestingly, using two primers outside the BZIP domain of O2, it was possible to visualize a basal level of mRNA from the O2 endogene (upper band, 1071 bp) and SO2 transgene (lower band, 849bp) in transgenic seeds; in wild type seeds, the lower band was naturally not amplified and the upper band was much more intense (Figure 26, panel A). The O2 mRNA detection in transgenic seeds indicates (i) that the SO2 transgene confers a post-transcriptional gene silencing, and does not affect, or only partially affects O2 gene expression, and so (ii) that the O2 gene is probably not, or only partially, regulated by the product of its own gene.

Specific RT-PCR analyses (Figure 23, panel E) from a member of each zein family reveal that the impact of SO2 transgene expression on transcriptional regulation of storage proteins is in direct relation to that observed in their accumulation. A marked reduction of mRNA corresponding to 22αZein was observed as well as a noticeable reduction of mRNA level from each of the 19αZein subfamilies and from 15βZein family. The mRNA level of other zein genes is not affected by the knock out of O2 as illustrated by RT-PCR carried out on 27γZein mRNA (Figure 23, panel E) and on δZein mRNA (data not shown).

Zein pattern analysis in mature seeds showed that O2 RNAi silencing led to a stable zein depletion during all stages of seeds development; all the modifications concerning zein accumulation in SO2 mutant maize endosperm at 18DAP (see above) held true in mature seeds (Panel A, B, C and D of figure 27, see SO2 lane) and were associated with the production of an opaque phenotype (Panel E of figure 27). T₂ and T₃ seeds generated from one T₀ event were all found to segregate with wild type and opaque phenotype. The mutant zein pattern transmitted was identical to that observed in T₁ seeds and the first homozygote plants were obtained from third generation (T₂) plants.

The inventors have thus managed to produce, for the first time, an *o2*-like maize mutant with the property to be dominant. The plant line created is able to confer the effect of an *o*2-like mutation without having to be necessarily homozygote for the SO2 transgene.

Creation of a trans-dominant inhibitor of O2 protein (O2-TDN).

From a total of 23 transgenic lines regenerated with the O2-TDN transgene (Figure 20, panel B) in their genome, only 3 were found to segregate seeds with normal and zein phenotype in maize endosperm at 18 DAP. These three lines presented a highly modified zein pattern (O2-TDN), very similar to the one obtained with O2 RNAi silencing (SO2). The 22αZein and 15βZein storage proteins were hardly, or not at all, synthesized, 19αZein accumulation was significantly reduced and, the 27γZein and the 16γZein polypeptides were strongly over accumulated (Figure 24, panel A). Thus, zeins affected by the expression of O2-TDN transgene are the same as those affected in SO2 (see above) and *o2* mutant lines (Hunter *et al.,* 2002). As shown using an anti-T7 antibody, the modifications observed here were correlated with the expression of O2ΔBD protein in O2-TDN mutant seeds (Figure 24, panel C).

The impact of O2ΔBD protein expression on zein gene regulation has been analyzed by RT-PCR experiments using RNA from T₁ seeds of the line studied above (Figure 24, panel B, D and E). It was shown that all zein genes, except perhaps the 16γZein gene, were more or less affected at the transcriptional level in the maize endosperm, and associated with a highly modified zein pattern. In accordance with the effects observed on zein accumulation, 22αZein mRNA level was strongly reduced whereas those from the 19αZein and 15βZein families were only partially decreased. Surprisingly, 27γZein mRNA level was also significantly reduced while 27γZein polypeptide seemed to be over-accumulated by a strong compensatory effect. Thus, the transcriptional down-regulation of the 27γZein gene by the O2ΔBD protein was cancelled out by a downstream regulatory mechanism, probably at post-transcriptional and/or translational and/or post-translational levels. An important reduction of mRNAs from the 10δZein gene was also observed; however, the impact on 10δZein polypeptide accumulation was not established. These results are very interesting because they show (i) that the O2-TDN transgene confers a more pleiotropic effect than the SO2 transgene on zein genes regulation and (ii) that O2 trans-acting factor is probably indirectly implicated in the transcriptional regulation of the 27γZein gene *via* its interaction with other BZIP trans-acting factors.

Zein pattern analysis in mature seeds shows that impact of O2-TDN transgene is maintained during all endosperm development stages; all the modifications concerning zein accumulation in O2-TDN mutant maize endosperm at 18DAP (see above) held true in mature seeds (cf. panel A, B, C and D of figure 25) and are associated with the production of an opaque phenotype, as illustrated in panel E of figure 27.

Finally, it was shown that the O2-TDN transgene was stable for at least two generations. All T₁ lines generated from the three functional O2-TDN events were all found to segregate T₂ seeds with normal and opaque phenotypes (data not shown).

Comparison of the impact of SO2 and O2-TDN transgenes on transcriptional regulation of maize endosperm genes.

The respective impacts of O2 RNAi silencing (SO2) and O2 trans-dominant inhibitor (O2-TDN) on expression of O2 *trans*-acting factor were evaluated. RT-PCR showed that endogenous O2 mRNAs were nearly totally depleted in SO2 and O2-TDN seeds with a similar apparent efficiency (Figure 26, panel B). In both cases, a residual level of mRNA was still present which suggests that a basal amount of O2 protein can be potentially produced in mutant seeds. Nevertheless, two distinct "knock outs" of O2 protein were obtained and even if it was not total, it was shown that they were very effective, due to their impact on zein gene expression in SO2 and O2-TDN seeds. Interestingly, several indications can be summarized from these results. First, since the O2 gene stays active in SO2 seeds, it can be supposed that O2 protein alone is not directly essential and necessary for its own regulation. However, one can not exclude a double effect, namely, post-transcriptional gene silencing and transcriptional down-regulation *via* O2 depletion. Second, O2ΔBD protein expressed in O2-TDN seeds acts directly by a specific or unspecific BZIP *trans*-acting factor trap, and indirectly by an O2 "knock out". So, it is not surprising that the mutant zein pattern of O2-TDN seeds is at least also as pronounced and perhaps more so than the one from SO2 seeds. Third, the O2-TDN impact on O2 transcriptional regulation reveals that O2 can be indirectly implied in its own regulation. The more probable hypothesis is that O2 protein is able to interact with BZIP trans-acting factors responsible for its transcriptional regulation.

The achievement of transgenic maize plants expressing an effective "knock out" of O2 protein was a good opportunity to study the degree of implication of the O2 protein on the transcriptional regulation of zein genes. In this perspective, the impact of SO2 was compared with that of O2-TDN with respect to the transcriptional regulation of OHP1 and PBF *trans*-acting factors in maize endosperm at 18 DAP. It was shown by RT-PCR that OHP1 and PBF mRNA levels were not disrupted in SO2 seeds whereas there were strongly decreased in O2-TDN seeds (Figure 26, panel B). These results indicate that O2 is not directly implied in transcriptional regulation of PBF and OHP1, but it is able to interact or regulate some *trans*-acting factors responsible for their regulation. As for its own regulation, the O2 protein is not a limiting trans-acting factor for OHP1 and PBF expression. To complete this study, it is also interesting to note that SγZ and SαZ have no effect on OHP1 and PBF transcriptional regulation (data not shown).

These results reveal a much more pleiotropic impact of the O2-TDN transgene than of the SO2 transgene on the transcriptional regulation of zeins genes, of trans-acting factor genes like PBF and OHP1 and probably many other genes. Differences observed between the transcriptional regulation driven by the product of SO2 and O2-TDN transgenes are induced by the intrinsic functional characteristics of O2ΔBD protein, notably through the possibility of initiating a specific or a unspecific bZIP *trans*-acting factor trap. The impact of the O2-TDN transgene can be amplified by the transcriptional down-regulation of some *trans*-acting factors like the PBF and OHP1 proteins, which are not affected by SO2 transgene. For example, Marzábal *et al*. (1998) have demonstrated that the 27γZein promoter is the target of the PBF *trans*-acting factor *via* a specific interaction with Pb3 *cis*-acting element. Consequently, the decrease of 27γZein mRNAs in O2-TDN mutant seeds would be directly bound to a partial depletion of PBF trans-acting factor.

Generation of double and triple-mutant maize lines highly deficient for zein accumulation.

SαZ/SγZ mutant maize line.

The inventors created SαZ/SγZ double-mutant maize lines in order to strongly reduce the protein contents of the alcohol-soluble fraction from maize endosperm. Indeed, αZein and γZein depletion enabled suppression of the near totality of zeins in SαZ/SγZ mutant maize endosperm; these two zeins families are the two most abundant groups of zeins in maize seed with respectively 60-80% (αZeins) and 15-25% (γZeins) of total zein content.

Twenty six SαZ/SγZ mutant maize lines were obtained from cross pollination of T₂ SαZ maize plants with T₂ or T₃ SγZ maize plants. Among the progeny seeds, the majority of transgenic lines were found to segregate with normal, SαZ, SγZ and SαZ/SγZ phenotypes and some transgenic lines were found to segregate with SαZ and SαZ/SγZ or with SγZ and SαZ/SγZ phenotypes. One transgenic line where all seeds had systematically inherited of SαZ and SγZ transgenes and presented an SαZ/SγZ zein phenotype was also obtained.

SαZ/SγZ double-mutant maize endosperm presented a zein pattern highly modified which was the result of the cumulative effect of SαZ and SγZ expression cassettes (Panel A of figure 28, see Sα/γ lane). The 27γZein and 16γZein polypeptides were simultaneously depleted with 19αZein and 22αZein polypeptide families in 18DAP maize endosperm. No apparent changes were observed in the 10δzein and 15βzein polypeptides accumulation; the 15βzein was the single zein detectable by coomassie blue staining. By immunoblot analysis (cf. figure 28, panel G, I and K, see Sα/γ lane), residual αZeins were also visualized with the protein of low molecular weight (<6.5kDa) detected using an anti-βzein antibody. The zein pattern of SγZ and SαZ mutant endosperms obtained by segregation of transgenes (Figure 28, panel A, G, I and K) was identical to the one previously described in panel A of figures 21 and 22 respectively.

Mature seeds analysis has showed that the opaque phenotype was bound to the SαZ expression cassette and all opaque seeds had either a SαZ phenotype or a SαZ/SγZ zein phenotype. The SαZ/SγZ zein phenotype in mature seeds was identical to the one previously described in this specification in immature seeds (Figure 28, compare panel A and D) indicating that the co-expression of SαZ and SγZ RNAi expression cassette led to a stable zein depletion during all stages of seeds development. As shown in panel M of figure 28, it was verified by PCR amplification that the zein pattern correlated with the presence of the corresponding transgene(s).

SαZ/SO2 mutant maize line.

SαZ/SO2 double-mutant maize lines were generated in order to obtain a αZein (19 and 22 kDa) free maize endosperm, different to the SαZ mutant maize endosperms described above. Fifteen SαZ/SO2 mutant maize lines were obtained from cross pollination of T₂ SαZ maize plants with T₂ SO2 maize plants. Among the progeny seeds, the majority of the transgenic lines were found to segregate with normal, SαZ, SO2 and SαZ/SO2 phenotypes and some transgenic lines were found to segregate with SαZ and SαZ/SO2 phenotypes or with SO2 and SαZ/SO2 phenotypes.

SαZ/SO2 double-mutant maize endosperm at 18DAP presented a highly modified zein pattern which was very similar to that obtained with the SαZ simple-mutant, except that the 15βZein polypeptide was co-depleted with the 19αZein and 22αZein polypeptides (Figure 28, panel B, see Sα*o*2 lane). It is also interesting to note in particular for the 22αzeins, that another difference between the SαZ/SO2 and SαZ mutants is the manner in which 22αZein polypeptides depletion was obtained: (i) for SαZ/SO2 mutant there was simultaneously a 22αZein transcriptional regulation (via SO2) and post-trancriptional gene silencing (via SαZ), whereas (ii) for the SαZ mutant, there was only a 22αZein post-transcriptional gene silencing (*via* SαZ). Apart from these differences, and in the same way as SO2 and SαZ mutant maize endosperms, a significant increase in 27γZeins polypeptide accumulation was also observed by coomassie blue staining and by western blot for the SαZ/SO2 zein pattern (Figure 28, panel B and J, see Sαo2). Immunoblot analysis also showed a protein of low molecular weight (<6.5kDa) that was detected using an anti-βzein antibody, and this low molecular weight protein was over-accumulated in the SαZ/SO2 double mutant seeds (Figure 28, panel L, see Sα*o*2 lane). Thus, the SαZ/SO2 mutant maize endosperm is the result of a cumulated impact of the SαZ and SO2 transgenes; the double mutant contains all the characteristics of both simple-mutants (Figure 28, panel B, H, J and L, see Sαo2). The zein pattern of SαZ and SO2 mutant endosperms (Figure 28, panel B) obtained by segregation of the transgenes was identical to that described in panel A of figures 22 and 23 respectively.

Mature seeds analysis showed that the opaque phenotype was dependent of the SαZ or SO2 transgenes and all opaque seeds had SαZ or SO2 or SαZ/SO2 zein phenotypes. The SαZ/SO2 zein phenotype in mature seeds was identical to that previously described above in immature seeds (Figure 28, compare panel B and E), indicating that the co-expression of SαZ and SO2 RNAi expression cassette led to a stable effect on zeins accumulation during all stages of development. As shown in panel N of figure 28, PCR amplification enabled verification that the zein pattern was correlated with the presence of the corresponding transgene(s).

SO2/SγZ mutant maize line.

Thirty one SγZ/SO2 double-mutant maize lines were generated from cross pollination of T₂ or T₃ SγZ maize plants with T₂ SO2 maize plants. Among the progeny seeds, the majority of transgenic lines were found to segregate with normal, SγZ, SO2 and SγZ/SO2 phenotypes and some transgenic lines were found to segregate with SγZ and SγZ/SO2 phenotypes or with SO2 and SγZ/SO2 phenotypes.

SγZ/SO2 double-mutant maize endosperm at 18DAP presented a zein pattern where the 16γZein and 27γZein polypeptides were co-depleted with 22αZein polypeptides (Figure 28, panel C, see Sγ*o2* lane). As for SO2 mutant maize endosperm, 15βZein polypeptide was partially depleted. Thus, the SγZ/SO2 mutant maize endosperm is the result of a cumulated impact of the SγZ and SO2 transgenes; the double mutant combines all of the characteristics of both simple-mutants. The zein pattern of SγZ and SO2 mutant endosperms (Figure 28, panel C) obtained by segregation of transgenes was identical to that previously described in panel A of figures 21 and 23 respectively.

Mature seeds analysis showed that the opaque phenotype was dependent of the SO2 transgene and all opaque seeds had SO2 or SγZ/SO2 zein phenotypes. The SγZ/SO2 zein phenotype in mature seeds was identical to that previously described into immature seeds (cf. Figure 28, compare panel C and F), indicating that the impact of SγZ and SO2 RNAi expression cassette was constant during all stages of seed development. As shown in panel O of figure 28, PCR amplifications confirmed that the zein pattern was correlated with the presence of corresponding transgene(s).

SαZ/SγZ/SO2 mutant maize line.

SαZ/SγZ/SO2 triple-mutant maize lines were generated in order to eliminate the near totality of the alcohol-soluble protein fraction from maize endosperm. In order to obtain this mutant maize line, SαZ/SγZ double-mutant maize seedlings of the first generation were selected using PCR technology to verify the presence of both SαZ and SγZ transgenes. Five SαZ/SγZ/SO2 mutant maize lines were obtained from cross pollination of these double-mutant maize plants with T₂ SO2 maize plants. Progeny seeds from one of the triple-mutant maize line were studied in detail. The zein patterns observed by coomassie blue staining from mature seeds (Figure 29, panel B) were further characterized by transgene detection from endosperm tissue using PCR technology, in particularly to dissociate SαZ and SαZ/SγZ mutant seeds from respectively SαZ/SO2 and SαZ/SγZ/SO2 mutant seeds. PCR results (Figure 29, panel C) confirmed that the SO2 transgene was associated with a significant reduction in βZein accumulation, which enabled dissociation of SαZ and SαZ/SγZ mutant seeds from respectively SαZ/SO2 and SαZ/SγZ/SO2 mutant seeds. The triple-mutant zein pattern was very similar to that one from SαZ/SγZ, except that a reduction of the βZein accumulation was observed in the presence of SO2 transgene. The SαZ/SγZ/SO2 triple-mutant maize line was the one in which the greatest reduction in zein accumulation in maize endosperm was obtained. The other zein patterns obtained by segregation of transgenes were identical to their homologs as previously described in the present application through the study of simple- and double-mutant maize lines.

Finally, mature seeds analysis also showed that the opaque phenotype was always bound to the SO2 or SαZ transgenes and all opaque seeds had at least one of these transgenes.

Comparison of zeins endosperm content of different mutant maize lines.

The inventors have thus shown, through the study of simple-, double- and triple-mutants maize endosperms that accumulation of every one of the zeins was disrupted by at least one of the transgenes previously described (SαZ, SγZ, SO2 or O2-TDN) and that each transgene conferred its own impact, which was more or less specific in zein accumulation. In Figure 30, a comparison of the impact of each transgene and of the association of different transgenes in zein accumulation in maize endosperm was established:
- SO2 transgene expression enabled reproduction of a mutant maize with the same characteristics as those of *o2* mutant lines (Mertz *et al.,* 1964; Inglett, 1970; Pérez-Grau et al., 1986; Schmidt *et al.,* 1987; Kodrzycki et al., 1989). The main difference between the SO2 and *o2* mutant lines is that the SO2 transgene is dominant whereas the o2 mutation is recessive.
- O2-TDN mutant endosperm is similar to that of SO2, except that the O2 truncated factor (O2ΔBD) seemed to have a negative impact on transcriptional regulation of 27γZein gene whereas 27γZein polypeptides were over-accumulated. These data show a more pleiotropic effect of the O2-TDN transgene, which acts negatively on transcriptional regulation of the 27γZein gene *via* an interaction of O2ΔBD with BZIP *trans-*acting factors from maize endosperm. Thus, transcriptional down regulation of the 27γZein gene by O2-TDN transgene (Figure 25, panel C) is compensated by post-transcriptional, translational and post-translational effects possibly linked to 22αZein depletion. The O2-TDN line is probably the most complex mutant maize line obtained in terms of modifications of biosynthetic pathways. This line is particularly interesting for studying the role of O2 protein in maize endosperm.
- SαZ mutant endosperm is characterized by the co-depletion of 19αZein and 22αZein polypeptides which make up the most abundant of zein families with at least 60-80% of total zein content. This line is, to the applicant's knowledge, the first mutant maize to be created with an endosperm having no αZein storage proteins. As for SO2 or *o2* mutant lines, a compensatory effect on 27γZein accumulation was observed which is probably a consequence of an energy release and/or a rerouting of metabolic pathways implied in the αZein biosynthesis. An advantage of the SαZ mutant is that the transcriptional regulation of zein genes is not affected by the SαZ transgene. As demonstrated above, the SαZ sequence under the control of γZ promoter does not negatively affect γZein accumulation. A major advantage of the selected approach is the possibility to use the same promoter separately for both silencing storage protein genes and expressing recombinant protein encoding gene or cDNA. For example, α- or β- or γ- or δ-zein promoters can be used for zein encoding gene silencing and heterologous recombinant protein production in maize seeds.
- SγZ transgene expression enabled production of a mutant maize endosperm with a decrease of at least 15-25% in total zein content, i.e. without γZein polypeptides (16 and 27 kDa). The γZein depletion had no impact on accumulation of other zein polypeptides and their implication in protein body stabilization does not appear to be crucial.
- Double and triple mutant endosperms described above conserve all the characteristics of each simple mutant. For example, SαZ/SγZ mutant endosperm associates the functional impact of SαZ transgene with that from SγZ. The result is a double mutant maize endosperm in which the αZein and the γZein polypeptides are near totally depleted. This mutant shows the most significant quantity of zein depletion (about 90 to 95% of total zein content) without disruption of the transcriptional regulation of the zein genes. The SαZ/SγZ/SO2 triple-mutant maize line shows the greatest reduction (about 98%) in total zein accumulation in maize endosperm, since the SO2 transgene confers to the SαZ/SγZ double mutant a significant reduction of the βZein polypeptide. Finally, the SαZ/SO2 mutant is also very interesting because an endosperm with a depletion of 19αZein and 22αZein, associated with over-expression of the 27γZeins was obtained.

All of the transgenes described (SαZ, SγZ, SO2 and O2-TDN) are dominant, stable during all stages of the maize endosperm development and functionally transmitted to descendants. All mutant seeds obtained and described above are able to germinate with the same efficacy that wild type seeds.

### EXAMPLE 2

Increasing recombinant human lactoferrin (rhLTF) content in maize endosperm.

Molecular constructs

The entire cDNA sequence (SEQ.ID62) of the human lactoferrin (hLTF) coding for the mature 692 amino acid protein preceded by the 19 amino acid signal peptide (PSLf) was isolated from pSB-Lf12 plasmid (Salmon *et al.,* 1998) using *Eco*RI and introduced upstream of the PolyA35S into *Eco*RI site of pMRT1175 (Comeau and Gruber, 2001; Figure 31) to generate pMRT1243 (Figure 32). The MPr1139 promoter from pMRT1139 (Norre *et al.*, 2002; Figure 33) was cloned upstream from PSLf-hLTF-PolyA35S as blunted EcoRl-BamHl into end-filled *Hind*III-*Bam*HI sites of the pMRT1243. Then, the MPr1139-PSLf-hLTF-PolyA35S expression cassette, isolated from the resulting pMRT1258 plasmid (Figure 34) using blunted *Kpn*I*-Xho*I*,* was introduced into *Hpa*I*-Xho*I sites of pMRT1195 (Figure 7; Comeau and Gruber, 2001) to give pMRT1270 binary plasmid (Figure 35).

Generation of maize plants expressing hLTF.

Immature embryos from the inbred line A188 were transformed with *Agrobacterium tumefaciens* LBA4404 that carried the pSB1 plasmid and the binary plasmid pMRT1270 described above, according to the procedure described by Ishida *et al.* (1996). Independent primary transformants were crossed with a commercial elite line to produce T₁ seeds. T₂, T₃ and T₄ seeds were obtained by successive self pollinations from T₁ plants. These lines expressing rhLTF were named LTF maize lines.

Generation of mutant maize plants expressing rhLTF.

To produce the hLTF protein in mutant maize lines described in example 1, cross pollinations of SαZ (T₃), SO2 (T₂), SαZ/SγZ (T₁) and SαZ/SO2 (T₁) mutant maize lines were carried out with a T₄ homozygote hLTF maize line obtained as described above. The mutant maize lines used in these crossing were selected by seed opacity before they are sowed. The selection of SαZ/SγZ and SαZ/SO2 double mutant maize lines was completed by a PCR screening from seedlings as previously described in example 1.

Analysis of mutant maize seeds expressing rhLTF.

Each cob was analyzed seed per seed to determine the differential rhLTF accumulation according to the genetic background of each seed. Thus, each dry mature seed was independently ground and the ground material was divided in two fractions. (i) The first fraction of the flour was used to extract TSP over night at 4°C with a 10-fold (w/v) excess of an aqueous buffer (50mM Tris-HCl, 100mM NaCl, EDTA 1mM, Triton X100 0.2%, pH 7.5). Then, insoluble materials were eliminated by centrifugation of the crude extract (16060g, 30min.). TSP concentration was systematically evaluated by means of the Bradford Assay (BioRad) in order to control the extraction efficacy for each sample. The maize rhLTF content in the clarified crude extracts was quantified by double sandwich ELISA using conventional techniques. ELISA plates were coated with a purified monoclonal mouse anti-human lactoferrin (MAHu/Lfr/MAb, Tebu). Plant cell TSP was then incubated in the wells to capture the rhLTF. Bound rhLTF was revealed by a goat anti-human lactoferrin HRP-conjugated (GAHu/Lfr/PO, Tebu) and TMB reagent (Sigma). The lactoferrin control curve was made using a purified lactoferrin from Calbiochem (#427269). Finally, the maize rhLTF was visualized from crude extract by 10% SDS-PAGE separation. Western blot analyses were realized under reducing conditions using a rabbit anti-human lactoferrin (L-3262, Sigma). Secondary antibody was the anti-rabbit immunoglobulins (IgG) conjugated to alkaline phosphatase (Sigma) which was detected using BCIP/NBT Alkaline Phosphatase Substrate (B-5655, Sigma). For each sample, the same volume of TSP extract was loaded in order to compare their immunoblot patterns. (ii) The second fraction of flour was submitted to alcohol-soluble protein extraction to determine the genetic background of each seed after analysis of the zein pattern as already described in example 1.

### RESULTS AND DISCUSSION.

The influence of differential zein depletion on recombinant protein accumulation was studied through the expression of hLTF protein in SαZ, SO2, SαZ/SγZ and SαZ/SO2 mutant maize lines previously described in example 1. The PSLf-hLTF cDNA was expressed under the control of the MPr1139 promoter which is active in maize seed specifically in the endosperm tissue (Norre *et al.,* 1999 and 2002; Norre, 2002) and the polyA35S terminator.

Analysis of the rhLTF produced in SαZ/SγZ double mutant maize line.

The inventors produced the human lactoferrin (hLTF) protein in a SαZ/SγZ double mutant maize line in order to evaluate the respective impact of αZ and/or γZ depletion on accumulation of a recombinant protein in endosperm tissue. One set of seeds from a SαZ/SγZ/rhLTF maize line, obtained following the fertilization of a SαZ/SγZ mutant maize plant by the pollen of an LTF maize plant, was analyzed in detail. Twenty seeds from this cob were independently analyzed, among which 50% were selected with a translucent phenotype and 50% with an opaque phenotype. All seeds from the descendants had inherited the hLTF transgene and the impact of each genetic background was analyzed *via* segregation of SγZ and SαZ transgenes. As shown in the panel A of the figure 36, translucent seeds (lanes 1 to 10) had either a wild type (lanes 1, 3, 4, 6, 7, 8 and 9) or a SγZ (lanes 2, 5 and 10) genetic background whereas opaque seeds (lanes 11 to 20) had either a SαZ (lanes 11, 13, 16 and 20) or a SαZ/SγZ (lanes 12, 14, 15, 17, 18 and 19) genetic background. Thus, the quantity of rhLTF accumulated (Figure 36, panel B) was only dependent on the genetic background; there was no possible variation bound to the potential environmental conditions. The zein depletion was systematically associated with a strong increase of rhLTF accumulation in maize endosperm (Figure 36, panel B); rhLTF was not detected in wild type endosperm used as negative control (data not shown). The quantity of rhLTF was respectively on average 1.9-, 3.8- and 7.0-fold higher in SγZ, SαZ and SαZ/SγZ seeds than in wild type seeds (Figure 40, SαZ/SγZ/rhLTF line). The positive impact was correlated to the quantity of depleted zeins and seemed to be independent of the type of zein silenced. Thus, the impact associated with γZ polypeptide depletion (∼ 15 to 25% of total zein content) was less important than the one associated with αZ polypeptide depletion (∼ 60 to 80% of total zein content) which was less important than the one bound to αZ and γZ polypeptides co-depletion (∼ 90 to 95% of total zein content). It is thus interesting to note that the positive impact associated with αZ and γZ elimination is synergistic when αZ and γZ are co-depleted. Characterization of the rhLTF produced in the different genetic backgrounds was conducted by western blot. Under reduced conditions (Figure 37), the electrophoretic profile showed a single band of rhLTF at the expected size (approximately 80kDa); this band was not detected when a negative control was loaded (data not shown). Thus, the immunoblot patterns of rhLTF confirmed that the positive impacts were proportional to the quantity of depleted zeins as already demonstrated by ELISA analysis. Finally, there was respectively in SγZ, SαZ and SαZ/SγZ seeds up to 0.32mg, 0.60 mg and 1.2mg of rhLTF per gram of seed dry weight whereas in wild type seed from this cob there was only at most 0.18mg of rhLTF per gram of seed dry weight.

Analysis of the rhLTF produced in SαZ/SO2 double mutant maize line.

Human lactoferrin (hLTF) in a SαZ/SO2 maize line was produced in order to compare the respective impact of SαZ, SO2 and both transgenes on accumulation of a recombinant protein in endosperm tissue. One set of seeds from a SαZ/SO2/rhLTF maize line, obtained following the fertilization of a SαZ/SO2 mutant maize plant by the pollen of an LTF maize plant, was analyzed in detail. Fourteen seeds from this cob were independently analyzed, among which five were selected with a translucent phenotype and nine with an opaque phenotype. All seeds from the descendants had inherited the hLTF transgene and the impact of each genetic background was analyzed *via* segregation of SαZ and SO2 transgenes. As shown in panel A of the figure 38, translucent seeds (lanes 1 to 5) had a wild type genetic background whereas opaque seeds (lanes 6 to 14) had either a SO2 (lanes 8, 11 and 13) or a SαZ/SO2 (lanes 6, 7, 9, 10, 12 and 14) genetic background. No seed was identified with a SαZ genetic background, indicating that SαZ/SO2 maize line used for this crossing was probably homozygote for the SO2 transgene. The quantity of rhLTF accumulated (Figure 38, panel B) was only dependent on the genetic background; there was no possible variation bound to potential environmental conditions. The zein depletion was systematically associated with a strong increase of rhLTF accumulation in maize endosperm (Figure 38, panel B). The quantity of rhLTF was respectively on average 3.1- and 3.9-fold higher in SO2 and SαZ/SO2 seeds than in wild type seeds (Figure 42, SαZ/SO2/rhLTF line). The positive impact was correlated to the quantity of depleted zeins; the impact associated with the SO2 genetic background (22αZ polypeptide depletion and βZ reduction, see Figure30) was less important than the one associated with the SαZ/SO2 genetic background (22αZ and 19αZ polypeptide depletion and βZ reduction, see Figure 30). The reduction of 15βZ accumulation probably has a negligible impact on rhLTF production in comparison to the elimination of the αZ polypeptides. Characterization of rhLTF produced in the different genetic backgrounds was conducted by western blot. Under reduced conditions (Figure 39), the electrophoretic profile showed a single band of rhLTF at the expected size (approximately 80kDa). Thus, the immunoblot patterns of rhLTF confirmed that zein depletion, *via* the SO2 and SαZ transgenes, had a very favourable impact on rhLTF accumulation in maize endosperm as already demonstrated by ELISA analysis. Finally, there was respectively in SO2 and SαZ/SO2 seeds up to 0.50mg and 0.67mg of rhLTF per gram of seed dry weight whereas in wild type seed from this cob there was only at maximum 0.16mg of rhLTF per gram of seed dry weight.

Analysis of the rhLTF produced in SαZ mutant maize line.

Human lactoferrin (hLTF) in a SαZ maize line was produced in order to confirm the positive impact of αZ depletion on accumulation of rhLTF protein in endosperm tissue. One set of seeds from a SαZ/rhLTF maize line, obtained after fertilization of a SαZ mutant maize plant by the pollen of an LTF maize plant, was analyzed in detail. Twelve seeds from this cob were independently analyzed, among which 50% were selected with a translucent phenotype and 50% with an opaque phenotype. All seeds from the descendants had inherited the hLTF transgene and the impact of SαZ genetic background was analyzed *via* segregation of the SαZ transgene. Translucent seeds had a wild type genetic background whereas opaque seeds had a SαZ genetic background (data not shown). Thus, the quantity of rhLTF accumulated was only dependent on the genetic background; there was no possible variation bound to the potential environmental conditions. The αZ depletion was systematically associated with a strong increase in rhLTF accumulation in maize endosperm. The quantity of rhLTF was on average 3.6-fold higher in six SαZ seeds than in six wild type seeds (Figure 40, SαZ/rhLTF line). Characterization of the rhLTF produced in wild type and SαZ genetic backgrounds was conducted by western blot (data not shown); under reduced conditions, the electrophoretic profile showed a single band of rhLTF at the expected size (approximately 80kDa). Finally, there was respectively in SαZ seeds up to 0.80 mg of rhLTF per gram of seed dry weight whereas in wild type seed from this cob there was only at most 0.21 mg of rhLTF per gram of seed dry weight.

Analysis of the rhLTF produced in SO2 mutant maize line.

Human lactoferrin (hLTF) in a SO2 maize line was produced in order to confirm the positive impact of the SO2 transgene on accumulation of rhLTF protein in endosperm tissue. One set of seeds from a SO2/rhLTF maize line, obtained after fertilization of a SO2 mutant maize plant by the pollen of an LTF maize plant, was analyzed in detail. Ten seeds from this cob were independently analyzed, among which five were selected with a translucent phenotype and five with an opaque phenotype. All seeds from the descendants had inherited the hLTF transgene and the impact of the SO2 genetic background was analyzed *via* segregation of the SO2 transgenes. Translucent seeds had a wild type genetic background whereas opaque seeds had a SO2 genetic background (data not shown). The quantity of rhLTF accumulated was only dependent on the genetic background; there was no possible variation bound to the potential environmental conditions. Zein depletion was systematically associated with a strong increase of rhLTF accumulation in maize endosperm. As shown in Figure 40 (SO2/rhLTF line), the quantity of rhLTF was on average 3.5-fold higher in SO2 seeds than in wild type seeds. Characterization of the rhLTF produced in the wild type and SO2 genetic backgrounds was conducted by western blot (data not shown); under reduced conditions, the electrophoretic profile showed a single band of rhLTF at the expected size (approximately 80kDa). Finally, there was in SO2 seeds up to 0.59mg of rhLTF per gram of seed dry weight whereas in wild type seed from this cob there was only at most 0.18mg of rhLTF per gram of seed dry weight.

Comparison of differential zein depletion on rhLTF accumulation in maize endosperm.

The quantity of rhLTF accumulated in seeds from SαZ/SγZ/rhLTF, SαZ/rhLTF, SαZ/SO2/rhLTF and SO2/rhLTF maize lines studied above was compared. As shown in Figure 40, the quantity of rhLTF produced in wild type seeds did not significantly vary between the four lines studied. This absence of variation in wild type seeds indicated that the quantity of rhLTF accumulated was only dependent on genetic background; there was no variation due to potential environmental conditions during the development of each plant in this study. This observation enabled comparison of the quantities of rhLTF produced in different sets of seeds and in varying genetic backgrounds. Zein depletion was systematically associated with a strong increase of rhLTF accumulation in maize endosperm. The positive impact was directly bound to the quantity of depleted zeins, but independent of the type of zein concerned. Thus, the new mutant maize lines described in the present invention can be summarized as follows according to their capacity to overproduce and store the rhLTF in endosperm tissue:
(1) the SαZ/SγZ double mutant maize line is the line where the greatest quantity of rhLTF was found. In SαZ/SγZ seeds, characterized by the simultaneous depletion of αZeins (19 and 22 kDa) and γZeins (16 and 27 kDa) polypeptides (∼ 90 to 95% of total zein content), the quantity of rhLTF was on average 7.0-fold higher than in wild type seeds;
(2) in SαZ seeds, characterized by the co-depletion of 19αZein and 22αZein polypeptides (∼ 60 to 80% of total zein content), the quantity of rhLTF was on average between 3.6- to 3.8-fold higher than in wild type seeds;
(3) in SαZ/SO2 seeds, characterized by the co-depletion of 19αZein and 22αZein polypeptides and by a reduction of the 15βZein, the quantity of rhLTF was not significantly different from the one in SαZ seeds. This result is not surprising, since the SO2 transgene has a more or less redundant function with that of the SαZ transgene. The reduction of about 50% of the βZ conferred by the silencing of O2 *trans*-acting factor does not seem to have a significant impact on rhLTF accumulation;
(4) in SO2 seeds, characterized by the depletion of 22αZein and by a reduction of the 15βZein polypeptides, the quantity of rhLTF was on average between 3.1- to 3.5-fold higher than in wild type seeds;
(5) in SγZ seeds, characterized by the co-depletion of 16γZein and 27γZein polypeptides (~ 15 to 25% of total zein content), the quantity of rhLTF was on average 1.9-fold higher than in wild type seeds.

To conclude, it can be seen from the above that the positive impact conferred by γZ depletion alone is not as important in comparison to that associated with αZ depletion alone, but when the two are associated together, there is a marked synergistic and surprising increase in over production of the heterologous protein. Thus, it has been shown that modulation of zein production profiles can be used advantageously in accordance with the present invention to overproduce another molecule of any desired choice, such at those previously listed in the specification, in a transformed plant or plant cell.

### Bibilographic References

Beccari, J.B. (1745). De frumento. De bononiensi scientarium et artium. Instituto atque Academia Commentarii, Bologna 2, 122-127.
Comeau, D. and Gruber, V. (2001) Vecteurs synthétiques propres, plasmides, plantes et parties de plantes transgéniques les contenant, et leurs méthodes d'obtention. PCT patent application published under n° WO 01/18192
Ellenberger, T.E., Brandl, C.J., Struhl, K., Harrison, S.C. (1992) The GCN4 basic region leucine zipper binds DNA as a dimer of uninterrupted alpha helices: crystal structure of the protein-DNA complex. Cell., 24, 1223-1237.
Fujiwaraa, T., Nambarab, E., Yamagishib, K., Gotoc, D.B., and Naito, S. (2002) Storage Proteins. The Arabidopsis Book*.*
Green, C. E., and Phillips R.L. (1975) Plant regeneration from tissue cultures of maize. Crop. Sci. 15, 417-421.
Huang, A.H.C., Qu, R., Wang, S.M., Vance, V.B., Cao, Y.Z. and Lin, Y.H. (1987) Synthesis and degradation of lipid bodies in the scutella of maize. In PK Stumpf, JB Mudd, WD Nes, eds, The Metabolism, Structure, and Function of Plant Lipids. Plenum Press, New York pp 239-246.
Hunter, B.G., Beatty, M.K., Singletary, G.W., Hamaker, B.R., Dilkes, B.P., Larkins, B.A. and Jung, R. (2002) Maize opaque endosperm mutations create extensive changes in patterns of gene expression. Plant Cell., 14, 2591-612.
Inglett, G.E. (1970) Corn: culture, processing, products. Major feed and food. Crops in agriculture and food series. AVI, Westport, Connecticut, U.S.A. 7, 123-137.
Ishida, Y., Saito, H., Ohta, S., Hiei, Y., Komari, T. and Kumashiro, T. (1996) High efficiency transformation of maize (Zea mays L.) mediated by Agrobacterium tumefaciens. Nature Biotech., 14, 745-750.
Kodrzycki, R. Boston, R.S. and Larkins, B.A. (1989). The opaque-2 mutation of maize differentially reduces zein gene transcription. Plant Cell. 1, 105-114. Lawrence, S.D., Greenwood, J.S., Korhnak, T.E. and Davis, J.M. (1997) A vegetative storage protein homolog is expressed in the growing shoot apex of hybrid poplar. Planta, 203, 237-244.
Marzábal, P., Busk, P.K., Ludevid, M.D. and Torrent, M. (1998) The bifactorial endosperm box of γ-zein gene: characterisation and function of the Pb3 and GZM cis-acting elements. Plant J., 16, 41-52.
Maschke, O. (1859). Ueber den Bau und die Bestandtheile der Kleberblaschen in Bertholletia, deren Entwickelung in Ricinus, nebst einigen Bemerkungen über Amylonblaschen. Botanische Zeitung, 17, 409-447.
Matzke, M.A., Matzke, A.J., Pruss, G.J. and Vance, V.B. (2001) RNA-based silencing strategies in plants. Curr. Opin. Genet. Dev., 11, 221-227.
Mertz, E.T., Bates, L.S. and Nelson, O.E. (1964) Mutant gene that changes protein composition and increases lysine content of maize endosperm. Science., 145, 279-280.
Miao, Z.H. and Lam, E. (1995) Construction of a trans-dominant inhibitor for members of the TGA family of transcription factors conserved in higher plants. Plant J., 7, 887-896.
Motto, M., Di fonzo, N., Hartings, H., Maddaloni, M., Salamini, F., Soave, C., and Thompson, R. D. (1989) Regulatory genes affecting maize storage protein synthesis. In Oxford Surveys of Plant Molecular and Cell Biology, 6, B.J. Miflin, ed (New York: Oxford University Press), 87-114.
Norre, F., Theisen, M. and Gruber, V. (1999) Synthetic and chimeric promoters, expression cassettes, plasmids, vectors, transgenic plants and seeds containing them, and method for producing them. Patent WO 01/23593.
Norre, F. and Gruber, V. (2001) Bifactorial endosperm box, trans-activating factor that binds therto, and method of regulation of promoter activity. Patent PCT/EP 02/08721.
Norre, F., Peyrot, C., Garcia, C., Rancé, I., Drevet, J., Theisen, M. and Gruber, V. (2002) Powerful effect of an atypical bifactorial endosperm box from wheat HMWG-Dx5 promoter in maize endosperm. Plant Mol. Biol. 50, 699-712.
Norre, F. (2002) Régulation transcriptionnelle du promoteur HMWG-Dx5 de Blé dans l'albumen de maïs et création de promoteurs chimériques. PhD, Université Blaise Pascal, Clermont II.
Osborne, T.B. (1924). The Vegetable Proteins. (London: Longmans, Green). P6rez-Grau, L.I., Cortadas, J., Puigdomènech, P., and Palau, J. (1986) Accumulation and subcellular lacalization of glutenin-2 transcripts during maturation of maize endosperm. FEBS, 202, 145-148.
Pontier, D., Miao, Z.H. and Lam, E. (2001) Trans-dominant suppression of plant TGA factors reveals their negative and positive roles in plant defense responses. Plant J., 27, 529-538.
Qu, R., Wang, S.M., Lin, Y.H., Vance, V.B. and Huang, A.H.C. (1986) Characteristics and biosynthesis of membrane proteins of lipid bodies in the scutella of maize (Zea mays L.). Biochem J., 234, 57-65.
Reina, M., Ponte, I., Guillén, P., Boronat, A. and Palau, J. (1990) Sequence analysis of a genomic clone encoding a Zc2 protein from Zea mays W64 A. Nucl. Acids Res., 18, 6426.
Rieping, M., Fritz, M., Prat, S. and Gatz, C. (1994) A dominant negative mutant of PG13 suppresses transcription from a cauliflower mosaic virus 35S truncated promoter in transgenic tobacco plants. Plant Cell., 6, 1087-1098.
Salmon, V., Legrand, D., Slomianny, M.C., el Yazidi, I., Spik, G., Gruber, V., Bournat, P., Olagnier, B., Mison, D., Theisen, M., Merot, B. (1998). Production of human lactoferrin in transgenic tobacco plants. Protein Expr Purif., 13, 127-35.
Sambrook, J., Fritsch, E.F. and Maniatis, T. (1989) In "Molecular cloning : a laboratory manual, second edition" . Cold Spring Harbor, New-York.
Schmidt, R.J., Burr, F.A. and Burr, B. (1987) Transposon tagging and molecular analysis of the maize regulatory locus opaque-2. Science. 238, 960-963.
Segal, G., Song, R., Messing, J. (2003) A new opaque variant of maize by a single dominant RNA-interference-inducing transgene. Genetics, 165, 387-397.
Shewry, R., Napier, Johnathan, A. and Tatham, Arthur S. (1995) Seed Storage Proteins: Structures and Biosynthesis. The Plant Cell, 7, 945-956.
Song, R., Llaca, V., Linton, E., Messing, J. (2001) Sequence, regulation, and evolution of the maize 22-kD alpha zein gene family. Genome Res., 11, 1817-1825.
Staswick, P.E. (1988). Soybean vegetative storage protein structure and gene expression. Plant Physiol. 89, 309-315.
Tavernarakis, N., Wang, S.L., Dorovkov, M., Ryazanov, A., Driscoll, M. (2000) Heritable and inducible genetic interference by double-stranded RNA encoded by transgenes. Nat. Genet., 24, 180-1833.
Vancanneyt, G., Schmidt, R., O'Connor-Sanchez, A., Willmitzer, L. and Rocha-Sosa, M. (1990) Construction of an intron-containing marker gene: Splicing of the intron in transgenic plants and its use in monitoring early events in Agrobacterium-mediated plant transformation. Mol. Gen. Genet., 220, 245-250.
Vance, V.B., Huang, A.H.C. (1987) The major protein from lipid bodies of maize. Characterization and structure based on cDNA cloning. J. Biol. Chem., 262, 11275-11279.
Vinson, C.R., Sigler, P.B., McKnight, S.L. (1989) Scissors-grip model for DNA recognition by a family of leucine zipper proteins. Science, 17, 911-916.
Wetzel, S. and Greenwood, J.S. (1991) The 32-Kilodalton Vegetative Storage Protein of Salix microstachya Turz - Characterization and Immunolocalization. Plant Physiol., 97, 771-777.
Wiltshire, J.J.J., and Cobb, A.H. (1996). A review of the physiology of potato tuber dormancy. Ann. Appl. Biol., 129, 553-569.
Wittenbach, V.A. (1982). Effect of pod removal on leaf senescence in soybeans. Plant Physiol. 70, 1544-1548.
Wittenbach, V.A. (1983). Effect of pod removal on leaf photoshythesis and soluble protein composition of field-grown soybeans. Plant Physiol. 73, 121-124.
Woo, Y.M., Hu, D.W., Larkins, B.A., Jung, R. (2001) Genomics analysis of genes expressed in maize endosperm identifies novel seed proteins and clarifies patterns of zein gene expression. Plant Cell., 13, 2297-2317.
Wurch, T., Lestienne, F. And Pauwels, P.J. (1998) A modified overlap extension PCR method to create chimeric genes in the absence of restriction enzymes. Biotechnology Techniques, 12, 653-657.
Zhu, B. and Coleman, G.D. (2001) The poplar bark storage proteingene (Bspa promoter is responsive to photoperiod andnitrogen in transgenic poplar and active in floral tissues, immatureseeds and germinating seeds of transgenic tobacco. Plant Mol. Biol., 46, 383-394.

## Claims

1. Genetically modified plant cell comprising at least one stably integrated nucleic acid sequence that specifically modulates a production profile of at least one plant storage protein.

2. Genetically modified plant cell according to claim 1, wherein the at least one stably integrated nucleic acid sequence comprises a first nucleic acid sequence that interferes directly with expression of the at least one plant storage protein.

3. Genetically modified plant cell according to claim 1, wherein the at least one stably integrated nucleic acid sequence comprises a first nucleic acid sequence that interferes indirectly with expression of the at least one plant storage protein.

4. Genetically modified plant cell according to any one of claims 1 to 3, wherein the modulation of the production profile of at least one plant storage protein occurs through the use of direct or indirect *trans-*dominant transcriptional factors.

5. Genetically modified plant cell according to any one of claims 1 to 3, wherein the modulation of the production profile of at least one plant storage protein occurs through direct action of the first nucleic acid sequence or its product on plant promoter activity.

6. Genetically modified plant cell according to any one of claims 1 to 3, wherein the modulation of the production profile of at least one plant storage protein occurs through direct or indirect action on transcriptional, post-transcriptional, translational or post-translational processing.

7. Genetically modified plant cell according to claim 1, wherein the at least one stably integrated nucleic acid sequence comprises a first nucleic acid sequence that interferes directly or indirectly with expression of the at least one plant storage protein, and is associated with a second nucleic acid sequence that codes for a heterologous molecule.

8. Genetically modified plant cell according to claim 7, wherein the heterologous molecule coded for by the second nucleic acid sequence is a therapeutically active polypeptide having an activity selected from the group consisting of digestive, pancreatic, biliary, antiviral, anti-inflammatory, pulmonary, antimicrobial, haematological, neurological, cardiovascular, ophthalmic, antigenic, cerebral, anti-tumour, immunostimulating, and immunomodulating activities.

9. Genetically modified plant cell according to claim 8, wherein the polypeptide is selected from the group consisting of insulins, interferons, gastric, pancreatic or biliary lipases, elastases, anti-proteases such as alpha-1 anti-trypsin, structure-forming proteins such as collagen, transferrins such as lactoferrin, blood-derived proteins, such as human haemoglobin or albumin, and blood cofactors, antioxidants such as superoxide dismutase, antibodies, antibody fragments and antigens.

10. Genetically modified plant cell according to claim 9, wherein the polypeptide is selected from the group of antibodies and antibody fragments consisting in immunoglobulin molecules, immunoglobulin heavy chain, substantially intact immunoglobulin molecules, and any portion of an immunoglobulin that contains the paratope, including Fab fragments, Fab' fragments, F(ab').sub.2 fragments and Fv fragments, immunoglobulin light chain, and F.sub.V fragments.

11. Genetically modified plant cell according to any one of the preceding claims, wherein the first nucleic acid sequence interfering directly or indirectly with the at least one storage protein production profile is selected from the group consisting of the nucleic acid sequences identified by the SEQ.ID numbers SEQ.ID01, SEQ.ID02, SEQ.ID03, SEQ.ID04, SEQ.ID05, SEQ.ID06, SEQ.ID13, SEQ.ID14, SEQ.ID15, SEQ.ID16, SEQ.ID17, SEQ.ID18, SEQ.ID19, SEQ.ID20, SEQ.ID21, SEQ.ID22, SEQ.ID23, SEQ.ID24, SEQ.ID25, SEQ.ID26, SEQ.ID27, SEQ.ID28, SEQ.ID29, SEQ.ID30, SEQ.ID33, SEQ.ID34, SEQ.ID35, SEQ.ID36, SEQ.ID37, SEQ.ID38, SEQ.ID39, SEQ.ID44, SEQ.ID45, SEQ.ID50, SEQ.ID51, SEQ.ID52, SEQ.ID53, SEQ.ID54, SEQ.ID56, SEQ.ID57, SEQ.ID58, SEQ.ID60, and SEQ.ID63, their complementary sequences, or sequences that hybridize thereto under stringent conditions.

12. Genetically modified plant cell according to any one of the preceding claims, wherein the at least one nucleic acid sequence modulating the storage protein production profile is selected from the group consisting of the nucleic acid sequences identified by the SEQ.ID numbers SEQ.ID01, SEQ.ID02, SEQ.ID03, SEQ. ID04, SEQ.ID05, SEQ.ID06, SEQ.ID13, SEQ.ID14, SEQ.ID15, SEQ.ID16, SEQ.ID17, SEQ.ID18, SEQ.ID19, SEQ. ID20, SEQ.ID21, SEQ.ID22, SEQ. ID23, SEQ.ID24, SEQ.ID25, SEQ.ID26, SEQ.ID27, SEQ.ID28, SEQ. ID29, SEQ.ID30, SEQ.ID33, SEQ.ID34, SEQ.ID35, SEQ.ID36, SEQ.ID37, SEQ. ID38, SEQ. ID39, SEQ.ID44, SEQ.ID45, SEQ.ID50, SEQ.ID51, SEQ. ID52, SEQ.ID53, SEQ.ID54, SEQ.ID56, SEQ.ID57, SEQ.ID58, SEQ.ID60, and SEQ.ID63, their complementary sequences, or sequences that hybridize thereto under stringent conditions.

13. Genetically modified plant cell according to claim 1, wherein the at least one plant storage protein is a seed storage protein.

14. Genetically modified plant cell according to any one of the preceding claims, wherein the plant storage protein is a gamma zein.

15. Genetically modified plant cell according to any one of the preceding claims, wherein the plant storage protein is an alpha zein.

16. Genetically modified plant cell according to any one of the preceding claims, wherein the plant storage protein is a beta zein.

17. Genetically modified plant cell according to any one of the preceding claims, wherein the plant storage protein is a delta zein.

18. Genetically modified plant cell according to any one of the preceding claims, wherein the production profile of said at least one storage protein shows reduced production of said at least one storage protein compared to a corresponding non-genetically modified plant cell.

19. Genetically modified plant cell according to any one of the preceding claims, wherein the production profile of said at least one storage protein shows substantial depletion of said at least one storage protein compared to a corresponding non-genetically modified plant cell.

20. Genetically modified plant cell according to any one of the preceding claims, wherein the production profile of said at least one storage protein shows a reduction greater than or equal to 90% in production of said at least one storage protein compared to a corresponding non-genetically modified plant cell.

21. Genetically modified plant cell according to any one of the preceding claims, wherein the production profile of said at least one storage protein shows a reduction greater than or equal to 75% in production of said at least one storage protein compared to a corresponding non-genetically modified plant cell.

22. Genetically modified plant cell according to any one of the preceding claims, wherein the production profile of said at least one storage protein shows a reduction greater than or equal to 50% in production of said at least one storage protein compared to a corresponding non-genetically modified plant cell.

23. Genetically modified plant cell according to any one of the preceding claims, wherein the production profile of said at least one storage protein shows a reduction greater than or equal to 25% in production of said at least one storage protein compared to a corresponding non-genetically modified plant cell.

24. Genetically modified plant cell according to any one of the preceding claims, wherein the production profile of the said at least one storage protein shows a reduction of up to about 100% in production of gamma zein compared to a corresponding non-genetically modified plant cell.

25. Genetically modified plant cell according to any one of the preceding claims, wherein the production profile of said at least one storage protein shows a reduction of up to about 95% in production of alpha zein compared to a corresponding non-genetically modified plant cell.

26. Genetically modified plant cell according to any one of the preceding claims, wherein the production profile of said at least one storage protein shows a reduction of between about 95% and about 50%, preferably up to about 50%, in production of beta zein compared to a corresponding non-genetically modified plant cell.

27. Genetically modified plant cell according to any one of the preceding claims, wherein the production profile of said at least one storage protein shows a reduction of up to about 80% in production of delta zein compared to a corresponding non-genetically modified plant cell.

28. Genetically modified plant cell according to any one of the preceding claims, wherein the production profile of said at least one storage protein is as follows compared to a corresponding non-genetically modified plant cell :
- gamma zein : between about 0% to about 15% of total zein content;
- delta zein: between about 0.08% to about 0.5% of total zein content;
- alpha zein : between about 5% to about 60% of total zein content;
- beta zein: between about 0.25% to about 1.25% of total zein content.

29. Genetically modified plant cell according to any one of the preceding claims, wherein the modulation of the production profile of said at least one storage protein causes the cell to produce and store an increased amount of heterologous molecule compared to a corresponding genetically modified plant cell producing the same molecule without the modulated production profile of said at least one storage protein.

30. Genetically modified plant cell according to any one of the preceding claims wherein a promoter is used that is chosen from any seed-specific promoter, or a target host cell promoter that is natively associated with the expression of the storage protein for which the production profile is to be modulated.

31. Genetically modified plant comprising genetically modified plant cells according to any one of claims 1 to 30.

32. Genetically modified plant seed obtainable from a genetically modified plant or cells according to any one of claims 1 to 31.

33. Genetically modified non-wild type plant seed having a modified production profile of at least one plant storage protein.

34. Genetically modified non-wild type plant seed according to claim 32, wherein the at least one plant storage protein is a seed storage protein, and preferably a zein.

35. Genetically modified non-wild type plant seed according to any one of the preceding claims 32 to 34, wherein the plant seed storage protein is a gamma zein.

36. Genetically modified non-wild type plant seed according to any one of the preceding claims 32 to 35, wherein the plant seed storage protein is an alpha zein.

37. Genetically modified non-wild type plant seed according to any one of the preceding claims 32 to 36, wherein the plant seed storage protein is a beta zein.

38. Genetically modified non-wild type plant seed according to any one of the preceding claims 32 to 37, wherein the plant seed storage protein is a delta zein.

39. Genetically modified plant seed according to any one of the preceding claims 32 to 38, wherein the production profile of said at least one storage protein shows reduced production of said at least one storage protein compared to a corresponding non-genetically modified plant seed.

40. Genetically modified plant seed according to any one of the preceding claims 32 to 39, wherein the production profile of said at least one storage protein shows substantial depletion of said at least one storage protein compared to a corresponding non-genetically modified plant seed.

41. Genetically modified plant seed according to any one of the preceding claims 32 to 40, wherein the production profile of said at least one storage protein shows a reduction greater than or equal to 90% in production of said at least one storage protein compared to a corresponding non-genetically modified plant seed.

42. Genetically modified plant seed according to any one of the preceding claims 32 to 41, wherein the production profile of said at least one storage protein shows a reduction greater than or equal to 75% in production of said at least one storage protein compared to a corresponding non-genetically modified plant seed.

43. Genetically modified plant seed according to any one of the preceding claims 32 to 42, wherein the production profile of said at least one storage protein shows a reduction greater than or equal to 50% in production of said at least one storage protein compared to a corresponding non-genetically modified plant seed.

44. Genetically modified plant seed according to any one of the preceding claims 32 to 43, wherein the production profile of said at least one storage protein shows a reduction greater than or equal to 25% in production of said at least one storage protein compared to a corresponding non-genetically modified plant seed.

45. Genetically modified plant seed according to any one of the preceding claims 32 to 44, wherein the production profile of said at least one storage protein shows a reduction of up to about 100% in production of gamma zein compared to a corresponding non-genetically modified plant seed.

46. Genetically modified plant seed according to any one of the preceding claims 32 to 45, wherein the production profile of said at least one storage protein shows a reduction of up to about 95% in production of alpha zein compared to a corresponding non-genetically modified plant seed.

47. Genetically modified plant seed according to any one of the preceding claims 32 to 46, wherein the production profile of said at least one storage protein shows a reduction of between about 95% and about 50%, but preferably, up to about 50%, in production of beta zein compared to a corresponding non-genetically modified plant seed.

48. Genetically modified plant seed according to any one of the preceding claims 32 to 47, wherein the production profile of said at least one storage protein shows a reduction of up to about 80% in production of delta zein compared to a corresponding non-genetically modified plant seed.

49. Genetically modified plant seed according to any one of the preceding claims 32 to 48, wherein the production profile of said at least one storage protein is as follows compared to a corresponding non-genetically modified plant seed :
- gamma zein : between about 0% to about 15% of total zein content;
- delta zein: between about 0.08% to about 0.5% of total zein content;
- alpha zein: between about 5% to about 60% of total zein content;
- beta zein: between about 0.25% to about 1.25% of total zein content.

50. Method for the production of a genetically modified plant cell comprising :
(a) stably introducing into the genome of said plant cell at least one nucleic acid sequence that specifically modulates a production profile of at least one plant storage protein;
(b) cultivating said plant cell.

51. Method for the production of a genetically modified plant seed comprising:
(a) cultivating a genetically modified plant cell according to claim 47 to yield a plant;
(b) pollinating the genetically modified plant obtained in step (a);
(c) harvesting the seeds obtained from the pollinated plant;
(d) optionally selecting the genetically modified seeds from said plant.

52. Method according to claim 51, wherein the selection in step (d) is carried out based on the degree of opacity of the modified plant seed.

53. Plant cell, plant seed or method according to any one of claims 1 to 52, wherein the plant cell or plant seed is selected from dicotyledonous or monocotyledonous plant species.

54. Plant cell, plant seed or method according to any one of claims 1 to 52, wherein the plant cell or plant seed is selected from the plant families consisting of Solanaceae, Cruciferae, Cucurbitaceae, Brassicae, and Graminae.

55. Plant cell, plant seed or method according to any one of claims 1 to 52, wherein the plant cell or plant seed is selected from the group consisting of arabidopsis, tobacco, alfalfa, potato, lettuce, tomato, bean, castor bean, lupin, pea, carrot, beetroot, turnip, parsnip, banana, pumpkin, melon, lucerne, alfalfa, soya, sorghum, barley, cotton, rice, wheat, maize, rye and oats.

56. Plant seed or method according to any one of claims 1 to 52, wherein the plant seed is homozygous for the at least one stably integrated nucleic acid sequence that specifically modulates a production profile of at least one plant seed storage protein.

57. Plant seed according to any one of the previous claims, wherein the plant seed is homozygous for the at least one stably integrated nucleic acid sequence that specifically modulates a production profile of at least one plant storage protein and also homozygous for the second nucleic acid sequence that codes for a heterologous molecule.

58. Genetically modified plant seed according to any one of the previous claims, wherein the genetically modified seed maintains germinative capability, and preferably displays a germination success rate comprised between about 90% and about 100%.

59. Genetically modified plant cell according to any one claims, or a genetically modified plant, or a genetically modified plant seed, according to any one of the previous claims, wherein said plant cell, plant, or plant seed displays the same abiotic and / or biotic resistance as a wild-type non-genetically modified correspondent.
